# EUROPEAN PATENT APPLICATION

(11) **EP 3 282 019 A1**
(43) Date of publication of application: **14.02.2018**
(21) Application number: 16183455.1
(22) Date of filing: 09.08.2016
(51) Int. Cl.: C12Q 1/68

(54) **GENOTYPING AND TREATMENT OF CANCER, IN PARTICULAR CHRONIC LYMPHOCYTIC LEUKEMIA**

(71) Applicant: Medizinische Universität Wien, 1090 Vienna (AT)
(72) Inventor: HUBMANN, Rainer, 2273 Hohenau (AT); SHEHATA, Medhat, 2344 Maria Enzersdorf (AT)
(74) Representative: Sonn & Partner Patentanwälte

(57) **Abstract**

The present invention relates to the field of genotyping and treating cancer such as leukemia and lymphoma, especially chronic lymphocytic leukemia (CLL). It is an object of the present invention to provide novel tools and methods for genotyping CLL (and other cancers), in particular for predicting disease progression and informing treatment decisions or for targeted treatment of CLL (and other cancers) based on cancer genotype. Surprisingly, in the course of the present invention, deletions in the NOTCH2 gene were found in cells obtained from blood samples of CLL patients. These deletions lead to the expression of N-terminally truncated, constitutively active NOTCH2 variants. The present invention provides a polynucleotide probe for detecting a region of the human NOTCH2 gene in a cell, the sequence of the region being at least 10 consecutive nucleotides selected from the sequence according to SEQ ID NO: 1, wherein the probe is specific for the region or for its reverse complement, and wherein the probe is bound to a molecular tag. The polynucleotide probe is preferentially used in fluorescence in-situ hybridization (FISH). Moreover, the present invention provides an antibody specific for an N-terminally truncated human Notch2 protein starting with the sequence MVYP or the sequence MAKR.

SEQUENCE LISTING

<110>  MEDIZINISCHE UNIVERSITÃ\204T WIEN

<120> Genotyping and treatment of cancer, in particular chronic lymphocytic
leukemia

<130> R68588

<160> 88

<170> BiSSAP 1.3

<210> 1
<211> 15656
<212> DNA
<213> Homo sapiens

<220>
<223> NOTCH2 exon 20 to last nucleotide upstream of START2

<400> 1
accctggtga atctctgcag tcggtctcca tgtaaaaaca aaggtacttg cgttcagaaa      60

aaagcagagt cccagtgcct atgtccatct ggatgggctg gtgcctattg tgacgtgccc     120

aatgtctctt gtgacatagc agcctccagg agaggtaagc tttgctccct tgggctcagg     180

tcattcactg cagtattgac tgtccagtgg catttagaga aggtatcatt tttttgtgga     240

ataagaggga agggcagata gtagtgggtg ggcagggctc tggggaaagg gctcaagaca     300

gaaggtgtaa gggtagagga ataggttcag agattcggag aagaatgtat ttgaaatgaa     360

actaaggtat attccacttt cagggtttat ttttaatatg catctggtac tgagggttgt     420

gaagaactgg ctaagaataa gattggatgt gtcagccagt ttagatttac ttgctcattg     480

gttgatttga accccatgtg tcacatgttc ttctatgaca tttctccctc actgggtttc     540

ccaggtgtgc ttgttgaaca cttgtgccag cactcaggtg tctgcatcaa tgctggcaac     600

acgcattact gtcagtgccc cctgggctat actgggagct actgtgagga gcaactcgat     660

gagtgtgcgt ccaacccctg ccagcacggg gcaacatgca gtgacttcat tggtggatac     720

agatgcgagg tgaggacact gagcaagcag aggtcatgga cctcccagga gattgtacaa     780

ctgtttgagc actgatatat tgaatttagc tgcttgtatc ttatattgat agtttataga     840

tttttaccaa tccccgtcat aatgtttacc actgtttctt tcagcctctc tgtgcaataa     900

gttaacaggt ccctagtatg atatttttat aggcgtctgg aaccatccca gctccttagg     960

cttgctttta ggattcagag ccaagagtca ctaccagagt gcattttaca ggcccaccca    1020

acatagcagt ccacacttcc ctgaatagca gctgagatag gaaaaactgc tttgtggagt    1080

gtccctttgg cccctgtcac ctttgtattt agtcattaat cagtcaacta gcaattttct    1140

ctcaaaaaaa aaaaatgttg ctcatcctgt tccccatagc atcatgctaa acgttacatg    1200

tatatatttg aggaagtatg tgtcttcaag gtacatgtca ctattttaat gtctgggctg    1260

ataatggtta gagcaagcaa gatattagca aaatgcctga gacattaatt tgataccaat 1320

tcaaaccatc aagcaacact gagaaaagct ttgcttctct attgttatat aaatgagcca 1380

acgatagctt ctgtattttg gcccctaggt tgtttatttc ttcacagcag gctggggctg 1440

ggtctcatta gctcaaaagc ctaccagtgc caaacccaaa ttttgacaca tctagttgtt 1500

ttaaacatag cccccaaata agcagatttt tagctattta gagcttgcct gctttgcata 1560

gcctgtaaaa ctgcagccaa aatctgctag ccatagtata agtaggacaa gatataccct 1620

ggggccatac agaccccagg ctgatgctgg cctttagagc tctctgaccc agagattcct 1680

gccactggct gttgagtgac atcacctaga ctccttctct gatccttctt tcccagagtt 1740

tacttgctgt cttcccctta ctcacctacc cctctggaag gtctcatgct atttgggact 1800

tctcccaggt gcaaacctgt caaagtgtcc aaataaagcc acctcacagc catatctttt 1860

ttcttggtca gccctgaaat ccttccaacc cctacagcca ccacagccag ccagccagcc 1920

ccacctccct ctacccctaa gcctatctca accagtttct cacacataga tccaatttcc 1980

taaacctgga gatcaataat tgcatgtctg tactaccatc cacatatgcc ttttgaccga 2040

gtcatagggt cctcatcaca tacggaggaa tgtgtatttt aaagatgagg aaacctgtac 2100

ccaggaggaa caaaaggttt gcacacaagt caaatctgaa acatccttca ttgtattcag 2160

tccttgcctt ccctttaaag tgcctttttc aactgaaaga ggagtaggta aatgccagta 2220

agctgcaggt tttctgtccc tgcatagaat gattagcaga gagtgtgatg gttccagaga 2280

aaacatacaa aagctaaatt aggaggtggg agacagtcac agaaatgttc cagatctcac 2340

tagctctcat ggtctgtttc cagtaatggt ccaatatttc ttgtcctttt tgtttcctca 2400

tggcagtgtg tcccaggcta tcagggtgtc aactgtgagt atgaagtgga tgagtgccag 2460

aatcagccct gccagaatgg aggcacctgt attgaccttg tgaaccattt caagtgctct 2520

tgcccaccag gcactcgggg tatgaaatca tccttatcca ttttccatcc agggcattgt 2580

cttaagttat aaatccattc ttagtgttca ggggatttta taaaattaaa gataggaaga 2640

ctagcttcat tccaagcatt tagttctaca tcctagtaat tcaagccatt ttattctccc 2700

atctcttgct agctctgatg ttgtggttta tgttgtcagt tttatctggt tgtttggcat 2760

cttgatattc catgaaacac agaatatgga agggatacaa cattagcata acattaaaaa 2820

attagcctgg tcagtaagat ttcttgttgc ttcacagaaa agcaactaat ggcctctaaa 2880

ataaacaatt tacatttacc tgcttttgta tttttttaat tagccaagtc ttacctctat 2940

aaggtggggg aatgaatgaa atgaaaatat ctgcctattt tcatagcttc aggggaggat 3000

actgatggtg taaggttcta ccttaggatg cagtaaggcc tgatgagaga tgcaaccctg 3060

gactgttgag tgatggggcc atcattcttc tgtgggcatt gccactgctt ttgaactgag 3120

tggtatggcc atcattctgc tgtggacatt gacactgctt ttgaaccatc ccttggaaag 3180

agctttgagt attgtttgtt agaagggatg tagttatgct ggggcctact ctgagaaggc 3240

tgtgaaaaaa atgtaaaagg ccatccacac tggtgagaaa ttcatccttg ttagtgtcaa 3300

agagaataga atgccagaga agtatttgta ttgccttaga gctttgtgtc agtaaagttt 3360

gataatttgc caccccaggt atcagagttt gactaaatta aaataggaga gtaccctagt 3420

agtaggctga ttattaagga atgaggttaa tgggaagaag aaaactaagg agaaacagtc 3480

tttaagtatt tgaagaattg tcctgtaaaa aggagaattg ttctttattc tagaaatcag 3540

tggttgagta gaaattaatt ccagttcatt gttagtttgt tcctttgaga aactgtactg 3600

gttaggacaa gctacgattc tggtgatagc tgttatcgta gttcatttat gttgctataa 3660

aagaatacct gagactaagt aatttatttt tcgaaagagg tatatttggc tcgcagttct 3720

ataggctgta caggaagcac ggtgccagca tctgcctctg gtgtgagcgt caggctgttt 3780

ccactcatgg tgaaaagtga agaagatcca gggacccaaa cacctcccat taggccccac 3840

ctccaacatt ggagatcaca ttttaacatg tggtttggtg cagtcaaact tcaagctata 3900

gcagctaatt aaacctcaaa tctcagagct taatataaca ctgattttgt cactcaaata 3960

aagtcgaata tgtgttgggc tacctttctc tgtgttttag atatgtggtt tgaaacacct 4020

ggcctccaag gcctccacag tacagggaag agggacagac ttgaagggtc atacagcagc 4080

ttccactagc tcagtctgga agcaacacat atcactatcc ctcgcagtat attgaccgga 4140

attattaaat ggtctctgtg acttcaaggg aggctgggaa atataggaga gtacatgagt 4200

attcatcaaa ctctaaaatc actactatac catgaacttt taattaataa gacatgtcca 4260

gtcctagaat ggatcatttc agaaggaaac aaaatttttg tctcttctaa ccatatggta 4320

aagatggctt agaatactag ttttgatatt atcattgaca gtctttttct ttcagcactt 4380

tgaatatatc atcccactgc tttctgatat ccatggtttt taatgagaaa tatgctgtta 4440

atcttattga ggttccctta tatgtgatac attgcttctt tcttgatgcc ttcaagattc 4500

tttatctttt gatagtttga ttatgatgta tgtaggtatg aatttctttg agtttatcct 4560

acttacagtt ccttggatta ttttactgtg tgtgcttgtt taggtatgtg gaacagtatt 4620

tttttatcct gacttaactt ctctggccat tggtaaacat tccaggaaac tctcaagtcc 4680

cataacccca ttgttagagt catcacaatg tgtgttttgg tcaccatatt tctaagcctt 4740

agatctttgc ctgcatgtcc caccagtttc ccaggtcaag ggctaaatgt aacagttctg 4800

cctcttccta ctactttgct tcaggctgag gttgagttct ctctcagttg atagatctgt 4860

ttatttctgg tggttccatt ctgatgggga aatatgcatt tatgctcatt ttactagcaa 4920

tccctaaata tcaaatattc tttgagacag ggagcatata ctagattttg tttgttttta 4980

cttttttgtt ttcaatttac tctatgtata aggtgatata tgcatatatg catatagacc 5040

aaaagtgtat tcacacatag aaaagtatac agtgaaaata cttctcattc ccatctctct 5100

tctggtgggc aaattttccc tctcctgcaa acatgtaatc gccacttttg gttgtttgtg 5160

tttctttcta gaatttttca aaaactgtat ccttcagaat ttctttatgt agagagaagc 5220

caaatgcaaa gatattttat ttcctttact ttttactctg aagatagaat attagacaca 5280

ttttttgcac ttgatttttt tttacttaat tggagatatt ttcatatcaa aatatagaac 5340

ttcctctctc tctctctgag atagatagat agatagatag atagatagat agatagatag 5400

atatttgttt gtttgtttgt tttgagatgg agttttgctc ttgttgccca ggctggagtg 5460

caatggtatg atctcagctc accgcaacct ccgcctcctg ggttcgagtg attttcctgc 5520

ctcagcgccc tgagtagctg ggattacagg catgtgccac catgcctggc tgattttgta 5580

tttttaatag agacagggtt tctccatgtt ggtcaggctg gtcttgaact cctggcctca 5640

agcaatccac ccgcctcagc ctcccaaagt gctgggatga caggcgtgag ccaccacacc 5700

cagcctgaac ttcctcaata ttttttatag ctacagaatt cccttttaca ggcataccat 5760

attttaacca gtgccatcat gatagatatt tcagttatct tgcaggcata taagtatatt 5820

ggtgaaataa attttcagaa atagaatcgc tggattggat aatatgtgca tttatcattt 5880

taatcagtga ttgcagtata tcagcatatt ggctttttct taaggtggag aatatatttt 5940

catttaagaa tcattttctt ttgatttttt ttgcagtttc tttgtttata tatatatata 6000

tgtatatata catatatata tatatacaca catatatctt ttttcccaca ttaagttgtt 6060

gatgtaatgt tttgtggaaa ttggctatga tataaaaaaa caaatatatt cccagtattt 6120

ttttatcttt tttctttata gtgattttta gtgcacactt tttactttta atcaagttga 6180

atttatcact tttaaaatgg cttctggatt ttgagttgta gttagcaaca tcatatacac 6240

ttgaagatcg taaagaaatt ctcccatatt ttcttcaagt acttttgcca tatgactact 6300

catttgttaa tacacaattt attgaatatt ttgtttttta tatatataat gatttgagat 6360

ggcacctttg ccagattcta aattcccatg tgtatttttg gtgttcttct ggattttctt 6420

tattttctgt gttattctat taattcacca ataacgttct atttcaatta ttaaggatct 6480

tcttcttcat agttttcctg gttatttttt ttcacataat tttataatca gcttgtctgt 6540

taataaattc ctgttgataa tttttagtgg ggtctcatta aatttatgaa ttatagtata 6600

gagaaaactg ataactttat gatgaggagt cttgctaacc aaatcctggt atgtctttcc 6660

atttattcaa gtctcttttg tgtctgttag gacagtatta aagttgtctt catttaggtg 6720

taacacattt cttgtgaaga ttgttcctag gtattttatc tttggtaatt tggtaattgc 6780

ttttgaaaat tgggccttac tttacattta aaaaaaaaaa tcttgctttt gttagtgtat 6840

gctacaatgc aagtggcagg aatacataca gaactgtttt cttttgtacc tttttttttt 6900

ttttttttaa gacagagtct tgctctgttg cccaggctgg agtgcagtag cgcaatctcg 6960
gcttactgca agctccacct cccaggttca tgccattctc ctgcctcagc ctcccgagta 7020
gctgggacta caagcgcccg ccaccatgcc tggctaattt tttttttgta ttttttagtg 7080
gagacggggt ttcaccatgt tagccaggat ggtctcgatc tcctgacctc gtgatccacc 7140
tgccttggcc tcccagagtg ctgggattac aggcttgagc cactgcaccc agcctgtacc 7200
ttttttttta attatgatta ttattatttt ttgagacaga gtcttgctct gttgcccagg 7260
ctggagtgca gtggcataac ctcggctcac tgcaagcttc atctcccggt ttcaagtgat 7320
tctcctgcct cagcctccca agtagctgag attacagaca tgtaccacca cacgcagcta 7380
atttttgtat atttagtaga gatggggttt cactgtgttg ggcagattgg tcttgaactc 7440
ctgacctcaa gtgatccgcc tgccttagcc tcccaaagtg ctgggattac aggtgtgagc 7500
cactgtgccc agcctctttt gtgccttttt gaattaatca tagaataatt tctagaatag 7560
tcattgattt cttttgtttc tttgtaagac actatatgca gatattcata ctagatggct 7620
catttacatc tatagtacaa taaattagtc ctgttttaaa tggaataact tttgaactct 7680
cagcaggaaa tttatcctct atttttgtta aataaaaagc attggtgcac ttatacacta 7740
ttacagaaaa aagccagtga ttataaaact taaaaatgta tttcattcaa aattcagttc 7800
cagttgttac caatattttt acataaccta tgtatagtca tgggtcactt aacagtgggg 7860
atatgttctg agaaatatgc ccttaggcaa tttcattgtt gtacaatcat catggagtgt 7920
aattacacaa acagagagag tatggcttac tacacaccta ggctccaaac ctgcaccttg 7980
ttttactgaa ctgaatattg taggcaatta taacacaatg gtaagtattt atgtgtctga 8040
acatatttaa gaagaggtac agtagaagat ttaaaaggta cagtaaaaat acaatataaa 8100
agataaaaaa tagtacacct gtatagggca tttactatga aaggagcttg caagactgga 8160
acttgctggg tgagtcagtg aatggtaagt gaatgtgaag gcctaggaca ttactgtaca 8220
ctactgcaga ctttgtaaat actgtacacc taagctacac tgaatttatt taaaaagtaa 8280
ttgtgctatg acattacaag gctatgatgt tactaggcaa caggaatttg tcagcttcag 8340
cttcattatg ggaccactgt cgtatgtgct gcctgcattg actaaaatgt cattatgcgg 8400
ttgcataact gtatttattg ttatgttctt ttttggactg tttttatatt ttggattata 8460
ctttggaaaa attactgagc ctctcttgag ctccagtttc cttctctttg agatgggaca 8520
aataatagga cctacaccat agggtgctac ttcactggat tgtgtgggag agtacatgcg 8580
gagccctagt atgttctttc ctttacttgt acaactcacc aggatgacag ctgtcatgaa 8640
taaagagtgt gctttgccct gcccaactac aaggtcccac taccagttga ggagtatact 8700
ctgattcatt gtgaaaatgc caacaagggt ggttatccct tgctggaaag caggttaaaa 8760
ggattttcat aattctgtac ctcttctctt tcctccaggc ctactctgtg aagagaacat 8820

tgatgactgt gcccggggtc cccattgcct taatggtggt cagtgcatgg ataggattgg 8880

aggctacagt tgtcgctgct tgcctggctt tgctggggag cgttgtgagg gagacatcaa 8940

cgagtgcctc tccaacccct gcagctctga gggcagcctg gactgtatac agctcaccaa 9000

tgactacctg tgtgtttgcc gtagtgcctt tactggtgag aagctctcta gcctcctggc 9060

cactctgcct tggaggatgg ttttagtgaa caaattgggc cctgtgagtt gtcccagacc 9120

caagtgaagc ttttcttata taagaaaaac aacagcagct taatttttta aagccagttt 9180

tttcttagtc tgtactttat ggatgttatg atcagtgtct acctttaatt tttcctaaga 9240

gattcttttg ttgtgcattg attatatcat gctatggcag gaatattttt tacaatgtat 9300

attgaaagta gttgaacaaa ataacatttt ttaatagtgg caaaatatcc aaaacctaaa 9360

atttactatc tgaatcattt taaagtgtac agtgtacagt tcagtggcat tgtttagtac 9420

attgacattg ttgttctact atcaccccca ttcattcaca gaactctttt catctgaaca 9480

aaataacatt ttgttttaag gaaccttacg tctaactccg gtttcttcac cctgcctcct 9540

ttgacatgtg atggcttgaa ttactgggta gttcctgcca ccatgatctt tccaccccta 9600

ggactcagag aagcttagga ccctgcttag atacttaaag ggggcaggtc caaattcaag 9660

tgtgtttaac caaagatcag ttgatgtggg tatgaaatga aaagttgcag tggtcacaag 9720

taatgccttg cccagtaaag gcttggccat gttcctactc tgttcccaag cttctttctt 9780

tcatcacagg gcctgaagat gaccaagctc tgcctcagaa taggggtctt atcacaagaa 9840

cttactagtt acttctggag agggcagcat ggtgactttt gggaactaaa atagttgttt 9900

gtatccagat gtggcaggat actcagattt gaaatttatc atgcatttcc tggggtctgt 9960

agttgtattt cttatatgga gggtgtgatc agattcctta ggagtcagaa aataggcttt 10020

aatagtctgt ttacaaacaa ttcatcaagc tgaactctgc tacatcctga gcccatctcc 10080

agccatcttt ccttccaact ttaaagatat gctaccttac aatcttggaa ttttgtaaag 10140

aatgggaggg attctgcaat atggaagctc cctggataca ctaacatcaa aaaagaaata 10200

gtccattgtg aacataatag ctattgaggc aatgtggaac agaataagta tgaataatct 10260

gtctacagcc tcgtattggc caaactgcac tttgaaagcc aaagcatcaa aactggctcc 10320

tgtttctttc attgcccctg aggctttttt ctctggaaac acaagcattt gcaaagtaag 10380

ggggaagcag gagaaggaag gaatggagac cactttgaag tagtatccca cagccaggaa 10440

gtagtgtttg tgcattttcc acatttgtga atgaaaaaaa aaaaatccct tgaatacatt 10500

gagcacagtt attgtgggat gctgttgcag agataaacag caagggactg gtggtccacc 10560

aagctgtgtc catgtggtgg ctgctcttgg ttagcggctg ctgctgtgat ttgaagagac 10620

aaacatttat acctcttgct gcttgatctc gttggccaaa aacattacta tttttatttc 10680

atttttaaag ctacctcttc tttcccaagt gaccaccggc caaatggggc ctatctcctg 10740
ctgttggcca ggagtcccag ctgtgccctg cgtctcctct gtggttccag ttgcctcgtt 10800
ctttatcttg gagcagtggc ccccaggggc cttccgtttt agaactattc ctagaggaaa 10860
ctaactctag gaaaattaag ctgtgtttta aggaatacga gttaggttac agaaaggcca 10920
aaaagtgatt gaaatgaggt atcacagatg aaaattctct ataccgcaaa tcaccattta 10980
aatgtattgg cctggtgtag cctctcctat gaagtagata acattaacaa aatgcagtga 11040
atagtttttc aactgttttt ttgcaactta tgaaagctaa aatgacttca aaaagctgaa 11100
gtgtgatttc tctttttttg atgggattta agtcactttt ctcacccacc ctgttgtgtg 11160
agttggtact tactgtgtag gcaagacaga gtaagggact ggcctgagct ggtgacaggc 11220
caatcatgtt gagatgcagt gagtgttttc tttctatgct cagattacca caggcctaga 11280
actgaactct ctcacacttg cttcacctat ggctagttcc catcccagca aagtttgttg 11340
agtaacttgc agtgactact tctgcctttt aaaacctttt ttatttttta ttttctgagg 11400
ccggcactgt gaaaccttcg tcgatgtgtg tccccagatg ccctgcctga atggagggac 11460
ttgtgctgtg gccagtaaca tgcctgatgg tttcatttgc cgttgtcccc cggtaagtgc 11520
cctgttgctc atctaagaag gggcattgtc tcttccatac aatctgtgaa cagaactgag 11580
attaaacctg gttttaccaa tcttcgaaat gggaagattt ctcaatttta gctctttcag 11640
aggaaagtgc ttgggttttt ccatttaaag ctgtttacaa gtgacaaaac aaaacaaaaa 11700
caaataaaag ctgtttagac actcccagaa acagtgagct tcccaatcca tgactgtcag 11760
tcttaagaaa aactaaggga aaaccacaaa aagtggtgga gtgccccctt gaaaataggg 11820
ctgatgtgtg gggcttcttg gccccaggct ggagggtaat ctcactgccc agattgggcc 11880
agagttggtg gaaatgctgc tcttacattc tttagcagga accatttgga agcaagttgt 11940
ctcttaaagt agagggaagc tcaattttag ctatttgcta tacccattct tttatcactg 12000
ccagaggttg aaaccttccc aaaacaagag atccctatgc ctcgggagat accaaaagga 12060
aacctgtcta gtaaccttgg cttgggctga acgggaagta cagaagggag tgagatgtgg 12120
aggttttcta gaaacctcaa acttccaata tgactgtttt cctaccaatg ccatttttat 12180
ttcctcactg atttccacag ggattttccg gggcaaggtg ccagagcagc tgtggacaag 12240
tgaaatgtag gaagggggag cagtgtgtgc acaccgcctc tggaccccgc tgcttctgcc 12300
ccagtccccg ggactgcgag tcaggctgtg ccagtagccc ctgccagcac gggggcagct 12360
gccaccctca gcgccagcct ccttattact cctgccagtg tgccccacca ttctcgggta 12420
gccgctgtga actctacacg gcacccccca gcacccctcc tgccacctgt ctgagccagt 12480
attgtgccga caaagctcgg gatggcgtct gtgatgaggc ctgcaacagc catgcctgcc 12540
agtgggatgg gggtgactgt tctctcacca tggagaaccc ctgggccaac tgctcctccc 12600

cacttccctg ctgggattat atcaacaacc agtgtgatga gctgtgcaac acggtcgagt 12660

gcctgtttga caactttgaa tgccagggga acagcaagac atgcaagtaa gggcttcacg 12720

ttttccagaa ctgaagggga ctgtcactaa cacacttcag tttctcgttc ttgctcctaa 12780

ccactccagt gcttcagcct aagctgcctc tgcttttcag atggtgccag ccttgtccca 12840

tcgcacactg ccctgtgctc tcccttgctg acaatgggcc aggaatgctc attgcataat 12900

gaccctctct ctctgcctat ctttgccttc tcacctgctg ccttcgtgac ctcctggggg 12960

atataacttt tggaagtggt gtttcccact tcaagcagcc tctcacataa cctgcctgca 13020

ggatggatgg aaaggtctgt atctctctct tcaccccatt cctttcccta agactgtttt 13080

tccccatcta gatttatcag gaagcatgaa ttagattaag tctgtgaacc ctcccggtct 13140

taattgtata ggatgctcta tggtttactt aggaactttc ctattaacta gccattccat 13200

ataatttaat cccaattaat tctcagcaac tcacgaggca taggtattac taacattctt 13260

tcataggtga gagaacttag gcccaagggt taaagaactt gccaaagcaa gttgtttctt 13320

tgactcctaa tcttctgatt gagctagcat gaaaagttgt accaaatagt ataaatatca 13380

aggggtaggg gatgaggaga ggagagtata gatggaatag tttgtacatc atttaggttt 13440

gggggttcta attccttttc caaatattga aagagcctgt acaagtaata tctggtgtgc 13500

tataaggaca tacccatctg cctttacctt ctgtagttgg gattcactct tgaaagggaa 13560

agaaatgggt catgttaata ttgttctctg tatggcaact ctcaatggtt cagggccttg 13620

tatcaagttt ctagaggcat ctttcacttc ctaaagcccg ttgttacctt actgggggca 13680

atggatttct ctaaagtaga ggaagcatga accaggttga cttttgtttc agatatgcag 13740

ctttgtttga aaacctgact taacaggctt gtaaactgcc tgggagcccc taagctcctg 13800

cagcatagtg agtacaatgt tcttcattta ggaagtactc aacaaggatg agtggcatag 13860

gcatcaggga taggagatgc aaagaaggtt ttgtggggca tttgggtcct gggtgtgggc 13920

cacagaaaat gggtaggaaa atccacagta gttccaaatg aaaaagcaaa tagagctcca 13980

gtctaatctg agctcttttg cctgttctct gcttcccctt acctaggtat gacaaatact 14040

gtgcagacca cttcaaagac aaccactgtg accaggggtg caacagtgag gagtgtggtt 14100

gggatgggct ggactgtgct gctgaccaac ctgagaacct ggcagaaggt accctggtta 14160

ttgtggtatt gatgccacct gaacaactgc tccaggatgc tcgcagcttc ttgcgggcac 14220

tgggtaccct gctccacacc aacctgcgca ttaagcggga ctcccagggg gaactcatgg 14280

tgtaccccta ttatggtgag aagtcagctg ctatgaagaa acagaggatg acacgcagat 14340

cccttcctgg tgaacaagaa caggaggtgg ctgggtaggt gtttggtttc tgaacttcaa 14400

ggctaatttt atgaagccta gcactttgac atatagcaca agtcataact caacctatag 14460

aaatgcaaag atcagaaaaa tagaatggcc caccacttaa gataaacccg ttaccccagt 14520

acttacgttt aaatgtaaag atatgccttc gtcttggctg aaaagtgggc aggggtacac 14580

aggaggcaga atgtctggaa ggcctcctca gatgccagaa tccataacct tttgccagag 14640

ttgcctcaaa tccctttatt agtgacattc ctttttacat taatcattgc cttattgtta 14700

aggcccttct ttagaatata tgtctctttg acacaggtaa tgatcttcag ttgctctgtc 14760

attcacacct cactctttgt tggtatattg tcaaactttc aaccctccag ctagctgaga 14820

taccaatgca gagacaaagg aaaataagga ctaaaaacac acagggatgt atgtttagag 14880

tagcaccaag taccactgag taggtatgaa agagtggaag aagaccactg aaaccttcgg 14940

gaggaaagca cagcttctca ctttgtcatg cattgcctca gccagaaggc tttggtcagc 15000

cagactaagg aaggtgggac atcctctatg tgggaattag tgtttaagat ctcaagggac 15060

agtattctaa ttcctgagat atgtcttaag gcagcatccc agaggtgggg acctgatgct 15120

gaaggggagg aagggaggct cctgtcaccc tttatgacat gttctgcctg acctgcactc 15180

ttctgtttta cccccatctc tcctcctccg cttctccctt ggcatcccac agctctaaag 15240

tctttctgga aattgacaac cgccagtgtg ttcaagactc agaccactgc ttcaagaaca 15300

cggatgcagc agcagctctc ctggcctctc acgccataca ggggaccctg tcataccctc 15360

ttgtgtctgt cgtcagtgag tggcactggt gtaaagggga aaggtggcgg gggaagagtg 15420

tcaattgggg gaacaatttt cattatgtag ccctctattt tttcaggact gttgaggtca 15480

aagttgccct ctaaatgaat ctaatgctga cattgagagg ttaatgtttt attcagcact 15540

ttccctgtct ctgtggactt tcaggtgaat ccctgactcc agaacgcact cagctcctct 15600

atctccttgc tgttgctgtt gtcatcattc tgtttattat tctgctgggg gtaatc 15656

<210> 2
<211> 15502
<212> DNA
<213> Homo sapiens

<220>
<223> NOTCH2 starting downstream of exon 20 and ending upstream of
      START2

<400> 2
gtaagctttg ctcccttggg ctcaggtcat tcactgcagt attgactgtc cagtggcatt 60

tagagaaggt atcatttttt tgtggaataa gagggaaggg cagatagtag tgggtgggca 120

gggctctggg gaaagggctc aagacagaag gtgtaagggt agaggaatag gttcagagat 180

tcggagaaga atgtatttga aatgaaacta aggtatattc cactttcagg gtttattttt 240

aatatgcatc tggtactgag ggttgtgaag aactggctaa gaataagatt ggatgtgtca 300

gccagtttag atttacttgc tcattggttg atttgaaccc catgtgtcac atgttcttct 360

atgacatttc tccctcactg ggtttcccag gtgtgcttgt tgaacacttg tgccagcact 420

caggtgtctg catcaatgct ggcaacacgc attactgtca gtgccccctg ggctatactg 480

ggagctactg tgaggagcaa ctcgatgagt gtgcgtccaa cccctgccag cacggggcaa 540

catgcagtga cttcattggt ggatacagat gcgaggtgag gacactgagc aagcagaggt 600

catggacctc ccaggagatt gtacaactgt ttgagcactg atatattgaa tttagctgct 660

tgtatcttat attgatagtt tatagatttt taccaatccc cgtcataatg tttaccactg 720

tttctttcag cctctctgtg caataagtta acaggtccct agtatgatat ttttataggc 780

gtctggaacc atcccagctc cttaggcttg cttttaggat tcagagccaa gagtcactac 840

cagagtgcat tttacaggcc cacccaacat agcagtccac acttccctga atagcagctg 900

agataggaaa aactgctttg tggagtgtcc ctttggcccc tgtcaccttt gtatttagtc 960

attaatcagt caactagcaa ttttctctca aaaaaaaaaa atgttgctca tcctgttccc 1020

catagcatca tgctaaacgt tacatgtata tatttgagga agtatgtgtc ttcaaggtac 1080

atgtcactat tttaatgtct gggctgataa tggttagagc aagcaagata ttagcaaaat 1140

gcctgagaca ttaatttgat accaattcaa accatcaagc aacactgaga aaagctttgc 1200

ttctctattg ttatataaat gagccaacga tagcttctgt attttggccc ctaggttgtt 1260

tatttcttca cagcaggctg gggctgggtc tcattagctc aaaagcctac cagtgccaaa 1320

cccaaatttt gacacatcta gttgttttaa acatagcccc caaataagca gatttttagc 1380

tatttagagc ttgcctgctt tgcatagcct gtaaaactgc agccaaaatc tgctagccat 1440

agtataagta ggacaagata taccctgggg ccatacagac cccaggctga tgctggcctt 1500

tagagctctc tgacccagag attcctgcca ctggctgttg agtgacatca cctagactcc 1560

ttctctgatc cttctttccc agagtttact tgctgtcttc cccttactca cctacccctc 1620

tggaaggtct catgctattt gggacttctc ccaggtgcaa acctgtcaaa gtgtccaaat 1680

aaagccacct cacagccata tcttttttct tggtcagccc tgaaatcctt ccaaccccta 1740

cagccaccac agccagccag ccagccccac ctccctctac ccctaagcct atctcaacca 1800

gtttctcaca catagatcca atttcctaaa cctggagatc aataattgca tgtctgtact 1860

accatccaca tatgcctttt gaccgagtca tagggtcctc atcacatacg gaggaatgtg 1920

tattttaaag atgaggaaac ctgtacccag gaggaacaaa aggtttgcac acaagtcaaa 1980

tctgaaacat ccttcattgt attcagtcct tgccttccct ttaaagtgcc tttttcaact 2040

gaaagaggag taggtaaatg ccagtaagct gcaggttttc tgtccctgca tagaatgatt 2100

agcagagagt gtgatggttc cagagaaaac atacaaaagc taaattagga ggtgggagac 2160

agtcacagaa atgttccaga tctcactagc tctcatggtc tgtttccagt aatggtccaa 2220

tatttcttgt cctttttgtt tcctcatggc agtgtgtccc aggctatcag ggtgtcaact 2280

gtgagtatga agtggatgag tgccagaatc agccctgcca gaatggaggc acctgtattg 2340

accttgtgaa ccatttcaag tgctcttgcc caccaggcac tcggggtatg aaatcatcct 2400

tatccatttt ccatccaggg cattgtctta agttataaat ccattcttag tgttcagggg 2460

attttataaa attaaagata ggaagactag cttcattcca agcatttagt tctacatcct 2520

agtaattcaa gccattttat tctcccatct cttgctagct ctgatgttgt ggtttatgtt 2580

gtcagtttta tctggttgtt tggcatcttg atattccatg aaacacagaa tatggaaggg 2640

atacaacatt agcataacat taaaaaatta gcctggtcag taagatttct tgttgcttca 2700

cagaaaagca actaatggcc tctaaaataa acaatttaca tttacctgct tttgtatttt 2760

tttaattagc caagtcttac ctctataagg tgggggaatg aatgaaatga aaatatctgc 2820

ctattttcat agcttcaggg gaggatactg atggtgtaag gttctacctt aggatgcagt 2880

aaggcctgat gagagatgca accctggact gttgagtgat ggggccatca ttcttctgtg 2940

ggcattgcca ctgcttttga actgagtggt atggccatca ttctgctgtg gacattgaca 3000

ctgcttttga accatccctt ggaaagagct ttgagtattg tttgttagaa gggatgtagt 3060

tatgctgggg cctactctga gaaggctgtg aaaaaaatgt aaaaggccat ccacactggt 3120

gagaaattca tccttgttag tgtcaaagag aatagaatgc cagagaagta tttgtattgc 3180

cttagagctt tgtgtcagta aagtttgata atttgccacc ccaggtatca gagtttgact 3240

aaattaaaat aggagagtac cctagtagta ggctgattat taaggaatga ggttaatggg 3300

aagaagaaaa ctaaggagaa acagtcttta agtatttgaa gaattgtcct gtaaaaagga 3360

gaattgttct ttattctaga aatcagtggt tgagtagaaa ttaattccag ttcattgtta 3420

gtttgttcct ttgagaaact gtactggtta ggacaagcta cgattctggt gatagctgtt 3480

atcgtagttc atttatgttg ctataaaaga atacctgaga ctaagtaatt tatttttcga 3540

aagaggtata tttggctcgc agttctatag gctgtacagg aagcacggtg ccagcatctg 3600

cctctggtgt gagcgtcagg ctgtttccac tcatggtgaa aagtgaagaa gatccaggga 3660

cccaaacacc tcccattagg ccccacctcc aacattggag atcacatttt aacatgtggt 3720

ttggtgcagt caaacttcaa gctatagcag ctaattaaac ctcaaatctc agagcttaat 3780

ataacactga ttttgtcact caaataaagt cgaatatgtg ttgggctacc tttctctgtg 3840

ttttagatat gtggtttgaa acacctggcc tccaaggcct ccacagtaca gggaagaggg 3900

acagacttga agggtcatac agcagcttcc actagctcag tctggaagca acacatatca 3960

ctatccctcg cagtatattg accggaatta ttaaatggtc tctgtgactt caagggaggc 4020

tgggaaatat aggagagtac atgagtattc atcaaactct aaaatcacta ctataccatg 4080

aacttttaat taataagaca tgtccagtcc tagaatggat catttcagaa ggaaacaaaa 4140

tttttgtctc ttctaaccat atggtaaaga tggcttagaa tactagtttt gatattatca 4200

ttgacagtct ttttctttca gcactttgaa tatatcatcc cactgctttc tgatatccat 4260

ggtttttaat gagaaatatg ctgttaatct tattgaggtt cccttatatg tgatacattg 4320

cttctttctt gatgccttca agattcttta tcttttgata gtttgattat gatgtatgta 4380

ggtatgaatt tctttgagtt tatcctactt acagttcctt ggattatttt actgtgtgtg 4440

cttgtttagg tatgtggaac agtatttttt tatcctgact taacttctct ggccattggt 4500

aaacattcca ggaaactctc aagtcccata accccattgt tagagtcatc acaatgtgtg 4560

ttttggtcac catatttcta agccttagat ctttgcctgc atgtcccacc agtttcccag 4620

gtcaagggct aaatgtaaca gttctgcctc ttcctactac tttgcttcag gctgaggttg 4680

agttctctct cagttgatag atctgtttat ttctggtggt tccattctga tggggaaata 4740

tgcatttatg ctcattttac tagcaatccc taaatatcaa atattctttg agacagggag 4800

catatactag attttgtttg tttttacttt tttgttttca atttactcta tgtataaggt 4860

gatatatgca tatatgcata tagaccaaaa gtgtattcac acatagaaaa gtatacagtg 4920

aaaatacttc tcattcccat ctctcttctg gtgggcaaat tttccctctc ctgcaaacat 4980

gtaatcgcca cttttggttg tttgtgtttc tttctagaat ttttcaaaaa ctgtatcctt 5040

cagaatttct ttatgtagag agaagccaaa tgcaaagata ttttatttcc tttacttttt 5100

actctgaaga tagaatatta gacacatttt ttgcacttga ttttttttta cttaattgga 5160

gatattttca tatcaaaata tagaacttcc tctctctctc tctgagatag atagatagat 5220

agatagatag atagatagat agatagatat ttgtttgttt gtttgttttg agatggagtt 5280

ttgctcttgt tgcccaggct ggagtgcaat ggtatgatct cagctcaccg caacctccgc 5340

ctcctgggtt cgagtgattt tcctgcctca gcgccctgag tagctgggat tacaggcatg 5400

tgccaccatg cctggctgat tttgtatttt taatagagac agggtttctc catgttggtc 5460

aggctggtct tgaactcctg gcctcaagca atccacccgc ctcagcctcc caaagtgctg 5520

ggatgacagg cgtgagccac cacacccagc ctgaacttcc tcaatatttt ttatagctac 5580

agaattccct tttacaggca taccatattt taaccagtgc catcatgata gatatttcag 5640

ttatcttgca ggcatataag tatattggtg aaataaattt tcagaaatag aatcgctgga 5700

ttggataata tgtgcattta tcattttaat cagtgattgc agtatatcag catattggct 5760

ttttcttaag gtggagaata tattttcatt taagaatcat tttcttttga ttttttttgc 5820

agtttctttg tttatatata tatatatgta tatatacata tatatatata tacacacata 5880

tatctttttt cccacattaa gttgttgatg taatgttttg tggaaattgg ctatgatata 5940

aaaaaacaaa tatattccca gtattttttt atcttttttc tttatagtga tttttagtgc 6000

acacttttac tttttaatca agttgaattt atcactttta aaatggcttc tggattttga 6060

gttgtagtta gcaacatcat atacacttga agatcgtaaa gaaattctcc catattttct 6120

tcaagtactt ttgccatatg actactcatt tgttaataca caatttattg aatattttgt 6180

tttttatata tataatgatt tgagatggca cctttgccag attctaaatt cccatgtgta 6240

tttttggtgt tcttctggat tttctttatt ttctgtgtta ttctattaat tcaccaataa 6300

cgttctattt caattattaa ggatcttctt cttcatagtt ttcctggtta ttttttttca 6360

cataatttta taatcagctt gtctgttaat aaattcctgt tgataatttt tagtggggtc 6420

tcattaaatt tatgaattat agtatagaga aaactgataa ctttatgatg aggagtcttg 6480

ctaaccaaat cctggtatgt ctttccattt attcaagtct cttttgtgtc tgttaggaca 6540

gtattaaagt tgtcttcatt taggtgtaac acatttcttg tgaagattgt tcctaggtat 6600

tttatctttg gtaatttggt aattgctttt gaaaattggg ccttacttta catttaaaaa 6660

aaaaaatctt gcttttgtta gtgtatgcta caatgcaagt ggcaggaata catacagaac 6720

tgttttcttt tgtacctttt tttttttttt ttttaagaca gagtcttgct ctgttgccca 6780

ggctggagtg cagtagcgca atctcggctt actgcaagct ccacctccca ggttcatgcc 6840

attctcctgc ctcagcctcc cgagtagctg ggactacaag cgcccgccac catgcctggc 6900

taattttttt tttgtatttt ttagtggaga cggggtttca ccatgttagc caggatggtc 6960

tcgatctcct gacctcgtga tccacctgcc ttggcctccc agagtgctgg gattacaggc 7020

ttgagccact gcacccagcc tgtacctttt tttttaatta tgattattat tattttttga 7080

gacagagtct tgctctgttg cccaggctgg agtgcagtgg cataacctcg gctcactgca 7140

agcttcatct cccggtttca agtgattctc ctgcctcagc ctcccaagta gctgagatta 7200

cagacatgta ccaccacacg cagctaattt ttgtatattt agtagagatg gggtttcact 7260

gtgttgggca gattggtctt gaactcctga cctcaagtga tccgcctgcc ttagcctccc 7320

aaagtgctgg gattacaggt gtgagccact gtgcccagcc tcttttgtgc ctttttgaat 7380

taatcataga ataatttcta gaatagtcat tgatttcttt tgtttctttg taagacacta 7440

tatgcagata ttcatactag atggctcatt tacatctata gtacaataaa ttagtcctgt 7500

tttaaatgga ataacttttg aactctcagc aggaaattta tcctctattt ttgttaaata 7560

aaaagcattg gtgcacttat acactattac agaaaaaagc cagtgattat aaaacttaaa 7620

aatgtatttc attcaaaatt cagttccagt tgttaccaat atttttacat aacctatgta 7680

tagtcatggg tcacttaaca gtggggatat gttctgagaa atatgccctt aggcaatttc 7740

attgttgtac aatcatcatg gagtgtaatt acacaaacag agagagtatg gcttactaca 7800

cacctaggct ccaaacctgc accttgtttt actgaactga atattgtagg caattataac 7860

acaatggtaa gtatttatgt gtctgaacat atttaagaag aggtacagta gaagatttaa 7920

aaggtacagt aaaaatacaa tataaaagat aaaaaatagt acacctgtat agggcattta 7980

ctatgaaagg agcttgcaag actggaactt gctgggtgag tcagtgaatg gtaagtgaat 8040

gtgaaggcct aggacattac tgtacactac tgcagacttt gtaaatactg tacacctaag 8100

ctacactgaa tttatttaaa aagtaattgt gctatgacat tacaaggcta tgatgttact 8160

aggcaacagg aatttgtcag cttcagcttc attatgggac cactgtcgta tgtgctgcct 8220

gcattgacta aaatgtcatt atgcggttgc ataactgtat ttattgttat gttctttttt 8280

ggactgtttt tatattttgg attatacttt ggaaaaatta ctgagcctct cttgagctcc 8340

agtttccttc tctttgagat gggacaaata ataggaccta caccataggg tgctacttca 8400

ctggattgtg tgggagagta catgcggagc cctagtatgt tctttccttt acttgtacaa 8460

ctcaccagga tgacagctgt catgaataaa gagtgtgctt tgccctgccc aactacaagg 8520

tcccactacc agttgaggag tatactctga ttcattgtga aaatgccaac aagggtggtt 8580

atcccttgct ggaaagcagg ttaaaaggat tttcataatt ctgtacctct tctctttcct 8640

ccaggcctac tctgtgaaga gaacattgat gactgtgccc ggggtcccca ttgccttaat 8700

ggtggtcagt gcatggatag gattggaggc tacagttgtc gctgcttgcc tggctttgct 8760

ggggagcgtt gtgagggaga catcaacgag tgcctctcca acccctgcag ctctgagggc 8820

agcctggact gtatacagct caccaatgac tacctgtgtg tttgccgtag tgcctttact 8880

ggtgagaagc tctctagcct cctggccact ctgccttgga ggatggtttt agtgaacaaa 8940

ttgggccctg tgagttgtcc cagacccaag tgaagctttt cttatataag aaaaacaaca 9000

gcagcttaat tttttaaagc cagttttttc ttagtctgta ctttatggat gttatgatca 9060

gtgtctacct ttaatttttc ctaagagatt cttttgttgt gcattgatta tatcatgcta 9120

tggcaggaat attttttaca atgtatattg aaagtagttg aacaaaataa cattttttaa 9180

tagtggcaaa atatccaaaa cctaaaattt actatctgaa tcattttaaa gtgtacagtg 9240

tacagttcag tggcattgtt tagtacattg acattgttgt tctactatca cccccattca 9300

ttcacagaac tcttttcatc tgaacaaaat aacattttgt tttaaggaac cttacgtcta 9360

actccggttt cttcaccctg cctcctttga catgtgatgg cttgaattac tgggtagttc 9420

ctgccaccat gatctttcca cccctaggac tcagagaagc ttaggaccct gcttagatac 9480

ttaaaggggg caggtccaaa ttcaagtgtg tttaaccaaa gatcagttga tgtgggtatg 9540

aaatgaaaag ttgcagtggt cacaagtaat gccttgccca gtaaaggctt ggccatgttc 9600

ctactctgtt cccaagcttc tttctttcat cacagggcct gaagatgacc aagctctgcc 9660

tcagaatagg ggtcttatca caagaactta ctagttactt ctggagaggg cagcatggtg 9720

acttttggga actaaaatag ttgtttgtat ccagatgtgg caggatactc agatttgaaa 9780

tttatcatgc atttcctggg gtctgtagtt gtatttctta tatggagggt gtgatcagat 9840

tccttaggag tcagaaaata ggctttaata gtctgtttac aaacaattca tcaagctgaa 9900

ctctgctaca tcctgagccc atctccagcc atctttcctt ccaactttaa agatatgcta 9960

ccttacaatc ttggaatttt gtaaagaatg ggagggattc tgcaatatgg aagctccctg 10020

gatacactaa catcaaaaaa gaaatagtcc attgtgaaca taatagctat tgaggcaatg 10080

tggaacagaa taagtatgaa taatctgtct acagcctcgt attggccaaa ctgcactttg 10140

aaagccaaag catcaaaact ggctcctgtt tctttcattg cccctgaggc ttttttctct 10200

ggaaacacaa gcatttgcaa agtaaggggg aagcaggaga aggaaggaat ggagaccact 10260

ttgaagtagt atcccacagc caggaagtag tgtttgtgca ttttccacat ttgtgaatga 10320

aaaaaaaaaa atcccttgaa tacattgagc acagttattg tgggatgctg ttgcagagat 10380

aaacagcaag ggactggtgg tccaccaagc tgtgtccatg tggtggctgc tcttggttag 10440

cggctgctgc tgtgatttga agagacaaac atttatacct cttgctgctt gatctcgttg 10500

gccaaaaaca ttactatttt tatttcattt ttaaagctac ctcttctttc ccaagtgacc 10560

accggccaaa tggggcctat ctcctgctgt tggccaggag tcccagctgt gccctgcgtc 10620

tcctctgtgg ttccagttgc ctcgttcttt atcttggagc agtggccccc aggggccttc 10680

cgttttagaa ctattcctag aggaaactaa ctctaggaaa attaagctgt gttttaagga 10740

atacgagtta ggttacagaa aggccaaaaa gtgattgaaa tgaggtatca cagatgaaaa 10800

ttctctatac cgcaaatcac catttaaatg tattggcctg gtgtagcctc tcctatgaag 10860

tagataacat taacaaaatg cagtgaatag tttttcaact gtttttttgc aacttatgaa 10920

agctaaaatg acttcaaaaa gctgaagtgt gatttctctt tttttgatgg gatttaagtc 10980

acttttctca cccaccctgt tgtgtgagtt ggtacttact gtgtaggcaa gacagagtaa 11040

gggactggcc tgagctggtg acaggccaat catgttgaga tgcagtgagt gttttctttc 11100

tatgctcaga ttaccacagg cctagaactg aactctctca cacttgcttc acctatggct 11160

agttcccatc ccagcaaagt ttgttgagta acttgcagtg actacttctg ccttttaaaa 11220

ccttttttat tttttatttt ctgaggccgg cactgtgaaa ccttcgtcga tgtgtgtccc 11280

cagatgccct gcctgaatgg agggacttgt gctgtggcca gtaacatgcc tgatggtttc 11340

atttgccgtt gtcccccggt aagtgccctg ttgctcatct aagaaggggc attgtctctt 11400

ccatacaatc tgtgaacaga actgagatta aacctggttt taccaatctt cgaaatggga 11460

agatttctca attttagctc tttcagagga aagtgcttgg gtttttccat ttaaagctgt 11520

ttacaagtga caaaacaaaa caaaaacaaa taaaagctgt ttagacactc ccagaaacag 11580

tgagcttccc aatccatgac tgtcagtctt aagaaaaact aagggaaaac cacaaaaagt 11640

ggtggagtgc ccccttgaaa atagggctga tgtgtggggc ttcttggccc caggctggag 11700

ggtaatctca ctgcccagat tgggccagag ttggtggaaa tgctgctctt acattcttta 11760

gcaggaacca tttggaagca agttgtctct taaagtagag ggaagctcaa ttttagctat 11820

ttgctatacc cattctttta tcactgccag aggttgaaac cttcccaaaa caagagatcc 11880

ctatgcctcg ggagatacca aaaggaaacc tgtctagtaa ccttggcttg ggctgaacgg 11940

gaagtacaga agggagtgag atgtggaggt tttctagaaa cctcaaactt ccaatatgac 12000

tgttttccta ccaatgccat ttttatttcc tcactgattt ccacagggat tttccggggc 12060

aaggtgccag agcagctgtg gacaagtgaa atgtaggaag ggggagcagt gtgtgcacac 12120

cgcctctgga ccccgctgct tctgccccag tccccgggac tgcgagtcag gctgtgccag 12180

tagcccctgc cagcacgggg gcagctgcca ccctcagcgc cagcctcctt attactcctg 12240

ccagtgtgcc ccaccattct cgggtagccg ctgtgaactc tacacggcac cccccagcac 12300

ccctcctgcc acctgtctga gccagtattg tgccgacaaa gctcgggatg gcgtctgtga 12360

tgaggcctgc aacagccatg cctgccagtg ggatgggggt gactgttctc tcaccatgga 12420

gaacccctgg gccaactgct cctccccact tccctgctgg gattatatca acaaccagtg 12480

tgatgagctg tgcaacacgg tcgagtgcct gtttgacaac tttgaatgcc aggggaacag 12540

caagacatgc aagtaagggc ttcacgtttt ccagaactga aggggactgt cactaacaca 12600

cttcagtttc tcgttcttgc tcctaaccac tccagtgctt cagcctaagc tgcctctgct 12660

tttcagatgg tgccagcctt gtcccatcgc acactgccct gtgctctccc ttgctgacaa 12720

tgggccagga atgctcattg cataatgacc ctctctctct gcctatcttt gccttctcac 12780

ctgctgcctt cgtgacctcc tgggggatat aacttttgga agtggtgttt cccacttcaa 12840

gcagcctctc acataacctg cctgcaggat ggatggaaag gtctgtatct ctctcttcac 12900

cccattcctt tccctaagac tgtttttccc catctagatt tatcaggaag catgaattag 12960

attaagtctg tgaaccctcc cggtcttaat tgtataggat gctctatggt ttacttagga 13020

actttcctat taactagcca ttccatataa tttaatccca attaattctc agcaactcac 13080

gaggcatagg tattactaac attctttcat aggtgagaga acttaggccc aagggttaaa 13140

gaacttgcca aagcaagttg tttctttgac tcctaatctt ctgattgagc tagcatgaaa 13200

agttgtacca aatagtataa atatcaaggg gtaggggatg aggagaggag agtatagatg 13260

gaatagtttg tacatcattt aggtttgggg gttctaattc cttttccaaa tattgaaaga 13320

gcctgtacaa gtaatatctg gtgtgctata aggacatacc catctgcctt taccttctgt 13380

agttgggatt cactcttgaa agggaaagaa atgggtcatg ttaatattgt tctctgtatg 13440

gcaactctca atggttcagg gccttgtatc aagtttctag aggcatcttt cacttcctaa 13500

agcccgttgt taccttactg ggggcaatgg atttctctaa agtagaggaa gcatgaacca 13560

ggttgacttt tgtttcagat atgcagcttt gtttgaaaac ctgacttaac aggcttgtaa 13620

actgcctggg agcccctaag ctcctgcagc atagtgagta caatgttctt catttaggaa 13680

gtactcaaca aggatgagtg gcataggcat cagggatagg agatgcaaag aaggttttgt 13740

ggggcatttg ggtcctgggt gtgggccaca gaaaatgggt aggaaaatcc acagtagttc 13800

caaatgaaaa agcaaataga gctccagtct aatctgagct cttttgcctg ttctctgctt 13860

ccccttacct aggtatgaca aatactgtgc agaccacttc aaagacaacc actgtgacca 13920

ggggtgcaac agtgaggagt gtggttggga tgggctggac tgtgctgctg accaacctga 13980

gaacctggca gaaggtaccc tggttattgt ggtattgatg ccacctgaac aactgctcca 14040

ggatgctcgc agcttcttgc gggcactggg taccctgctc cacaccaacc tgcgcattaa 14100

gcgggactcc cagggggaac tcatggtgta cccctattat ggtgagaagt cagctgctat 14160

gaagaaacag aggatgacac gcagatccct tcctggtgaa caagaacagg aggtggctgg 14220

gtaggtgttt ggtttctgaa cttcaaggct aattttatga agcctagcac tttgacatat 14280

agcacaagtc ataactcaac ctatagaaat gcaaagatca gaaaaataga atggcccacc 14340

acttaagata aacccgttac cccagtactt acgtttaaat gtaaagatat gccttcgtct 14400

tggctgaaaa gtgggcaggg gtacacagga ggcagaatgt ctggaaggcc tcctcagatg 14460

ccagaatcca taaccttttg ccagagttgc ctcaaatccc tttattagtg acattccttt 14520

ttacattaat cattgcctta ttgttaaggc ccttctttag aatatatgtc tctttgacac 14580

aggtaatgat cttcagttgc tctgtcattc acacctcact ctttgttggt atattgtcaa 14640

actttcaacc ctccagctag ctgagatacc aatgcagaga caaaggaaaa taaggactaa 14700

aaacacacag ggatgtatgt ttagagtagc accaagtacc actgagtagg tatgaaagag 14760

tggaagaaga ccactgaaac cttcgggagg aaagcacagc ttctcacttt gtcatgcatt 14820

gcctcagcca gaaggctttg gtcagccaga ctaaggaagg tgggacatcc tctatgtggg 14880

aattagtgtt taagatctca agggacagta ttctaattcc tgagatatgt cttaaggcag 14940

catcccagag gtggggacct gatgctgaag ggaggaaggg gaggctcctg tcacccttta 15000

tgacatgttc tgcctgacct gcactcttct gttttacccc catctctcct cctccgcttc 15060

tcccttggca tcccacagct ctaaagtctt tctggaaatt gacaaccgcc agtgtgttca 15120

agactcagac cactgcttca agaacacgga tgcagcagca gctctcctgg cctctcacgc 15180

catacagggg accctgtcat accctcttgt gtctgtcgtc agtgagtggc actggtgtaa 15240

aggggaaagg tggcggggga agagtgtcaa ttgggggaac aattttcatt atgtagccct 15300

ctattttttc aggactgttg aggtcaaagt tgccctctaa atgaatctaa tgctgacatt 15360

gagaggttaa tgttttattc agcactttcc ctgtctctgt ggactttcag gtgaatccct 15420

gactccagaa cgcactcagc tcctctatct ccttgctgtt gctgttgtca tcattctgtt 15480

tattattctg ctgggggtaa tc                                          15502

<210> 3
<211> 2627
<212> DNA
<213> Homo sapiens

<220>
<223> NOTCH2 minimal deleted region

<400> 3
gccggcactg tgaaaccttc gtcgatgtgt gtccccagat gccctgcctg aatggaggga      60

cttgtgctgt ggccagtaac atgcctgatg gtttcatttg ccgttgtccc ccggtaagtg     120

ccctgttgct catctaagaa ggggcattgt ctcttccata caatctgtga acagaactga     180

gattaaacct ggttttacca atcttcgaaa tgggaagatt tctcaatttt agctctttca     240

gaggaaagtg cttgggtttt tccatttaaa gctgtttaca agtgacaaaa caaaacaaaa     300

acaaataaaa gctgtttaga cactcccaga aacagtgagc ttcccaatcc atgactgtca     360

gtcttaagaa aaactaaggg aaaaccacaa aaagtggtgg agtgccccct tgaaaatagg     420

gctgatgtgt ggggcttctt ggccccaggc tggagggtaa tctcactgcc cagattgggc     480

cagagttggt ggaaatgctg ctcttacatt ctttagcagg aaccatttgg aagcaagttg     540

tctcttaaag tagagggaag ctcaatttta gctatttgct atacccattc ttttatcact     600

gccagaggtt gaaaccttcc caaaacaaga gatccctatg cctcgggaga taccaaaagg     660

aaacctgtct agtaaccttg gcttgggctg aacgggaagt acagaaggga gtgagatgtg     720

gaggttttct agaaacctca aacttccaat atgactgttt tcctaccaat gccattttta     780

tttcctcact gatttccaca gggattttcc ggggcaaggt gccagagcag ctgtggacaa     840

gtgaaatgta ggaaggggga gcagtgtgtg cacaccgcct ctggaccccg ctgcttctgc     900

cccagtcccc gggactgcga gtcaggctgt gccagtagcc cctgccagca cgggggcagc     960

tgccaccctc agcgccagcc tccttattac tcctgccagt gtgccccacc attctcgggt    1020

agccgctgtg aactctacac ggcacccccc agcacccctc ctgccacctg tctgagccag    1080

tattgtgccg acaaagctcg ggatggcgtc tgtgatgagg cctgcaacag ccatgcctgc    1140

cagtgggatg ggggtgactg ttctctcacc atggagaacc cctgggccaa ctgctcctcc    1200

ccacttccct gctgggatta tatcaacaac cagtgtgatg agctgtgcaa cacggtcgag    1260

tgcctgtttg acaactttga atgccagggg aacagcaaga catgcaagta agggcttcac    1320

gttttccaga actgaagggg actgtcacta acacacttca gtttctcgtt cttgctccta    1380

accactccag tgcttcagcc taagctgcct ctgcttttca gatggtgcca gccttgtccc    1440

atcgcacact gccctgtgct ctcccttgct gacaatgggc caggaatgct cattgcataa    1500

tgaccctctc tctctgccta tctttgcctt ctcacctgct gccttcgtga cctcctgggg    1560

gatataactt ttggaagtgg tgtttcccac ttcaagcagc ctctcacata acctgcctgc 1620

aggatggatg gaaaggtctg tatctctctc ttcaccccat tcctttccct aagactgttt 1680

ttccccatct agatttatca ggaagcatga attagattaa gtctgtgaac cctcccggtc 1740

ttaattgtat aggatgctct atggtttact taggaacttt cctattaact agccattcca 1800

tataatttaa tcccaattaa ttctcagcaa ctcacgaggc ataggtatta ctaacattct 1860

ttcataggtg agagaactta ggcccaaggg ttaaagaact tgccaaagca agttgtttct 1920

ttgactccta atcttctgat tgagctagca tgaaaagttg taccaaatag tataaatatc 1980

aaggggtagg ggatgaggag aggagagtat agatggaata gtttgtacat catttaggtt 2040

tgggggttct aattcctttt ccaaatattg aaagagcctg tacaagtaat atctggtgtg 2100

ctataaggac atacccatct gcctttacct tctgtagttg ggattcactc ttgaaaggga 2160

aagaaatggg tcatgttaat attgttctct gtatggcaac tctcaatggt tcagggcctt 2220

gtatcaagtt tctagaggca tctttcactt cctaaagccc gttgttacct tactgggggc 2280

aatggatttc tctaaagtag aggaagcatg aaccaggttg acttttgttt cagatatgca 2340

gctttgtttg aaaacctgac ttaacaggct tgtaaactgc ctgggagccc ctaagctcct 2400

gcagcatagt gagtacaatg ttcttcattt aggaagtact caacaaggat gagtggcata 2460

ggcatcaggg ataggagatg caaagaaggt tttgtggggc atttgggtcc tgggtgtggg 2520

ccacagaaaa tgggtaggaa aatccacagt agttccaaat gaaaaagcaa atagagctcc 2580

agtctaatct gagctctttt gcctgttctc tgcttcccct tacctag 2627

<210> 4
<211> 4
<212> PRT
<213> Homo sapiens

<220>
<223> NOTCH2 fragment

<400> 4
Met Val Tyr Pro
1

<210> 5
<211> 4
<212> PRT
<213> Homo sapiens

<220>
<223> NOTCH2 fragment

<400> 5
Met Ala Lys Arg
1

<210> 6

<211> 1377
<212> DNA
<213> Homo sapiens

<220>
<223> NOTCH2 between START1 and START2

<400> 6
gtgtacccct attatggtga gaagtcagct gctatgaaga aacagaggat gacacgcaga      60

tcccttcctg gtgaacaaga acaggaggtg gctgggtagg tgtttggttt ctgaacttca     120

aggctaattt tatgaagcct agcactttga catatagcac aagtcataac tcaacctata     180

gaaatgcaaa gatcagaaaa atagaatggc ccaccactta agataaaccc gttaccccag     240

tacttacgtt taaatgtaaa gatatgcctt cgtcttggct gaaaagtggg caggggtaca     300

caggaggcag aatgtctgga aggcctcctc agatgccaga atccataacc ttttgccaga     360

gttgcctcaa atccctttat tagtgacatt cctttttaca ttaatcattg ccttattgtt     420

aaggcccttc tttagaatat atgtctcttt gacacaggta atgatcttca gttgctctgt     480

cattcacacc tcactctttg ttggtatatt gtcaaacttt caaccctcca gctagctgag     540

ataccaatgc agagacaaag gaaaataagg actaaaaaca cacagggatg tatgtttaga     600

gtagcaccaa gtaccactga gtaggtatga aagagtggaa gaagaccact gaaaccttcg     660

ggaggaaagc acagcttctc actttgtcat gcattgcctc agccagaagg ctttggtcag     720

ccagactaag gaaggtggga catcctctat gtgggaatta gtgtttaaga tctcaaggga     780

cagtattcta attcctgaga tatgtcttaa ggcagcatcc cagaggtggg gacctgatgc     840

tgaaggggag gaagggaggc tcctgtcacc ctttatgaca tgttctgcct gacctgcact     900

cttctgtttt acccccatct ctcctcctcc gcttctccct tggcatccca cagctctaaa     960

gtctttctgg aaattgacaa ccgccagtgt gttcaagact cagaccactg cttcaagaac    1020

acggatgcag cagcagctct cctggcctct cacgccatac aggggaccct gtcataccct    1080

cttgtgtctg tcgtcagtga gtggcactgg tgtaaagggg aaaggtggcg ggggaagagt    1140

gtcaattggg ggaacaattt tcattatgta gccctctatt ttttcaggac tgttgaggtc    1200

aaagttgccc tctaaatgaa tctaatgctg acattgagag gttaatgttt tattcagcac    1260

tttccctgtc tctgtggact ttcaggtgaa tccctgactc cagaacgcac tcagctcctc    1320

tatctccttg ctgttgctgt tgtcatcatt ctgtttatta ttctgctggg ggtaatc       1377


<210> 7
<211> 14276
<212> DNA
<213> Homo sapiens

<220>
<223> NOTCH2 exon 20 to last nucleotide upstream of START1

<400> 7
accctggtga atctctgcag tcggtctcca tgtaaaaaca aaggtacttg cgttcagaaa 60
aaagcagagt cccagtgcct atgtccatct ggatgggctg gtgcctattg tgacgtgccc 120
aatgtctctt gtgacatagc agcctccagg agaggtaagc tttgctccct tgggctcagg 180
tcattcactg cagtattgac tgtccagtgg catttagaga aggtatcatt tttttgtgga 240
ataagaggga agggcagata gtagtgggtg ggcagggctc tggggaaagg gctcaagaca 300
gaaggtgtaa gggtagagga ataggttcag agattcggag aagaatgtat ttgaaatgaa 360
actaaggtat attccacttt cagggtttat ttttaatatg catctggtac tgagggttgt 420
gaagaactgg ctaagaataa gattggatgt gtcagccagt ttagatttac ttgctcattg 480
gttgatttga accccatgtg tcacatgttc ttctatgaca tttctccctc actgggtttc 540
ccaggtgtgc ttgttgaaca cttgtgccag cactcaggtg tctgcatcaa tgctggcaac 600
acgcattact gtcagtgccc cctgggctat actgggagct actgtgagga gcaactcgat 660
gagtgtgcgt ccaacccctg ccagcacggg gcaacatgca gtgacttcat tggtggatac 720
agatgcgagg tgaggacact gagcaagcag aggtcatgga cctcccagga gattgtacaa 780
ctgtttgagc actgatatat tgaatttagc tgcttgtatc ttatattgat agtttataga 840
tttttaccaa tccccgtcat aatgtttacc actgtttctt tcagcctctc tgtgcaataa 900
gttaacaggt ccctagtatg atatttttat aggcgtctgg aaccatccca gctccttagg 960
cttgctttta ggattcagag ccaagagtca ctaccagagt gcattttaca ggcccaccca 1020
acatagcagt ccacacttcc ctgaatagca gctgagatag gaaaaactgc tttgtggagt 1080
gtccctttgg cccctgtcac ctttgtattt agtcattaat cagtcaacta gcaattttct 1140
ctcaaaaaaa aaaaatgttg ctcatcctgt tccccatagc atcatgctaa acgttacatg 1200
tatatatttg aggaagtatg tgtcttcaag gtacatgtca ctattttaat gtctgggctg 1260
ataatggtta gagcaagcaa gatattagca aaatgcctga gacattaatt tgataccaat 1320
tcaaaccatc aagcaacact gagaaaagct ttgcttctct attgttatat aaatgagcca 1380
acgatagctt ctgtattttg gcccctaggt tgtttatttc ttcacagcag gctggggctg 1440
ggtctcatta gctcaaaagc ctaccagtgc caaacccaaa ttttgacaca tctagttgtt 1500
ttaaacatag cccccaaata agcagatttt tagctattta gagcttgcct gctttgcata 1560
gcctgtaaaa ctgcagccaa aatctgctag ccatagtata agtaggacaa gatataccct 1620
ggggccatac agaccccagg ctgatgctgg cctttagagc tctctgaccc agagattcct 1680
gccactggct gttgagtgac atcacctaga ctccttctct gatccttctt tcccagagtt 1740
tacttgctgt cttcccctta ctcacctacc cctctggaag gtctcatgct atttgggact 1800
tctcccaggt gcaaacctgt caaagtgtcc aaataaagcc acctcacagc catatctttt 1860

ttcttggtca gccctgaaat ccttccaacc cctacagcca ccacagccag ccagccagcc 1920
ccacctccct ctacccctaa gcctatctca accagtttct cacacataga tccaatttcc 1980
taaacctgga gatcaataat tgcatgtctg tactaccatc cacatatgcc ttttgaccga 2040
gtcatagggt cctcatcaca tacggaggaa tgtgtatttt aaagatgagg aaacctgtac 2100
ccaggaggaa caaaaggttt gcacacaagt caaatctgaa acatccttca ttgtattcag 2160
tccttgcctt ccctttaaag tgcctttttc aactgaaaga ggagtaggta aatgccagta 2220
agctgcaggt tttctgtccc tgcatagaat gattagcaga gagtgtgatg gttccagaga 2280
aaacatacaa aagctaaatt aggaggtggg agacagtcac agaaatgttc cagatctcac 2340
tagctctcat ggtctgtttc cagtaatggt ccaatatttc ttgtcctttt tgtttcctca 2400
tggcagtgtg tcccaggcta tcagggtgtc aactgtgagt atgaagtgga tgagtgccag 2460
aatcagccct gccagaatgg aggcacctgt attgaccttg tgaaccattt caagtgctct 2520
tgcccaccag gcactcgggg tatgaaatca tccttatcca ttttccatcc agggcattgt 2580
cttaagttat aaatccattc ttagtgttca ggggatttta taaaattaaa gataggaaga 2640
ctagcttcat tccaagcatt tagttctaca tcctagtaat tcaagccatt ttattctccc 2700
atctcttgct agctctgatg ttgtggttta tgttgtcagt tttatctggt tgtttggcat 2760
cttgatattc catgaaacac agaatatgga agggatacaa cattagcata acattaaaaa 2820
attagcctgg tcagtaagat ttcttgttgc ttcacagaaa agcaactaat ggcctctaaa 2880
ataaacaatt tacatttacc tgcttttgta tttttttaat tagccaagtc ttacctctat 2940
aaggtggggg aatgaatgaa atgaaaatat ctgcctattt tcatagcttc aggggaggat 3000
actgatggtg taaggttcta ccttaggatg cagtaaggcc tgatgagaga tgcaaccctg 3060
gactgttgag tgatggggcc atcattcttc tgtgggcatt gccactgctt ttgaactgag 3120
tggtatggcc atcattctgc tgtggacatt gacactgctt ttgaaccatc ccttggaaag 3180
agctttgagt attgtttgtt agaagggatg tagttatgct ggggcctact ctgagaaggc 3240
tgtgaaaaaa atgtaaaagg ccatccacac tggtgagaaa ttcatccttg ttagtgtcaa 3300
agagaataga atgccagaga agtatttgta ttgccttaga gctttgtgtc agtaaagttt 3360
gataatttgc caccccaggt atcagagttt gactaaatta aaataggaga gtaccctagt 3420
agtaggctga ttattaagga atgaggttaa tgggaagaag aaaactaagg agaaacagtc 3480
tttaagtatt tgaagaattg tcctgtaaaa aggagaattg ttctttattc tagaaatcag 3540
tggttgagta gaaattaatt ccagttcatt gttagtttgt tcctttgaga aactgtactg 3600
gttaggacaa gctacgattc tggtgatagc tgttatcgta gttcatttat gttgctataa 3660
aagaatacct gagactaagt aatttatttt tcgaaagagg tatatttggc tcgcagttct 3720
ataggctgta caggaagcac ggtgccagca tctgcctctg gtgtgagcgt caggctgttt 3780

ccactcatgg tgaaaagtga agaagatcca gggacccaaa cacctcccat taggccccac 3840

ctccaacatt ggagatcaca ttttaacatg tggtttggtg cagtcaaact tcaagctata 3900

gcagctaatt aaacctcaaa tctcagagct taatataaca ctgattttgt cactcaaata 3960

aagtcgaata tgtgttgggc tacctttctc tgtgttttag atatgtggtt tgaaacacct 4020

ggcctccaag gcctccacag tacagggaag agggacagac ttgaagggtc atacagcagc 4080

ttccactagc tcagtctgga agcaacacat atcactatcc ctcgcagtat attgaccgga 4140

attattaaat ggtctctgtg acttcaaggg aggctgggaa atataggaga gtacatgagt 4200

attcatcaaa ctctaaaatc actactatac catgaacttt taattaataa gacatgtcca 4260

gtcctagaat ggatcatttc agaaggaaac aaaatttttg tctcttctaa ccatatggta 4320

aagatggctt agaatactag ttttgatatt atcattgaca gtctttttct ttcagcactt 4380

tgaatatatc atcccactgc tttctgatat ccatggtttt taatgagaaa tatgctgtta 4440

atcttattga ggttccctta tatgtgatac attgcttctt tcttgatgcc ttcaagattc 4500

tttatctttt gatagtttga ttatgatgta tgtaggtatg aatttctttg agtttatcct 4560

acttacagtt ccttggatta ttttactgtg tgtgcttgtt taggtatgtg gaacagtatt 4620

tttttatcct gacttaactt ctctggccat tggtaaacat tccaggaaac tctcaagtcc 4680

cataacccca ttgttagagt catcacaatg tgtgttttgg tcaccatatt tctaagcctt 4740

agatctttgc ctgcatgtcc caccagtttc ccaggtcaag ggctaaatgt aacagttctg 4800

cctcttccta ctactttgct tcaggctgag gttgagttct ctctcagttg atagatctgt 4860

ttatttctgg tggttccatt ctgatgggga aatatgcatt tatgctcatt ttactagcaa 4920

tccctaaata tcaaatattc tttgagacag ggagcatata ctagattttg tttgttttta 4980

cttttttgtt ttcaatttac tctatgtata aggtgatata tgcatatatg catatagacc 5040

aaaagtgtat tcacacatag aaaagtatac agtgaaaata cttctcattc ccatctctct 5100

tctggtgggc aaattttccc tctcctgcaa acatgtaatc gccacttttg gttgtttgtg 5160

tttctttcta gaatttttca aaaactgtat ccttcagaat ttctttatgt agagagaagc 5220

caaatgcaaa gatattttat ttcctttact ttttactctg aagatagaat attagacaca 5280

ttttttgcac ttgatttttt tttacttaat tggagatatt ttcatatcaa aatatagaac 5340

ttcctctctc tctctctgag atagatagat agatagatag atagatagat agatagatag 5400

atatttgttt gtttgtttgt tttgagatgg agttttgctc ttgttgccca ggctggagtg 5460

caatggtatg atctcagctc accgcaacct ccgcctcctg ggttcgagtg attttcctgc 5520

ctcagcgccc tgagtagctg ggattacagg catgtgccac catgcctggc tgattttgta 5580

tttttaatag agacagggtt tctccatgtt ggtcaggctg gtcttgaact cctggcctca 5640

agcaatccac ccgcctcagc ctcccaaagt gctgggatga caggcgtgag ccaccacacc 5700

cagcctgaac ttcctcaata ttttttatag ctacagaatt cccttttaca ggcataccat 5760

attttaacca gtgccatcat gatagatatt tcagttatct tgcaggcata taagtatatt 5820

ggtgaaataa attttcagaa atagaatcgc tggattggat aatatgtgca tttatcattt 5880

taatcagtga ttgcagtata tcagcatatt ggctttttct taaggtggag aatatatttt 5940

catttaagaa tcattttctt ttgatttttt ttgcagtttc tttgtttata tatatatata 6000

tgtatatata catatatata tatatacaca catatatctt ttttcccaca ttaagttgtt 6060

gatgtaatgt tttgtggaaa ttggctatga tataaaaaaa caaatatatt cccagtattt 6120

ttttatcttt tttctttata gtgattttta gtgcacactt ttacttttta atcaagttga 6180

atttatcact tttaaaatgg cttctggatt ttgagttgta gttagcaaca tcatatacac 6240

ttgaagatcg taaagaaatt ctcccatatt ttcttcaagt acttttgcca tatgactact 6300

catttgttaa tacacaattt attgaatatt ttgtttttta tatatataat gatttgagat 6360

ggcacctttg ccagattcta aattcccatg tgtatttttg gtgttcttct ggattttctt 6420

tattttctgt gttattctat taattcacca ataacgttct atttcaatta ttaaggatct 6480

tcttcttcat agttttcctg gttatttttt ttcacataat tttataatca gcttgtctgt 6540

taataaattc ctgttgataa tttttagtgg ggtctcatta aatttatgaa ttatagtata 6600

gagaaaactg ataactttat gatgaggagt cttgctaacc aaatcctggt atgtctttcc 6660

atttattcaa gtctcttttg tgtctgttag gacagtatta aagttgtctt catttaggtg 6720

taacacattt cttgtgaaga ttgttcctag gtattttatc tttggtaatt tggtaattgc 6780

ttttgaaaat tgggccttac tttacattta aaaaaaaaaa tcttgctttt gttagtgtat 6840

gctacaatgc aagtggcagg aatacataca gaactgtttt cttttgtacc tttttttttt 6900

ttttttttaa gacagagtct tgctctgttg cccaggctgg agtgcagtag cgcaatctcg 6960

gcttactgca agctccacct cccaggttca tgccattctc ctgcctcagc ctcccgagta 7020

gctgggacta caagcgcccg ccaccatgcc tggctaattt tttttttgta ttttttagtg 7080

gagacggggt ttcaccatgt tagccaggat ggtctcgatc tcctgacctc gtgatccacc 7140

tgccttggcc tcccagagtg ctgggattac aggcttgagc cactgcaccc agcctgtacc 7200

ttttttttta attatgatta ttattatttt ttgagacaga gtcttgctct gttgcccagg 7260

ctggagtgca gtggcataac ctcggctcac tgcaagcttc atctcccggt ttcaagtgat 7320

tctcctgcct cagcctccca agtagctgag attacagaca tgtaccacca cacgcagcta 7380

atttttgtat atttagtaga gatggggttt cactgtgttg ggcagattgg tcttgaactc 7440

ctgacctcaa gtgatccgcc tgccttagcc tcccaaagtg ctgggattac aggtgtgagc 7500

cactgtgccc agcctctttt gtgccttttt gaattaatca tagaataatt tctagaatag 7560

tcattgattt cttttgtttc tttgtaagac actatatgca gatattcata ctagatggct 7620

catttacatc tatagtacaa taaattagtc ctgttttaaa tggaataact tttgaactct 7680

cagcaggaaa tttatcctct atttttgtta aataaaaagc attggtgcac ttatacacta 7740

ttacagaaaa aagccagtga ttataaaact taaaaatgta tttcattcaa aattcagttc 7800

cagttgttac caatattttt acataaccta tgtatagtca tgggtcactt aacagtgggg 7860

atatgttctg agaaatatgc ccttaggcaa tttcattgtt gtacaatcat catggagtgt 7920

aattacacaa acagagagag tatggcttac tacacaccta ggctccaaac ctgcaccttg 7980

ttttactgaa ctgaatattg taggcaatta taacacaatg gtaagtattt atgtgtctga 8040

acatatttaa gaagaggtac agtagaagat ttaaaaggta cagtaaaaat acaatataaa 8100

agataaaaaa tagtacacct gtatagggca tttactatga aaggagcttg caagactgga 8160

acttgctggg tgagtcagtg aatggtaagt gaatgtgaag gcctaggaca ttactgtaca 8220

ctactgcaga ctttgtaaat actgtacacc taagctacac tgaatttatt taaaaagtaa 8280

ttgtgctatg acattacaag gctatgatgt tactaggcaa caggaatttg tcagcttcag 8340

cttcattatg ggaccactgt cgtatgtgct gcctgcattg actaaaatgt cattatgcgg 8400

ttgcataact gtatttattg ttatgttctt ttttggactg tttttatatt ttggattata 8460

ctttggaaaa attactgagc ctctcttgag ctccagtttc cttctctttg agatgggaca 8520

aataatagga cctacaccat agggtgctac ttcactggat tgtgtgggag agtacatgcg 8580

gagccctagt atgttctttc ctttacttgt acaactcacc aggatgacag ctgtcatgaa 8640

taaagagtgt gctttgccct gcccaactac aaggtcccac taccagttga ggagtatact 8700

ctgattcatt gtgaaaatgc caacaagggt ggttatccct tgctggaaag caggttaaaa 8760

ggattttcat aattctgtac ctcttctctt tcctccaggc ctactctgtg aagagaacat 8820

tgatgactgt gcccggggtc cccattgcct taatggtggt cagtgcatgg ataggattgg 8880

aggctacagt tgtcgctgct tgcctggctt tgctggggag cgttgtgagg gagacatcaa 8940

cgagtgcctc tccaacccct gcagctctga gggcagcctg gactgtatac agctcaccaa 9000

tgactacctg tgtgtttgcc gtagtgcctt tactggtgag aagctctcta gcctcctggc 9060

cactctgcct tggaggatgg ttttagtgaa caaattgggc cctgtgagtt gtcccagacc 9120

caagtgaagc ttttcttata taagaaaaac aacagcagct taatttttta aagccagttt 9180

tttcttagtc tgtactttat ggatgttatg atcagtgtct acctttaatt tttcctaaga 9240

gattcttttg ttgtgcattg attatatcat gctatggcag gaatattttt tacaatgtat 9300

attgaaagta gttgaacaaa ataacatttt ttaatagtgg caaaatatcc aaaacctaaa 9360

atttactatc tgaatcattt taaagtgtac agtgtacagt tcagtggcat tgtttagtac 9420

attgacattg ttgttctact atcaccccca ttcattcaca gaactctttt catctgaaca 9480

aaataacatt ttgttttaag gaaccttacg tctaactccg gtttcttcac cctgcctcct 9540

ttgacatgtg atggcttgaa ttactgggta gttcctgcca ccatgatctt tccaccccta 9600

ggactcagag aagcttagga ccctgcttag atacttaaag ggggcaggtc caaattcaag 9660

tgtgtttaac caaagatcag ttgatgtggg tatgaaatga aaagttgcag tggtcacaag 9720

taatgccttg cccagtaaag gcttggccat gttcctactc tgttcccaag cttctttctt 9780

tcatcacagg gcctgaagat gaccaagctc tgcctcagaa taggggtctt atcacaagaa 9840

cttactagtt acttctggag agggcagcat ggtgactttt gggaactaaa atagttgttt 9900

gtatccagat gtggcaggat actcagattt gaaatttatc atgcatttcc tggggtctgt 9960

agttgtattt cttatatgga gggtgtgatc agattcctta ggagtcagaa aataggcttt 10020

aatagtctgt ttacaaacaa ttcatcaagc tgaactctgc tacatcctga gcccatctcc 10080

agccatcttt ccttccaact ttaaagatat gctaccttac aatcttggaa ttttgtaaag 10140

aatgggaggg attctgcaat atggaagctc cctggataca ctaacatcaa aaaagaaata 10200

gtccattgtg aacataatag ctattgaggc aatgtggaac agaataagta tgaataatct 10260

gtctacagcc tcgtattggc caaactgcac tttgaaagcc aaagcatcaa aactggctcc 10320

tgtttctttc attgcccctg aggctttttt ctctggaaac acaagcattt gcaaagtaag 10380

ggggaagcag gagaaggaag gaatggagac cactttgaag tagtatccca cagccaggaa 10440

gtagtgtttg tgcattttcc acatttgtga atgaaaaaaa aaaaatccct tgaatacatt 10500

gagcacagtt attgtgggat gctgttgcag agataaacag caagggactg gtggtccacc 10560

aagctgtgtc catgtggtgg ctgctcttgg ttagcggctg ctgctgtgat ttgaagagac 10620

aaacatttat acctcttgct gcttgatctc gttggccaaa aacattacta tttttatttc 10680

atttttaaag ctacctcttc tttcccaagt gaccaccggc caaatggggc ctatctcctg 10740

ctgttggcca ggagtcccag ctgtgccctg cgtctcctct gtggttccag ttgcctcgtt 10800

ctttatcttg gagcagtggc ccccaggggc cttccgtttt agaactattc ctagaggaaa 10860

ctaactctag gaaaattaag ctgtgtttta aggaatacga gttaggttac agaaaggcca 10920

aaaagtgatt gaaatgaggt atcacagatg aaaattctct ataccgcaaa tcaccattta 10980

aatgtattgg cctggtgtag cctctcctat gaagtagata acattaacaa aatgcagtga 11040

atagtttttc aactgttttt ttgcaactta tgaaagctaa aatgacttca aaaagctgaa 11100

gtgtgatttc tctttttttg atgggattta agtcactttt ctcacccacc ctgttgtgtg 11160

agttggtact tactgtgtag gcaagacaga gtaagggact ggcctgagct ggtgacaggc 11220

caatcatgtt gagatgcagt gagtgttttc tttctatgct cagattacca caggcctaga 11280

actgaactct ctcacacttg cttcacctat ggctagttcc catcccagca aagtttgttg 11340

agtaacttgc agtgactact tctgcctttt aaaacctttt ttatttttta ttttctgagg 11400

ccggcactgt gaaaccttcg tcgatgtgtg tccccagatg ccctgcctga atggagggac 11460

ttgtgctgtg gccagtaaca tgcctgatgg tttcatttgc cgttgtcccc cggtaagtgc 11520

cctgttgctc atctaagaag gggcattgtc tcttccatac aatctgtgaa cagaactgag 11580

attaaacctg gttttaccaa tcttcgaaat gggaagattt ctcaatttta gctctttcag 11640

aggaaagtgc ttgggttttt ccatttaaag ctgtttacaa gtgacaaaac aaaacaaaaa 11700

caaataaaag ctgtttagac actcccagaa acagtgagct tcccaatcca tgactgtcag 11760

tcttaagaaa aactaaggga aaaccacaaa aagtggtgga gtgccccctt gaaaataggg 11820

ctgatgtgtg gggcttcttg gccccaggct ggagggtaat ctcactgccc agattgggcc 11880

agagttggtg gaaatgctgc tcttacattc tttagcagga accatttgga agcaagttgt 11940

ctcttaaagt agagggaagc tcaattttag ctatttgcta tacccattct tttatcactg 12000

ccagaggttg aaaccttccc aaaacaagag atccctatgc ctcgggagat accaaaagga 12060

aacctgtcta gtaaccttgg cttgggctga acgggaagta cagaagggag tgagatgtgg 12120

aggttttcta gaaacctcaa acttccaata tgactgtttt cctaccaatg ccatttttat 12180

ttcctcactg atttccacag ggattttccg gggcaaggtg ccagagcagc tgtggacaag 12240

tgaaatgtag gaagggggag cagtgtgtgc acaccgcctc tggaccccgc tgcttctgcc 12300

ccagtccccg ggactgcgag tcaggctgtg ccagtagccc ctgccagcac gggggcagct 12360

gccaccctca gcgccagcct ccttattact cctgccagtg tgccccacca ttctcgggta 12420

gccgctgtga actctacacg gcacccccca gcacccctcc tgccacctgt ctgagccagt 12480

attgtgccga caaagctcgg gatggcgtct gtgatgaggc ctgcaacagc catgcctgcc 12540

agtgggatgg gggtgactgt tctctcacca tggagaaccc ctgggccaac tgctcctccc 12600

cacttccctg ctgggattat atcaacaacc agtgtgatga gctgtgcaac acggtcgagt 12660

gcctgtttga caactttgaa tgccagggga acagcaagac atgcaagtaa gggcttcacg 12720

ttttccagaa ctgaagggga ctgtcactaa cacacttcag tttctcgttc ttgctcctaa 12780

ccactccagt gcttcagcct aagctgcctc tgcttttcag atggtgccag ccttgtccca 12840

tcgcacactg ccctgtgctc tcccttgctg acaatgggcc aggaatgctc attgcataat 12900

gaccctctct ctctgcctat ctttgccttc tcacctgctg ccttcgtgac ctcctggggg 12960

atataacttt tggaagtggt gtttcccact tcaagcagcc tctcacataa cctgcctgca 13020

ggatggatgg aaaggtctgt atctctctct tcaccccatt cctttcccta agactgtttt 13080

tccccatcta gatttatcag gaagcatgaa ttagattaag tctgtgaacc ctcccggtct 13140

taattgtata ggatgctcta tggtttactt aggaactttc ctattaacta gccattccat 13200

ataatttaat cccaattaat tctcagcaac tcacgaggca taggtattac taacattctt 13260

tcataggtga gagaacttag gcccaagggt taaagaactt gccaaagcaa gttgtttctt 13320

tgactcctaa tcttctgatt gagctagcat gaaaagttgt accaaatagt ataaatatca 13380

aggggtaggg gatgaggaga ggagagtata gatggaatag tttgtacatc atttaggttt 13440

gggggttcta attccttttc caaatattga aagagcctgt acaagtaata tctggtgtgc 13500

tataaggaca tacccatctg cctttacctt ctgtagttgg gattcactct tgaaagggaa 13560

agaaatgggt catgttaata ttgttctctg tatggcaact ctcaatggtt cagggccttg 13620

tatcaagttt ctagaggcat ctttcacttc ctaaagcccg ttgttacctt actgggggca 13680

atggatttct ctaaagtaga ggaagcatga accaggttga cttttgtttc agatatgcag 13740

ctttgtttga aaacctgact taacaggctt gtaaactgcc tgggagcccc taagctcctg 13800

cagcatagtg agtacaatgt tcttcattta ggaagtactc aacaaggatg agtggcatag 13860

gcatcaggga taggagatgc aaagaaggtt ttgtggggca tttgggtcct gggtgtgggc 13920

cacagaaaat gggtaggaaa atccacagta gttccaaatg aaaaagcaaa tagagctcca 13980

gtctaatctg agctcttttg cctgttctct gcttcccctt acctaggtat gacaaatact 14040

gtgcagacca cttcaaagac aaccactgtg accaggggtg caacagtgag gagtgtggtt 14100

gggatgggct ggactgtgct gctgaccaac ctgagaacct ggcagaaggt accctggtta 14160

ttgtggtatt gatgccacct gaacaactgc tccaggatgc tcgcagcttc ttgcgggcac 14220

tgggtaccct gctccacacc aacctgcgca ttaagcggga ctcccagggg gaactc 14276

<210> 8
<211> 20
<212> PRT
<213> Homo sapiens

<220>
<223> NOTCH2 fragment

<400> 8
Met Val Tyr Pro Tyr Tyr Gly Glu Lys Ser Ala Ala Met Lys Lys Gln
1               5                   10                  15
Arg Met Thr Arg
            20

<210> 9
<211> 5
<212> PRT
<213> Homo sapiens

<220>
<223> NOTCH2 fragment

<400> 9
Met Val Tyr Pro Tyr
1               5

<210> 10
<211> 6
<212> PRT
<213> Homo sapiens

<220>
<223> NOTCH2 fragment

<400> 10
Met Val Tyr Pro Tyr Tyr
1               5

<210> 11
<211> 7
<212> PRT
<213> Homo sapiens

<220>
<223> NOTCH2 fragment

<400> 11
Met Val Tyr Pro Tyr Tyr Gly
1               5

<210> 12
<211> 8
<212> PRT
<213> Homo sapiens

<220>
<223> NOTCH2 fragment

<400> 12
Met Val Tyr Pro Tyr Tyr Gly Glu
1               5

<210> 13
<211> 9
<212> PRT
<213> Homo sapiens

<220>
<223> NOTCH2 fragment

<400> 13
Met Val Tyr Pro Tyr Tyr Gly Glu Lys
1               5

<210> 14
<211> 10
<212> PRT
<213> Homo sapiens

<220>
<223> NOTCH2 fragment

<400> 14
Met Val Tyr Pro Tyr Tyr Gly Glu Lys Ser
1               5                   10

<210> 15
<211> 11
<212> PRT

<213> Homo sapiens

<220>
<223> NOTCH2 fragment

<400> 15
Met Val Tyr Pro Tyr Tyr Gly Glu Lys Ser Ala
1               5                   10

<210> 16
<211> 12
<212> PRT
<213> Homo sapiens

<220>
<223> NOTCH2 fragment

<400> 16
Met Val Tyr Pro Tyr Tyr Gly Glu Lys Ser Ala Ala
1               5                   10

<210> 17
<211> 13
<212> PRT
<213> Homo sapiens

<220>
<223> NOTCH2 fragment

<400> 17
Met Val Tyr Pro Tyr Tyr Gly Glu Lys Ser Ala Ala Met
1               5                   10

<210> 18
<211> 14
<212> PRT
<213> Homo sapiens

<220>
<223> NOTCH2 fragment

<400> 18
Met Val Tyr Pro Tyr Tyr Gly Glu Lys Ser Ala Ala Met Lys
1               5                   10

<210> 19
<211> 15
<212> PRT
<213> Homo sapiens

<220>
<223> NOTCH2 fragment

<400> 19
Met Val Tyr Pro Tyr Tyr Gly Glu Lys Ser Ala Ala Met Lys Lys
1               5                   10                  15

<210> 20
<211> 16
<212> PRT
<213> Homo sapiens

<220>

<223> NOTCH2 fragment

<400> 20
Met Val Tyr Pro Tyr Tyr Gly Glu Lys Ser Ala Ala Met Lys Lys Gln
1               5                   10                  15

<210> 21
<211> 17
<212> PRT
<213> Homo sapiens

<220>
<223> NOTCH2 fragment

<400> 21
Met Val Tyr Pro Tyr Tyr Gly Glu Lys Ser Ala Ala Met Lys Lys Gln
1               5                   10                  15
Arg

<210> 22
<211> 18
<212> PRT
<213> Homo sapiens

<220>
<223> NOTCH2 fragment

<400> 22
Met Val Tyr Pro Tyr Tyr Gly Glu Lys Ser Ala Ala Met Lys Lys Gln
1               5                   10                  15
Arg Met

<210> 23
<211> 19
<212> PRT
<213> Homo sapiens

<220>
<223> NOTCH2 fragment

<400> 23
Met Val Tyr Pro Tyr Tyr Gly Glu Lys Ser Ala Ala Met Lys Lys Gln
1               5                   10                  15
Arg Met Thr

<210> 24
<211> 20
<212> PRT
<213> Homo sapiens

<220>
<223> NOTCH2 fragment

<400> 24
Met Ala Lys Arg Lys Arg Lys His Gly Ser Leu Trp Leu Pro Glu Gly
1               5                   10                  15
Phe Thr Leu Arg
            20

<210> 25
<211> 5
<212> PRT
<213> Homo sapiens

<220>
<223> NOTCH2 fragment

<400> 25
Met Ala Lys Arg Lys
1               5

<210> 26
<211> 6
<212> PRT
<213> Homo sapiens

<220>
<223> NOTCH2 fragment

<400> 26
Met Ala Lys Arg Lys Arg
1               5

<210> 27
<211> 7
<212> PRT
<213> Homo sapiens

<220>
<223> NOTCH2 fragment

<400> 27
Met Ala Lys Arg Lys Arg Lys
1               5

<210> 28
<211> 8
<212> PRT
<213> Homo sapiens

<220>
<223> NOTCH2 fragment

<400> 28
Met Ala Lys Arg Lys Arg Lys His
1               5

<210> 29
<211> 9
<212> PRT
<213> Homo sapiens

<220>
<223> NOTCH2 fragment

<400> 29
Met Ala Lys Arg Lys Arg Lys His Gly
1               5

<210> 30
<211> 10

<212> PRT
<213> Homo sapiens

<220>
<223> NOTCH2 fragment

<400> 30
Met Ala Lys Arg Lys Arg Lys His Gly Ser
1               5                   10

<210> 31
<211> 11
<212> PRT
<213> Homo sapiens

<220>
<223> NOTCH2 fragment

<400> 31
Met Ala Lys Arg Lys Arg Lys His Gly Ser Leu
1               5                   10

<210> 32
<211> 12
<212> PRT
<213> Homo sapiens

<220>
<223> NOTCH2 fragment

<400> 32
Met Ala Lys Arg Lys Arg Lys His Gly Ser Leu Trp
1               5                   10

<210> 33
<211> 13
<212> PRT
<213> Homo sapiens

<220>
<223> NOTCH2 fragment

<400> 33
Met Ala Lys Arg Lys Arg Lys His Gly Ser Leu Trp Leu
1               5                   10

<210> 34
<211> 14
<212> PRT
<213> Homo sapiens

<220>
<223> NOTCH2 fragment

<400> 34
Met Ala Lys Arg Lys Arg Lys His Gly Ser Leu Trp Leu Pro
1               5                   10

<210> 35
<211> 15
<212> PRT
<213> Homo sapiens

<220>
<223> NOTCH2 fragment

<400> 35
Met Ala Lys Arg Lys Arg Lys His Gly Ser Leu Trp Leu Pro Glu
1               5                   10                  15

<210> 36
<211> 16
<212> PRT
<213> Homo sapiens

<220>
<223> NOTCH2 fragment

<400> 36
Met Ala Lys Arg Lys Arg Lys His Gly Ser Leu Trp Leu Pro Glu Gly
1               5                   10                  15

<210> 37
<211> 17
<212> PRT
<213> Homo sapiens

<220>
<223> NOTCH2 fragment

<400> 37
Met Ala Lys Arg Lys Arg Lys His Gly Ser Leu Trp Leu Pro Glu Gly
1               5                   10                  15
Phe

<210> 38
<211> 18
<212> PRT
<213> Homo sapiens

<220>
<223> NOTCH2 fragment

<400> 38
Met Ala Lys Arg Lys Arg Lys His Gly Ser Leu Trp Leu Pro Glu Gly
1               5                   10                  15
Phe Thr

<210> 39
<211> 19
<212> PRT
<213> Homo sapiens

<220>
<223> NOTCH2 fragment

<400> 39
Met Ala Lys Arg Lys Arg Lys His Gly Ser Leu Trp Leu Pro Glu Gly
1               5                   10                  15
Phe Thr Leu

<210> 40
<211> 21
<212> DNA
<213> Artificial Sequence

<220>
<223> EMSA probe

<400> 40
cagccctgtg ggaacttgct g                                                21


<210> 41
<211> 20
<212> DNA
<213> Homo sapiens

<220>
<223> Polynucleotide probe

<400> 41
   gcccccttga aaatagggct                                                 20


<210> 42
<211> 20
<212> DNA
<213> Homo sapiens

<220>
<223> Polynucleotide probe

<400> 42
agattgggcc agagttggtg                                                  20


<210> 43
<211> 20
<212> DNA
<213> Homo sapiens

<220>
<223> Polynucleotide probe

<400> 43
ttccacaggg attttccggg                                                  20


<210> 44
<211> 20
<212> DNA
<213> Homo sapiens

<220>
<223> Polynucleotide probe

<400> 44
attactcctg ccagtgtgcc                                                  20


<210> 45
<211> 20
<212> DNA

<213> Homo sapiens

<220>
<223> Polynucleotide probe

<400> 45
tgtgaactct acacggcacc                                                    20


<210> 46
<211> 20
<212> DNA
<213> Homo sapiens

<220>
<223> Polynucleotide probe

<400> 46
ccaggggaac agcaagacat                                                    20


<210> 47
<211> 20
<212> DNA
<213> Homo sapiens

<220>
<223> Polynucleotide probe

<400> 47
ccacttcaag cagcctctca                                                    20


<210> 48
<211> 20
<212> DNA
<213> Homo sapiens

<220>
<223> Polynucleotide probe

<400> 48
accttcgtcg atgtgtgtcc                                                    20


<210> 49
<211> 20
<212> DNA
<213> Homo sapiens

<220>
<223> Polynucleotide probe

<400> 49
agcttaggct gaagcactgg                                                    20


<210> 50
<211> 20
<212> DNA
<213> Homo sapiens

<220>

<223> Polynucleotide probe

<400> 50
atgtaggaag ggggagcagt                                                    20


<210> 51
<211> 20
<212> DNA
<213> Homo sapiens

<220>
<223> Polynucleotide probe

<400> 51
atgtcttgct gttcccctgg                                                    20


<210> 52
<211> 20
<212> DNA
<213> Homo sapiens

<220>
<223> Polynucleotide probe

<400> 52
gagtcaggct gtgccagtag                                                    20


<210> 53
<211> 20
<212> DNA
<213> Homo sapiens

<220>
<223> Polynucleotide probe

<400> 53
taataaggag gctggcgctg                                                    20


<210> 54
<211> 20
<212> DNA
<213> Homo sapiens

<220>
<223> Polynucleotide probe

<400> 54
gcactgtgaa accttcgtcg                                                    20


<210> 55
<211> 20
<212> DNA
<213> Homo sapiens

<220>
<223> Polynucleotide probe

<400> 55

cacagcacaa gtccctccat                                                    20

<210> 56
<211> 20
<212> DNA
<213> Homo sapiens

<220>
<223> Polynucleotide probe

<400> 56
tttcagatgg tgccagcctt                                                    20

<210> 57
<211> 20
<212> DNA
<213> Homo sapiens

<220>
<223> Polynucleotide probe

<400> 57
taagaccggg agggttcaca                                                    20

<210> 58
<211> 20
<212> DNA
<213> Homo sapiens

<220>
<223> Polynucleotide probe

<400> 58
tgtgaaccct cccggtctta                                                    20

<210> 59
<211> 20
<212> DNA
<213> Homo sapiens

<220>
<223> Polynucleotide probe

<400> 59
ctcctcatcc cctacccctt                                                    20

<210> 60
<211> 20
<212> DNA
<213> Homo sapiens

<220>
<223> Polynucleotide probe

<400> 60
gtgctctccc ttgctgacaa                                                    20

<210> 61
<211> 20
<212> DNA
<213> Homo sapiens

<220>
<223> Polynucleotide probe

<400> 61
ggctgcttga agtgggaaac                                                    20


<210> 62
<211> 20
<212> DNA
<213> Homo sapiens

<220>
<223> Polynucleotide probe

<400> 62
tgagcaacag ggcacttacc                                                    20


<210> 63
<211> 20
<212> DNA
<213> Homo sapiens

<220>
<223> Polynucleotide probe

<400> 63
tgcctgaatg gagggacttg                                                    20


<210> 64
<211> 20
<212> DNA
<213> Homo sapiens

<220>
<223> Polynucleotide probe

<400> 64
cagcccaagc caaggttact                                                    20


<210> 65
<211> 20
<212> DNA
<213> Homo sapiens

<220>
<223> Polynucleotide probe

<400> 65
accttttgcc agagttgcct                                                    20


<210> 66
<211> 20
<212> DNA

<213> Homo sapiens

<220>
<223> Polynucleotide probe

<400> 66
ccgccagtgt gttcaagact                                                    20


<210> 67
<211> 20
<212> DNA
<213> Homo sapiens

<220>
<223> Polynucleotide probe

<400> 67
gtctgtcgtc agtgagtggc                                                    20


<210> 68
<211> 20
<212> DNA
<213> Homo sapiens

<220>
<223> Polynucleotide probe

<400> 68
tcttgaacac actggcggtt                                                    20


<210> 69
<211> 20
<212> DNA
<213> Homo sapiens

<220>
<223> Polynucleotide probe

<400> 69
ggatgacacg cagatccctt                                                    20


<210> 70
<211> 20
<212> DNA
<213> Homo sapiens

<220>
<223> Polynucleotide probe

<400> 70
gtacccctgc ccacttttca                                                    20


<210> 71
<211> 20
<212> DNA
<213> Homo sapiens

<220>

<223> Polynucleotide probe

<400> 71
gaaaagtggg caggggtaca                                                    20


<210> 72
<211> 20
<212> DNA
<213> Homo sapiens

<220>
<223> Polynucleotide probe

<400> 72
ctccccttca gcatcaggtc                                                    20


<210> 73
<211> 20
<212> DNA
<213> Homo sapiens

<220>
<223> Polynucleotide probe

<400> 73
gtcttggctg aaaagtgggc                                                    20


<210> 74
<211> 20
<212> DNA
<213> Homo sapiens

<220>
<223> Polynucleotide probe

<400> 74
gtgctttcct cccgaaggtt                                                    20


<210> 75
<211> 20
<212> DNA
<213> Homo sapiens

<220>
<223> Polynucleotide probe

<400> 75
aatccctgac tccagaacgc                                                    20


<210> 76
<211> 20
<212> DNA
<213> Homo sapiens

<220>
<223> Polynucleotide probe

<400> 76

ttgccatgat tacccccagc                                                    20


<210> 77
<211> 20
<212> DNA
<213> Homo sapiens

<220>
<223> Polynucleotide probe

<400> 77
agaccactga aaccttcggg                                                    20


<210> 78
<211> 20
<212> DNA
<213> Homo sapiens

<220>
<223> Polynucleotide probe

<400> 78
cccaccttcc ttagtctggc                                                    20


<210> 79
<211> 20
<212> DNA
<213> Homo sapiens

<220>
<223> Polynucleotide probe

<400> 79
ctgaaaagtg ggcaggggta                                                    20


<210> 80
<211> 20
<212> DNA
<213> Homo sapiens

<220>
<223> Polynucleotide probe

<400> 80
cctcccgaag gtttcagtgg                                                    20


<210> 81
<211> 20
<212> DNA
<213> Homo sapiens

<220>
<223> Polynucleotide probe

<400> 81
gccagactaa ggaaggtggg                                                    20

<210> 82
<211> 20
<212> DNA
<213> Homo sapiens

<220>
<223> Polynucleotide probe

<400> 82
agtcttgaac acactggcgg                                                   20


<210> 83
<211> 20
<212> DNA
<213> Homo sapiens

<220>
<223> Polynucleotide probe

<400> 83
gtggggacct gatgctgaag                                                   20


<210> 84
<211> 20
<212> DNA
<213> Homo sapiens

<220>
<223> Polynucleotide probe

<400> 84
ccgtgttctt gaagcagtgg                                                   20


<210> 85
<211> 14122
<212> DNA
<213> Homo sapiens

<220>
<223> NOTCH2 starting downstream of exon 20 and ending upstream of
      START1

<400> 85
   gtaagctttg ctcccttggg ctcaggtcat tcactgcagt attgactgtc cagtggcatt      60
   tagagaaggt atcatttttt tgtggaataa gagggaaggg cagatagtag tgggtgggca     120
   gggctctggg gaaagggctc aagacagaag gtgtaagggt agaggaatag gttcagagat     180
   tcggagaaga atgtatttga aatgaaacta aggtatattc cactttcagg gtttattttt     240
   aatatgcatc tggtactgag ggttgtgaag aactggctaa gaataagatt ggatgtgtca     300
   gccagtttag atttacttgc tcattggttg atttgaaccc catgtgtcac atgttcttct     360
   atgacatttc tccctcactg ggtttcccag gtgtgcttgt tgaacacttg tgccagcact     420
   caggtgtctg catcaatgct ggcaacacgc attactgtca gtgccccctg ggctatactg     480
   ggagctactg tgaggagcaa ctcgatgagt gtgcgtccaa cccctgccag cacggggcaa     540
   catgcagtga cttcattggt ggatacagat gcgaggtgag gacactgagc aagcagaggt 600
   catggacctc ccaggagatt gtacaactgt ttgagcactg atatattgaa tttagctgct 660
   tgtatcttat attgatagtt tatagatttt taccaatccc cgtcataatg tttaccactg 720
   tttctttcag cctctctgtg caataagtta acaggtccct agtatgatat ttttataggc 780
   gtctggaacc atcccagctc cttaggcttg cttttaggat tcagagccaa gagtcactac 840
   cagagtgcat tttacaggcc cacccaacat agcagtccac acttccctga atagcagctg 900
   agataggaaa aactgctttg tggagtgtcc ctttggcccc tgtcaccttt gtatttagtc 960
   attaatcagt caactagcaa ttttctctca aaaaaaaaaa atgttgctca tcctgttccc 1020
   catagcatca tgctaaacgt tacatgtata tatttgagga agtatgtgtc ttcaaggtac 1080
   atgtcactat tttaatgtct gggctgataa tggttagagc aagcaagata ttagcaaaat 1140
   gcctgagaca ttaatttgat accaattcaa accatcaagc aacactgaga aaagctttgc 1200
   ttctctattg ttatataaat gagccaacga tagcttctgt attttggccc ctaggttgtt 1260
   tatttcttca cagcaggctg gggctgggtc tcattagctc aaaagcctac cagtgccaaa 1320
   cccaaatttt gacacatcta gttgttttaa acatagcccc caaataagca gatttttagc 1380
   tatttagagc ttgcctgctt tgcatagcct gtaaaactgc agccaaaatc tgctagccat 1440
   agtataagta ggacaagata taccctgggg ccatacagac cccaggctga tgctggcctt 1500
   tagagctctc tgacccagag attcctgcca ctggctgttg agtgacatca cctagactcc 1560
   ttctctgatc cttctttccc agagtttact tgctgtcttc cccttactca cctacccctc 1620
   tggaaggtct catgctattt gggacttctc ccaggtgcaa acctgtcaaa gtgtccaaat 1680
   aaagccacct cacagccata tcttttttct tggtcagccc tgaaatcctt ccaaccccta 1740
   cagccaccac agccagccag ccagccccac ctccctctac ccctaagcct atctcaacca 1800
   gtttctcaca catagatcca atttcctaaa cctggagatc aataattgca tgtctgtact 1860
   accatccaca tatgcctttt gaccgagtca tagggtcctc atcacatacg gaggaatgtg 1920
   tattttaaag atgaggaaac ctgtacccag gaggaacaaa aggtttgcac acaagtcaaa 1980
   tctgaaacat ccttcattgt attcagtcct tgccttccct ttaaagtgcc tttttcaact 2040
   gaaagaggag taggtaaatg ccagtaagct gcaggttttc tgtccctgca tagaatgatt 2100
   agcagagagt gtgatggttc cagagaaaac atacaaaagc taaattagga ggtgggagac 2160
   agtcacagaa atgttccaga tctcactagc tctcatggtc tgtttccagt aatggtccaa 2220
   tatttcttgt cctttttgtt tcctcatggc agtgtgtccc aggctatcag ggtgtcaact 2280
   gtgagtatga agtggatgag tgccagaatc agccctgcca gaatggaggc acctgtattg 2340
   accttgtgaa ccatttcaag tgctcttgcc caccaggcac tcggggtatg aaatcatcct 2400
   tatccatttt ccatccaggg cattgtctta agttataaat ccattcttag tgttcagggg 2460
   attttataaa attaaagata ggaagactag cttcattcca agcatttagt tctacatcct 2520
   agtaattcaa gccattttat tctcccatct cttgctagct ctgatgttgt ggtttatgtt 2580
   gtcagtttta tctggttgtt tggcatcttg atattccatg aaacacagaa tatggaaggg 2640
   atacaacatt agcataacat taaaaaatta gcctggtcag taagatttct tgttgcttca 2700
   cagaaaagca actaatggcc tctaaaataa acaatttaca tttacctgct tttgtatttt 2760
   tttaattagc caagtcttac ctctataagg tgggggaatg aatgaaatga aaatatctgc 2820
   ctattttcat agcttcaggg gaggatactg atggtgtaag gttctacctt aggatgcagt 2880
   aaggcctgat gagagatgca accctggact gttgagtgat ggggccatca ttcttctgtg 2940
   ggcattgcca ctgcttttga actgagtggt atggccatca ttctgctgtg gacattgaca 3000
   ctgcttttga accatccctt ggaaagagct ttgagtattg tttgttagaa gggatgtagt 3060
   tatgctgggg cctactctga gaaggctgtg aaaaaaatgt aaaaggccat ccacactggt 3120
   gagaaattca tccttgttag tgtcaaagag aatagaatgc cagagaagta tttgtattgc 3180
   cttagagctt tgtgtcagta aagtttgata atttgccacc ccaggtatca gagtttgact 3240
   aaattaaaat aggagagtac cctagtagta ggctgattat taaggaatga ggttaatggg 3300
   aagaagaaaa ctaaggagaa acagtcttta agtatttgaa gaattgtcct gtaaaaagga 3360
   gaattgttct ttattctaga aatcagtggt tgagtagaaa ttaattccag ttcattgtta 3420
   gtttgttcct ttgagaaact gtactggtta ggacaagcta cgattctggt gatagctgtt 3480
   atcgtagttc atttatgttg ctataaaaga atacctgaga ctaagtaatt tatttttcga 3540
   aagaggtata tttggctcgc agttctatag gctgtacagg aagcacggtg ccagcatctg 3600
   cctctggtgt gagcgtcagg ctgtttccac tcatggtgaa aagtgaagaa gatccaggga 3660
   cccaaacacc tcccattagg ccccacctcc aacattggag atcacatttt aacatgtggt 3720
   ttggtgcagt caaacttcaa gctatagcag ctaattaaac ctcaaatctc agagcttaat 3780
   ataacactga ttttgtcact caaataaagt cgaatatgtg ttgggctacc tttctctgtg 3840
   ttttagatat gtggtttgaa acacctggcc tccaaggcct ccacagtaca gggaagaggg 3900
   acagacttga agggtcatac agcagcttcc actagctcag tctggaagca acacatatca 3960
   ctatccctcg cagtatattg accggaatta ttaaatggtc tctgtgactt caagggaggc 4020
   tgggaaatat aggagagtac atgagtattc atcaaactct aaaatcacta ctataccatg 4080
   aacttttaat taataagaca tgtccagtcc tagaatggat catttcagaa ggaaacaaaa 4140
   tttttgtctc ttctaaccat atggtaaaga tggcttagaa tactagtttt gatattatca 4200
   ttgacagtct ttttctttca gcactttgaa tatatcatcc cactgctttc tgatatccat 4260
   ggtttttaat gagaaatatg ctgttaatct tattgaggtt cccttatatg tgatacattg 4320
   cttctttctt gatgccttca agattcttta tcttttgata gtttgattat gatgtatgta 4380
   ggtatgaatt tctttgagtt tatcctactt acagttcctt ggattatttt actgtgtgtg 4440
   cttgtttagg tatgtggaac agtatttttt tatcctgact taacttctct ggccattggt 4500
   aaacattcca ggaaactctc aagtcccata accccattgt tagagtcatc acaatgtgtg 4560
   ttttggtcac catatttcta agccttagat ctttgcctgc atgtcccacc agtttcccag 4620
   gtcaagggct aaatgtaaca gttctgcctc ttcctactac tttgcttcag gctgaggttg 4680
   agttctctct cagttgatag atctgtttat ttctggtggt tccattctga tggggaaata 4740
   tgcatttatg ctcattttac tagcaatccc taaatatcaa atattctttg agacagggag 4800
   catatactag attttgtttg tttttacttt tttgttttca atttactcta tgtataaggt 4860
   gatatatgca tatatgcata tagaccaaaa gtgtattcac acatagaaaa gtatacagtg 4920
   aaaatacttc tcattcccat ctctcttctg gtgggcaaat tttccctctc ctgcaaacat 4980
   gtaatcgcca cttttggttg tttgtgtttc tttctagaat ttttcaaaaa ctgtatcctt 5040
   cagaatttct ttatgtagag agaagccaaa tgcaaagata ttttatttcc tttacttttt 5100
   actctgaaga tagaatatta gacacatttt ttgcacttga ttttttttta cttaattgga 5160
   gatattttca tatcaaaata tagaacttcc tctctctctc tctgagatag atagatagat 5220
   agatagatag atagatagat agatagatat ttgtttgttt gtttgttttg agatggagtt 5280
   ttgctcttgt tgcccaggct ggagtgcaat ggtatgatct cagctcaccg caacctccgc 5340
   ctcctgggtt cgagtgattt tcctgcctca gcgccctgag tagctgggat tacaggcatg 5400
   tgccaccatg cctggctgat tttgtatttt taatagagac agggtttctc catgttggtc 5460
   aggctggtct tgaactcctg gcctcaagca atccacccgc ctcagcctcc caaagtgctg 5520
   ggatgacagg cgtgagccac cacacccagc ctgaacttcc tcaatatttt ttatagctac 5580
   agaattccct tttacaggca taccatattt taaccagtgc catcatgata gatatttcag 5640
   ttatcttgca ggcatataag tatattggtg aaataaattt tcagaaatag aatcgctgga 5700
   ttggataata tgtgcattta tcattttaat cagtgattgc agtatatcag catattggct 5760
   ttttcttaag gtggagaata tattttcatt taagaatcat tttcttttga ttttttttgc 5820
   agtttctttg tttatatata tatatatgta tatatacata tatatatata tacacacata 5880
   tatctttttt cccacattaa gttgttgatg taatgttttg tggaaattgg ctatgatata 5940
   aaaaaacaaa tatattccca gtattttttt atcttttttc tttatagtga tttttagtgc 6000
   acacttttac tttttaatca agttgaattt atcactttta aaatggcttc tggattttga 6060
   gttgtagtta gcaacatcat atacacttga agatcgtaaa gaaattctcc catattttct 6120
   tcaagtactt ttgccatatg actactcatt tgttaataca caatttattg aatattttgt 6180
   tttttatata tataatgatt tgagatggca cctttgccag attctaaatt cccatgtgta 6240
   tttttggtgt tcttctggat tttctttatt ttctgtgtta ttctattaat tcaccaataa 6300
   cgttctattt caattattaa ggatcttctt cttcatagtt ttcctggtta ttttttttca 6360
   cataatttta taatcagctt gtctgttaat aaattcctgt tgataatttt tagtggggtc 6420
   tcattaaatt tatgaattat agtatagaga aaactgataa ctttatgatg aggagtcttg 6480
   ctaaccaaat cctggtatgt ctttccattt attcaagtct cttttgtgtc tgttaggaca 6540
   gtattaaagt tgtcttcatt taggtgtaac acatttcttg tgaagattgt tcctaggtat 6600
   tttatctttg gtaatttggt aattgctttt gaaaattggg ccttacttta catttaaaaa 6660
   aaaaaatctt gcttttgtta gtgtatgcta caatgcaagt ggcaggaata catacagaac 6720
   tgttttcttt tgtacctttt tttttttttt ttttaagaca gagtcttgct ctgttgccca 6780
   ggctggagtg cagtagcgca atctcggctt actgcaagct ccacctccca ggttcatgcc 6840
   attctcctgc ctcagcctcc cgagtagctg ggactacaag cgcccgccac catgcctggc 6900
   taattttttt tttgtatttt ttagtggaga cggggtttca ccatgttagc caggatggtc 6960
   tcgatctcct gacctcgtga tccacctgcc ttggcctccc agagtgctgg gattacaggc 7020
   ttgagccact gcacccagcc tgtacctttt tttttaatta tgattattat tattttttga 7080
   gacagagtct tgctctgttg cccaggctgg agtgcagtgg cataacctcg gctcactgca 7140
   agcttcatct cccggtttca agtgattctc ctgcctcagc ctcccaagta gctgagatta 7200
   cagacatgta ccaccacacg cagctaattt ttgtatattt agtagagatg gggtttcact 7260
   gtgttgggca gattggtctt gaactcctga cctcaagtga tccgcctgcc ttagcctccc 7320
   aaagtgctgg gattacaggt gtgagccact gtgcccagcc tcttttgtgc ctttttgaat 7380
   taatcataga ataatttcta gaatagtcat tgatttcttt tgtttctttg taagacacta 7440
   tatgcagata ttcatactag atggctcatt tacatctata gtacaataaa ttagtcctgt 7500
   tttaaatgga ataacttttg aactctcagc aggaaattta tcctctattt ttgttaaata 7560
   aaaagcattg gtgcacttat acactattac agaaaaaagc cagtgattat aaaacttaaa 7620
   aatgtatttc attcaaaatt cagttccagt tgttaccaat atttttacat aacctatgta 7680
   tagtcatggg tcacttaaca gtggggatat gttctgagaa atatgccctt aggcaatttc 7740
   attgttgtac aatcatcatg gagtgtaatt acacaaacag agagagtatg gcttactaca 7800
   cacctaggct ccaaacctgc accttgtttt actgaactga atattgtagg caattataac 7860
   acaatggtaa gtatttatgt gtctgaacat atttaagaag aggtacagta gaagatttaa 7920
   aaggtacagt aaaaatacaa tataaaagat aaaaaatagt acacctgtat agggcattta 7980
   ctatgaaagg agcttgcaag actggaactt gctgggtgag tcagtgaatg gtaagtgaat 8040
   gtgaaggcct aggacattac tgtacactac tgcagacttt gtaaatactg tacacctaag 8100
   ctacactgaa tttatttaaa aagtaattgt gctatgacat tacaaggcta tgatgttact 8160
   aggcaacagg aatttgtcag cttcagcttc attatgggac cactgtcgta tgtgctgcct 8220
   gcattgacta aaatgtcatt atgcggttgc ataactgtat ttattgttat gttctttttt 8280
   ggactgtttt tatattttgg attatacttt ggaaaaatta ctgagcctct cttgagctcc 8340
   agtttccttc tctttgagat gggacaaata ataggaccta caccataggg tgctacttca 8400
   ctggattgtg tgggagagta catgcggagc cctagtatgt tctttccttt acttgtacaa 8460
   ctcaccagga tgacagctgt catgaataaa gagtgtgctt tgccctgccc aactacaagg 8520
   tcccactacc agttgaggag tatactctga ttcattgtga aaatgccaac aagggtggtt 8580
   atcccttgct ggaaagcagg ttaaaaggat tttcataatt ctgtacctct tctctttcct 8640
   ccaggcctac tctgtgaaga gaacattgat gactgtgccc ggggtcccca ttgccttaat 8700
   ggtggtcagt gcatggatag gattggaggc tacagttgtc gctgcttgcc tggctttgct 8760
   ggggagcgtt gtgagggaga catcaacgag tgcctctcca acccctgcag ctctgagggc 8820
   agcctggact gtatacagct caccaatgac tacctgtgtg tttgccgtag tgcctttact 8880
   ggtgagaagc tctctagcct cctggccact ctgccttgga ggatggtttt agtgaacaaa 8940
   ttgggccctg tgagttgtcc cagacccaag tgaagctttt cttatataag aaaaacaaca 9000
   gcagcttaat tttttaaagc cagttttttc ttagtctgta ctttatggat gttatgatca 9060
   gtgtctacct ttaatttttc ctaagagatt cttttgttgt gcattgatta tatcatgcta 9120
   tggcaggaat attttttaca atgtatattg aaagtagttg aacaaaataa cattttttaa 9180
   tagtggcaaa atatccaaaa cctaaaattt actatctgaa tcattttaaa gtgtacagtg 9240
   tacagttcag tggcattgtt tagtacattg acattgttgt tctactatca cccccattca 9300
   ttcacagaac tcttttcatc tgaacaaaat aacattttgt tttaaggaac cttacgtcta 9360
   actccggttt cttcaccctg cctcctttga catgtgatgg cttgaattac tgggtagttc 9420
   ctgccaccat gatctttcca cccctaggac tcagagaagc ttaggaccct gcttagatac 9480
   ttaaaggggg caggtccaaa ttcaagtgtg tttaaccaaa gatcagttga tgtgggtatg 9540
   aaatgaaaag ttgcagtggt cacaagtaat gccttgccca gtaaaggctt ggccatgttc 9600
   ctactctgtt cccaagcttc tttctttcat cacagggcct gaagatgacc aagctctgcc 9660
   tcagaatagg ggtcttatca caagaactta ctagttactt ctggagaggg cagcatggtg 9720
   acttttggga actaaaatag ttgtttgtat ccagatgtgg caggatactc agatttgaaa 9780
   tttatcatgc atttcctggg gtctgtagtt gtatttctta tatggagggt gtgatcagat 9840
   tccttaggag tcagaaaata ggctttaata gtctgtttac aaacaattca tcaagctgaa 9900
   ctctgctaca tcctgagccc atctccagcc atctttcctt ccaactttaa agatatgcta 9960
   ccttacaatc ttggaatttt gtaaagaatg ggagggattc tgcaatatgg aagctccctg 10020
   gatacactaa catcaaaaaa gaaatagtcc attgtgaaca taatagctat tgaggcaatg 10080
   tggaacagaa taagtatgaa taatctgtct acagcctcgt attggccaaa ctgcactttg 10140
   aaagccaaag catcaaaact ggctcctgtt tctttcattg cccctgaggc ttttttctct 10200
   ggaaacacaa gcatttgcaa agtaaggggg aagcaggaga aggaaggaat ggagaccact 10260
   ttgaagtagt atcccacagc caggaagtag tgtttgtgca ttttccacat ttgtgaatga 10320
   aaaaaaaaaa atcccttgaa tacattgagc acagttattg tgggatgctg ttgcagagat 10380
   aaacagcaag ggactggtgg tccaccaagc tgtgtccatg tggtggctgc tcttggttag 10440
   cggctgctgc tgtgatttga agagacaaac atttatacct cttgctgctt gatctcgttg 10500
   gccaaaaaca ttactatttt tatttcattt ttaaagctac ctcttctttc ccaagtgacc 10560
   accggccaaa tggggcctat ctcctgctgt tggccaggag tcccagctgt gccctgcgtc 10620
   tcctctgtgg ttccagttgc ctcgttcttt atcttggagc agtggccccc aggggccttc 10680
   cgttttagaa ctattcctag aggaaactaa ctctaggaaa attaagctgt gttttaagga 10740
   atacgagtta ggttacagaa aggccaaaaa gtgattgaaa tgaggtatca cagatgaaaa 10800
   ttctctatac cgcaaatcac catttaaatg tattggcctg gtgtagcctc tcctatgaag 10860
   tagataacat taacaaaatg cagtgaatag tttttcaact gtttttttgc aacttatgaa 10920
   agctaaaatg acttcaaaaa gctgaagtgt gatttctctt tttttgatgg gatttaagtc 10980
   acttttctca cccaccctgt tgtgtgagtt ggtacttact gtgtaggcaa gacagagtaa 11040
   gggactggcc tgagctggtg acaggccaat catgttgaga tgcagtgagt gttttctttc 11100
   tatgctcaga ttaccacagg cctagaactg aactctctca cacttgcttc acctatggct 11160
   agttcccatc ccagcaaagt ttgttgagta acttgcagtg actacttctg ccttttaaaa 11220
   ccttttttat tttttatttt ctgaggccgg cactgtgaaa ccttcgtcga tgtgtgtccc 11280
   cagatgccct gcctgaatgg agggacttgt gctgtggcca gtaacatgcc tgatggtttc 11340
   atttgccgtt gtcccccggt aagtgccctg ttgctcatct aagaaggggc attgtctctt 11400
   ccatacaatc tgtgaacaga actgagatta aacctggttt taccaatctt cgaaatggga 11460
   agatttctca attttagctc tttcagagga aagtgcttgg gtttttccat ttaaagctgt 11520
   ttacaagtga caaaacaaaa caaaaacaaa taaaagctgt ttagacactc ccagaaacag 11580
   tgagcttccc aatccatgac tgtcagtctt aagaaaaact aagggaaaac cacaaaaagt 11640
   ggtggagtgc ccccttgaaa atagggctga tgtgtggggc ttcttggccc caggctggag 11700
   ggtaatctca ctgcccagat tgggccagag ttggtggaaa tgctgctctt acattcttta 11760
   gcaggaacca tttggaagca agttgtctct taaagtagag ggaagctcaa ttttagctat 11820
   ttgctatacc cattctttta tcactgccag aggttgaaac cttcccaaaa caagagatcc 11880
   ctatgcctcg ggagatacca aaaggaaacc tgtctagtaa ccttggcttg ggctgaacgg 11940
   gaagtacaga agggagtgag atgtggaggt tttctagaaa cctcaaactt ccaatatgac 12000
   tgttttccta ccaatgccat ttttatttcc tcactgattt ccacagggat tttccggggc 12060
   aaggtgccag agcagctgtg gacaagtgaa atgtaggaag ggggagcagt gtgtgcacac 12120
   cgcctctgga ccccgctgct tctgccccag tccccgggac tgcgagtcag gctgtgccag 12180
   tagcccctgc cagcacgggg gcagctgcca ccctcagcgc cagcctcctt attactcctg 12240
   ccagtgtgcc ccaccattct cgggtagccg ctgtgaactc tacacggcac cccccagcac 12300
   ccctcctgcc acctgtctga gccagtattg tgccgacaaa gctcgggatg gcgtctgtga 12360
   tgaggcctgc aacagccatg cctgccagtg ggatgggggt gactgttctc tcaccatgga 12420
   gaacccctgg gccaactgct cctccccact tccctgctgg gattatatca acaaccagtg 12480
   tgatgagctg tgcaacacgg tcgagtgcct gtttgacaac tttgaatgcc aggggaacag 12540
   caagacatgc aagtaagggc ttcacgtttt ccagaactga aggggactgt cactaacaca 12600
   cttcagtttc tcgttcttgc tcctaaccac tccagtgctt cagcctaagc tgcctctgct 12660
   tttcagatgg tgccagcctt gtcccatcgc acactgccct gtgctctccc ttgctgacaa 12720
   tgggccagga atgctcattg cataatgacc ctctctctct gcctatcttt gccttctcac 12780
   ctgctgcctt cgtgacctcc tgggggatat aacttttgga agtggtgttt cccacttcaa 12840
   gcagcctctc acataacctg cctgcaggat ggatggaaag gtctgtatct ctctcttcac 12900
   cccattcctt tccctaagac tgtttttccc catctagatt tatcaggaag catgaattag 12960
   attaagtctg tgaaccctcc cggtcttaat tgtataggat gctctatggt ttacttagga 13020
   actttcctat taactagcca ttccatataa tttaatccca attaattctc agcaactcac 13080
   gaggcatagg tattactaac attctttcat aggtgagaga acttaggccc aagggttaaa 13140
   gaacttgcca aagcaagttg tttctttgac tcctaatctt ctgattgagc tagcatgaaa 13200
   agttgtacca aatagtataa atatcaaggg gtaggggatg aggagaggag agtatagatg 13260
   gaatagtttg tacatcattt aggtttgggg gttctaattc cttttccaaa tattgaaaga 13320
   gcctgtacaa gtaatatctg gtgtgctata aggacatacc catctgcctt taccttctgt 13380
   agttgggatt cactcttgaa agggaaagaa atgggtcatg ttaatattgt tctctgtatg 13440
   gcaactctca atggttcagg gccttgtatc aagtttctag aggcatcttt cacttcctaa 13500
   agcccgttgt taccttactg ggggcaatgg atttctctaa agtagaggaa gcatgaacca 13560
   ggttgacttt tgtttcagat atgcagcttt gtttgaaaac ctgacttaac aggcttgtaa 13620
   actgcctggg agcccctaag ctcctgcagc atagtgagta caatgttctt catttaggaa 13680
   gtactcaaca aggatgagtg gcataggcat cagggatagg agatgcaaag aaggttttgt 13740
   ggggcatttg ggtcctgggt gtgggccaca gaaaatgggt aggaaaatcc acagtagttc 13800
   caaatgaaaa agcaaataga gctccagtct aatctgagct cttttgcctg ttctctgctt 13860
   ccccttacct aggtatgaca aatactgtgc agaccacttc aaagacaacc actgtgacca 13920
   ggggtgcaac agtgaggagt gtggttggga tgggctggac tgtgctgctg accaacctga 13980
   gaacctggca gaaggtaccc tggttattgt ggtattgatg ccacctgaac aactgctcca 14040
   ggatgctcgc agcttcttgc gggcactggg taccctgctc cacaccaacc tgcgcattaa 14100
   gcgggactcc cagggggaac tc 14122

<210> 86
<211> 2471
<212> PRT
<213> Homo sapiens

<220>
<223> Wild-type NOTCH2 (with signal peptide)

<400> 86
Met Pro Ala Leu Arg Pro Ala Leu Leu Trp Ala Leu Leu Ala Leu Trp
1               5                   10                  15
Leu Cys Cys Ala Ala Pro Ala His Ala Leu Gln Cys Arg Asp Gly Tyr
            20                  25                  30
Glu Pro Cys Val Asn Glu Gly Met Cys Val Thr Tyr His Asn Gly Thr
        35                  40                  45
Gly Tyr Cys Lys Cys Pro Glu Gly Phe Leu Gly Glu Tyr Cys Gln His
    50                  55                  60
Arg Asp Pro Cys Glu Lys Asn Arg Cys Gln Asn Gly Gly Thr Cys Val
65                  70                  75                  80
Ala Gln Ala Met Leu Gly Lys Ala Thr Cys Arg Cys Ala Ser Gly Phe
                85                  90                  95
Thr Gly Glu Asp Cys Gln Tyr Ser Thr Ser His Pro Cys Phe Val Ser
            100                 105                 110
Arg Pro Cys Leu Asn Gly Gly Thr Cys His Met Leu Ser Arg Asp Thr
        115                 120                 125
Tyr Glu Cys Thr Cys Gln Val Gly Phe Thr Gly Lys Glu Cys Gln Trp
    130                 135                 140
Thr Asp Ala Cys Leu Ser His Pro Cys Ala Asn Gly Ser Thr Cys Thr
145                 150                 155                 160
Thr Val Ala Asn Gln Phe Ser Cys Lys Cys Leu Thr Gly Phe Thr Gly
                165                 170                 175
Gln Lys Cys Glu Thr Asp Val Asn Glu Cys Asp Ile Pro Gly His Cys
            180                 185                 190
Gln His Gly Gly Thr Cys Leu Asn Leu Pro Gly Ser Tyr Gln Cys Gln
        195                 200                 205
Cys Pro Gln Gly Phe Thr Gly Gln Tyr Cys Asp Ser Leu Tyr Val Pro
    210                 215                 220
Cys Ala Pro Ser Pro Cys Val Asn Gly Gly Thr Cys Arg Gln Thr Gly
225                 230                 235                 240
Asp Phe Thr Phe Glu Cys Asn Cys Leu Pro Gly Phe Glu Gly Ser Thr
                245                 250                 255
Cys Glu Arg Asn Ile Asp Asp Cys Pro Asn His Arg Cys Gln Asn Gly
            260                 265                 270
Gly Val Cys Val Asp Gly Val Asn Thr Tyr Asn Cys Arg Cys Pro Pro
        275                 280                 285
Gln Trp Thr Gly Gln Phe Cys Thr Glu Asp Val Asp Glu Cys Leu Leu
    290                 295                 300
Gln Pro Asn Ala Cys Gln Asn Gly Gly Thr Cys Ala Asn Arg Asn Gly

305                 310                 315                 320
Gly Tyr Gly Cys Val Cys Val Asn Gly Trp Ser Gly Asp Asp Cys Ser
                325                 330                 335
Glu Asn Ile Asp Asp Cys Ala Phe Ala Ser Cys Thr Pro Gly Ser Thr
            340                 345                 350
Cys Ile Asp Arg Val Ala Ser Phe Ser Cys Met Cys Pro Glu Gly Lys
        355                 360                 365
Ala Gly Leu Leu Cys His Leu Asp Asp Ala Cys Ile Ser Asn Pro Cys
    370                 375                 380
His Lys Gly Ala Leu Cys Asp Thr Asn Pro Leu Asn Gly Gln Tyr Ile
385                 390                 395                 400
Cys Thr Cys Pro Gln Gly Tyr Lys Gly Ala Asp Cys Thr Glu Asp Val
                405                 410                 415
Asp Glu Cys Ala Met Ala Asn Ser Asn Pro Cys Glu His Ala Gly Lys
            420                 425                 430
Cys Val Asn Thr Asp Gly Ala Phe His Cys Glu Cys Leu Lys Gly Tyr
        435                 440                 445
Ala Gly Pro Arg Cys Glu Met Asp Ile Asn Glu Cys His Ser Asp Pro
    450                 455                 460
Cys Gln Asn Asp Ala Thr Cys Leu Asp Lys Ile Gly Gly Phe Thr Cys
465                 470                 475                 480
Leu Cys Met Pro Gly Phe Lys Gly Val His Cys Glu Leu Glu Ile Asn
                485                 490                 495
Glu Cys Gln Ser Asn Pro Cys Val Asn Asn Gly Gln Cys Val Asp Lys
            500                 505                 510
Val Asn Arg Phe Gln Cys Leu Cys Pro Pro Gly Phe Thr Gly Pro Val
        515                 520                 525
Cys Gln Ile Asp Ile Asp Asp Cys Ser Ser Thr Pro Cys Leu Asn Gly
    530                 535                 540
Ala Lys Cys Ile Asp His Pro Asn Gly Tyr Glu Cys Gln Cys Ala Thr
545                 550                 555                 560
Gly Phe Thr Gly Val Leu Cys Glu Glu Asn Ile Asp Asn Cys Asp Pro
                565                 570                 575
Asp Pro Cys His His Gly Gln Cys Gln Asp Gly Ile Asp Ser Tyr Thr
            580                 585                 590
Cys Ile Cys Asn Pro Gly Tyr Met Gly Ala Ile Cys Ser Asp Gln Ile
        595                 600                 605
Asp Glu Cys Tyr Ser Ser Pro Cys Leu Asn Asp Gly Arg Cys Ile Asp
    610                 615                 620
Leu Val Asn Gly Tyr Gln Cys Asn Cys Gln Pro Gly Thr Ser Gly Val
625                 630                 635                 640
Asn Cys Glu Ile Asn Phe Asp Asp Cys Ala Ser Asn Pro Cys Ile His
                645                 650                 655
Gly Ile Cys Met Asp Gly Ile Asn Arg Tyr Ser Cys Val Cys Ser Pro
            660                 665                 670
Gly Phe Thr Gly Gln Arg Cys Asn Ile Asp Ile Asp Glu Cys Ala Ser
        675                 680                 685
Asn Pro Cys Arg Lys Gly Ala Thr Cys Ile Asn Gly Val Asn Gly Phe
    690                 695                 700
Arg Cys Ile Cys Pro Glu Gly Pro His His Pro Ser Cys Tyr Ser Gln
705                 710                 715                 720
Val Asn Glu Cys Leu Ser Asn Pro Cys Ile His Gly Asn Cys Thr Gly
                725                 730                 735
Gly Leu Ser Gly Tyr Lys Cys Leu Cys Asp Ala Gly Trp Val Gly Ile
            740                 745                 750
Asn Cys Glu Val Asp Lys Asn Glu Cys Leu Ser Asn Pro Cys Gln Asn
        755                 760                 765
Gly Gly Thr Cys Asp Asn Leu Val Asn Gly Tyr Arg Cys Thr Cys Lys
    770                 775                 780
Lys Gly Phe Lys Gly Tyr Asn Cys Gln Val Asn Ile Asp Glu Cys Ala
785                 790                 795                 800
Ser Asn Pro Cys Leu Asn Gln Gly Thr Cys Phe Asp Asp Ile Ser Gly
                805                 810                 815

Tyr Thr Cys His Cys Val Leu Pro Tyr Thr Gly Lys Asn Cys Gln Thr
            820                 825                 830
Val Leu Ala Pro Cys Ser Pro Asn Pro Cys Glu Asn Ala Ala Val Cys
        835                 840                 845
Lys Glu Ser Pro Asn Phe Glu Ser Tyr Thr Cys Leu Cys Ala Pro Gly
    850                 855                 860
Trp Gln Gly Gln Arg Cys Thr Ile Asp Ile Asp Glu Cys Ile Ser Lys
865                 870                 875                 880
Pro Cys Met Asn His Gly Leu Cys His Asn Thr Gln Gly Ser Tyr Met
                885                 890                 895
Cys Glu Cys Pro Pro Gly Phe Ser Gly Met Asp Cys Glu Glu Asp Ile
            900                 905                 910
Asp Asp Cys Leu Ala Asn Pro Cys Gln Asn Gly Gly Ser Cys Met Asp
        915                 920                 925
Gly Val Asn Thr Phe Ser Cys Leu Cys Leu Pro Gly Phe Thr Gly Asp
    930                 935                 940
Lys Cys Gln Thr Asp Met Asn Glu Cys Leu Ser Glu Pro Cys Lys Asn
945                 950                 955                 960
Gly Gly Thr Cys Ser Asp Tyr Val Asn Ser Tyr Thr Cys Lys Cys Gln
                965                 970                 975
Ala Gly Phe Asp Gly Val His Cys Glu Asn Asn Ile Asn Glu Cys Thr
            980                 985                 990
Glu Ser Ser Cys Phe Asn Gly Gly Thr Cys Val Asp Gly Ile Asn Ser
        995                 1000                1005
Phe Ser Cys Leu Cys Pro Val Gly Phe Thr Gly Ser Phe Cys Leu His
    1010                1015                1020
Glu Ile Asn Glu Cys Ser Ser His Pro Cys Leu Asn Glu Gly Thr Cys
1025                1030                1035                1040
Val Asp Gly Leu Gly Thr Tyr Arg Cys Ser Cys Pro Leu Gly Tyr Thr
                1045                1050                1055
Gly Lys Asn Cys Gln Thr Leu Val Asn Leu Cys Ser Arg Ser Pro Cys
            1060                1065                1070
Lys Asn Lys Gly Thr Cys Val Gln Lys Lys Ala Glu Ser Gln Cys Leu
        1075                1080                1085
Cys Pro Ser Gly Trp Ala Gly Ala Tyr Cys Asp Val Pro Asn Val Ser
    1090                1095                1100
Cys Asp Ile Ala Ala Ser Arg Arg Gly Val Leu Val Glu His Leu Cys
1105                1110                1115                1120
Gln His Ser Gly Val Cys Ile Asn Ala Gly Asn Thr His Tyr Cys Gln
                1125                1130                1135
Cys Pro Leu Gly Tyr Thr Gly Ser Tyr Cys Glu Glu Gln Leu Asp Glu
            1140                1145                1150
Cys Ala Ser Asn Pro Cys Gln His Gly Ala Thr Cys Ser Asp Phe Ile
        1155                1160                1165
Gly Gly Tyr Arg Cys Glu Cys Val Pro Gly Tyr Gln Gly Val Asn Cys
    1170                1175                1180
Glu Tyr Glu Val Asp Glu Cys Gln Asn Gln Pro Cys Gln Asn Gly Gly
1185                1190                1195                1200
Thr Cys Ile Asp Leu Val Asn His Phe Lys Cys Ser Cys Pro Pro Gly
                1205                1210                1215
Thr Arg Gly Leu Leu Cys Glu Glu Asn Ile Asp Asp Cys Ala Arg Gly
            1220                1225                1230
Pro His Cys Leu Asn Gly Gly Gln Cys Met Asp Arg Ile Gly Gly Tyr
        1235                1240                1245
Ser Cys Arg Cys Leu Pro Gly Phe Ala Gly Glu Arg Cys Glu Gly Asp
    1250                1255                1260
Ile Asn Glu Cys Leu Ser Asn Pro Cys Ser Ser Glu Gly Ser Leu Asp
1265                1270                1275                1280
Cys Ile Gln Leu Thr Asn Asp Tyr Leu Cys Val Cys Arg Ser Ala Phe
                1285                1290                1295
Thr Gly Arg His Cys Glu Thr Phe Val Asp Val Cys Pro Gln Met Pro
            1300                1305                1310
Cys Leu Asn Gly Gly Thr Cys Ala Val Ala Ser Asn Met Pro Asp Gly

1315                1320                1325
Phe Ile Cys Arg Cys Pro Pro Gly Phe Ser Gly Ala Arg Cys Gln Ser
    1330                1335                1340
Ser Cys Gly Gln Val Lys Cys Arg Lys Gly Glu Gln Cys Val His Thr
1345                1350                1355                1360
Ala Ser Gly Pro Arg Cys Phe Cys Pro Ser Pro Arg Asp Cys Glu Ser
                1365                1370                1375
Gly Cys Ala Ser Ser Pro Cys Gln His Gly Gly Ser Cys His Pro Gln
            1380                1385                1390
Arg Gln Pro Pro Tyr Tyr Ser Cys Gln Cys Ala Pro Pro Phe Ser Gly
        1395                1400                1405
Ser Arg Cys Glu Leu Tyr Thr Ala Pro Pro Ser Thr Pro Pro Ala Thr
    1410                1415                1420
Cys Leu Ser Gln Tyr Cys Ala Asp Lys Ala Arg Asp Gly Val Cys Asp
1425                1430                1435                1440
Glu Ala Cys Asn Ser His Ala Cys Gln Trp Asp Gly Gly Asp Cys Ser
                1445                1450                1455
Leu Thr Met Glu Asn Pro Trp Ala Asn Cys Ser Ser Pro Leu Pro Cys
            1460                1465                1470
Trp Asp Tyr Ile Asn Asn Gln Cys Asp Glu Leu Cys Asn Thr Val Glu
        1475                1480                1485
Cys Leu Phe Asp Asn Phe Glu Cys Gln Gly Asn Ser Lys Thr Cys Lys
    1490                1495                1500
Tyr Asp Lys Tyr Cys Ala Asp His Phe Lys Asp Asn His Cys Asp Gln
1505                1510                1515                1520
Gly Cys Asn Ser Glu Glu Cys Gly Trp Asp Gly Leu Asp Cys Ala Ala
                1525                1530                1535
Asp Gln Pro Glu Asn Leu Ala Glu Gly Thr Leu Val Ile Val Val Leu
            1540                1545                1550
Met Pro Pro Glu Gln Leu Leu Gln Asp Ala Arg Ser Phe Leu Arg Ala
        1555                1560                1565
Leu Gly Thr Leu Leu His Thr Asn Leu Arg Ile Lys Arg Asp Ser Gln
    1570                1575                1580
Gly Glu Leu Met Val Tyr Pro Tyr Tyr Gly Glu Lys Ser Ala Ala Met
1585                1590                1595                1600
Lys Lys Gln Arg Met Thr Arg Arg Ser Leu Pro Gly Glu Gln Glu Gln
                1605                1610                1615
Glu Val Ala Gly Ser Lys Val Phe Leu Glu Ile Asp Asn Arg Gln Cys
            1620                1625                1630
Val Gln Asp Ser Asp His Cys Phe Lys Asn Thr Asp Ala Ala Ala Ala
        1635                1640                1645
Leu Leu Ala Ser His Ala Ile Gln Gly Thr Leu Ser Tyr Pro Leu Val
    1650                1655                1660
Ser Val Val Ser Glu Ser Leu Thr Pro Glu Arg Thr Gln Leu Leu Tyr
1665                1670                1675                1680
Leu Leu Ala Val Ala Val Val Ile Ile Leu Phe Ile Ile Leu Leu Gly
                1685                1690                1695
Val Ile Met Ala Lys Arg Lys Arg Lys His Gly Ser Leu Trp Leu Pro
            1700                1705                1710
Glu Gly Phe Thr Leu Arg Arg Asp Ala Ser Asn His Lys Arg Arg Glu
        1715                1720                1725
Pro Val Gly Gln Asp Ala Val Gly Leu Lys Asn Leu Ser Val Gln Val
    1730                1735                1740
Ser Glu Ala Asn Leu Ile Gly Thr Gly Thr Ser Glu His Trp Val Asp
1745                1750                1755                1760
Asp Glu Gly Pro Gln Pro Lys Lys Val Lys Ala Glu Asp Glu Ala Leu
                1765                1770                1775
Leu Ser Glu Glu Asp Asp Pro Ile Asp Arg Arg Pro Trp Thr Gln Gln
            1780                1785                1790
His Leu Glu Ala Ala Asp Ile Arg Arg Thr Pro Ser Leu Ala Leu Thr
        1795                1800                1805
Pro Pro Gln Ala Glu Gln Glu Val Asp Val Leu Asp Val Asn Val Arg
    1810                1815                1820

Gly Pro Asp Gly Cys Thr Pro Leu Met Leu Ala Ser Leu Arg Gly Gly
1825                1830                1835                1840
Ser Ser Asp Leu Ser Asp Glu Asp Glu Asp Ala Glu Asp Ser Ser Ala
                1845                1850                1855
Asn Ile Ile Thr Asp Leu Val Tyr Gln Gly Ala Ser Leu Gln Ala Gln
            1860                1865                1870
Thr Asp Arg Thr Gly Glu Met Ala Leu His Leu Ala Ala Arg Tyr Ser
        1875                1880                1885
Arg Ala Asp Ala Ala Lys Arg Leu Leu Asp Ala Gly Ala Asp Ala Asn
    1890                1895                1900
Ala Gln Asp Asn Met Gly Arg Cys Pro Leu His Ala Ala Val Ala Ala
1905                1910                1915                1920
Asp Ala Gln Gly Val Phe Gln Ile Leu Ile Arg Asn Arg Val Thr Asp
                1925                1930                1935
Leu Asp Ala Arg Met Asn Asp Gly Thr Thr Pro Leu Ile Leu Ala Ala
            1940                1945                1950
Arg Leu Ala Val Glu Gly Met Val Ala Glu Leu Ile Asn Cys Gln Ala
        1955                1960                1965
Asp Val Asn Ala Val Asp Asp His Gly Lys Ser Ala Leu His Trp Ala
    1970                1975                1980
Ala Ala Val Asn Asn Val Glu Ala Thr Leu Leu Leu Leu Lys Asn Gly
1985                1990                1995                2000
Ala Asn Arg Asp Met Gln Asp Asn Lys Glu Glu Thr Pro Leu Phe Leu
                2005                2010                2015
Ala Ala Arg Glu Gly Ser Tyr Glu Ala Ala Lys Ile Leu Leu Asp His
            2020                2025                2030
Phe Ala Asn Arg Asp Ile Thr Asp His Met Asp Arg Leu Pro Arg Asp
        2035                2040                2045
Val Ala Arg Asp Arg Met His His Asp Ile Val Arg Leu Leu Asp Glu
    2050                2055                2060
Tyr Asn Val Thr Pro Ser Pro Pro Gly Thr Val Leu Thr Ser Ala Leu
2065                2070                2075                2080
Ser Pro Val Ile Cys Gly Pro Asn Arg Ser Phe Leu Ser Leu Lys His
                2085                2090                2095
Thr Pro Met Gly Lys Lys Ser Arg Arg Pro Ser Ala Lys Ser Thr Met
            2100                2105                2110
Pro Thr Ser Leu Pro Asn Leu Ala Lys Glu Ala Lys Asp Ala Lys Gly
        2115                2120                2125
Ser Arg Arg Lys Lys Ser Leu Ser Glu Lys Val Gln Leu Ser Glu Ser
    2130                2135                2140
Ser Val Thr Leu Ser Pro Val Asp Ser Leu Glu Ser Pro His Thr Tyr
2145                2150                2155                2160
Val Ser Asp Thr Thr Ser Ser Pro Met Ile Thr Ser Pro Gly Ile Leu
                2165                2170                2175
Gln Ala Ser Pro Asn Pro Met Leu Ala Thr Ala Ala Pro Pro Ala Pro
            2180                2185                2190
Val His Ala Gln His Ala Leu Ser Phe Ser Asn Leu His Glu Met Gln
        2195                2200                2205
Pro Leu Ala His Gly Ala Ser Thr Val Leu Pro Ser Val Ser Gln Leu
    2210                2215                2220
Leu Ser His His His Ile Val Ser Pro Gly Ser Gly Ser Ala Gly Ser
2225                2230                2235                2240
Leu Ser Arg Leu His Pro Val Pro Val Pro Ala Asp Trp Met Asn Arg
                2245                2250                2255
Met Glu Val Asn Glu Thr Gln Tyr Asn Glu Met Phe Gly Met Val Leu
            2260                2265                2270
Ala Pro Ala Glu Gly Thr His Pro Gly Ile Ala Pro Gln Ser Arg Pro
        2275                2280                2285
Pro Glu Gly Lys His Ile Thr Thr Pro Arg Glu Pro Leu Pro Pro Ile
    2290                2295                2300
Val Thr Phe Gln Leu Ile Pro Lys Gly Ser Ile Ala Gln Pro Ala Gly
2305                2310                2315                2320
Ala Pro Gln Pro Gln Ser Thr Cys Pro Pro Ala Val Ala Gly Pro Leu

2325                    2330                    2335
Pro Thr Met Tyr Gln Ile Pro Glu Met Ala Arg Leu Pro Ser Val Ala
            2340                    2345                    2350
Phe Pro Thr Ala Met Met Pro Gln Gln Asp Gly Gln Val Ala Gln Thr
        2355                    2360                    2365
Ile Leu Pro Ala Tyr His Pro Phe Pro Ala Ser Val Gly Lys Tyr Pro
    2370                    2375                    2380
Thr Pro Pro Ser Gln His Ser Tyr Ala Ser Ser Asn Ala Ala Glu Arg
2385                    2390                    2395                    2400
Thr Pro Ser His Ser Gly His Leu Gln Gly Glu His Pro Tyr Leu Thr
                2405                    2410                    2415
Pro Ser Pro Glu Ser Pro Asp Gln Trp Ser Ser Ser Ser Pro His Ser
            2420                    2425                    2430
Ala Ser Asp Trp Ser Asp Val Thr Thr Ser Pro Thr Pro Gly Gly Ala
        2435                    2440                    2445
Gly Gly Gly Gln Arg Gly Pro Gly Thr His Met Ser Glu Pro Pro His
    2450                    2455                    2460
Asn Asn Met Gln Val Tyr Ala
2465                    2470

<210> 87
<211> 884
<212> PRT
<213> Homo sapiens

<220>
<223> N2TM truncation

<400> 87
Met Val Tyr Pro Tyr Tyr Gly Glu Lys Ser Ala Ala Met Lys Lys Gln
1               5                   10                  15
Arg Met Thr Arg Arg Ser Leu Pro Gly Glu Gln Glu Gln Glu Val Ala
            20                  25                  30
Gly Ser Lys Val Phe Leu Glu Ile Asp Asn Arg Gln Cys Val Gln Asp
        35                  40                  45
Ser Asp His Cys Phe Lys Asn Thr Asp Ala Ala Ala Ala Leu Leu Ala
    50                  55                  60
Ser His Ala Ile Gln Gly Thr Leu Ser Tyr Pro Leu Val Ser Val Val
65                  70                  75                  80
Ser Glu Ser Leu Thr Pro Glu Arg Thr Gln Leu Leu Tyr Leu Leu Ala
                85                  90                  95
Val Ala Val Val Ile Ile Leu Phe Ile Ile Leu Leu Gly Val Ile Met
            100                 105                 110
Ala Lys Arg Lys Arg Lys His Gly Ser Leu Trp Leu Pro Glu Gly Phe
        115                 120                 125
Thr Leu Arg Arg Asp Ala Ser Asn His Lys Arg Arg Glu Pro Val Gly
    130                 135                 140
Gln Asp Ala Val Gly Leu Lys Asn Leu Ser Val Gln Val Ser Glu Ala
145                 150                 155                 160
Asn Leu Ile Gly Thr Gly Thr Ser Glu His Trp Val Asp Asp Glu Gly
                165                 170                 175
Pro Gln Pro Lys Lys Val Lys Ala Glu Asp Glu Ala Leu Leu Ser Glu
            180                 185                 190
Glu Asp Asp Pro Ile Asp Arg Arg Pro Trp Thr Gln Gln His Leu Glu
        195                 200                 205
Ala Ala Asp Ile Arg Arg Thr Pro Ser Leu Ala Leu Thr Pro Pro Gln
    210                 215                 220
Ala Glu Gln Glu Val Asp Val Leu Asp Val Asn Val Arg Gly Pro Asp
225                 230                 235                 240
Gly Cys Thr Pro Leu Met Leu Ala Ser Leu Arg Gly Gly Ser Ser Asp
                245                 250                 255
Leu Ser Asp Glu Asp Glu Asp Ala Glu Asp Ser Ser Ala Asn Ile Ile
            260                 265                 270

Thr Asp Leu Val Tyr Gln Gly Ala Ser Leu Gln Ala Gln Thr Asp Arg
            275                 280                 285
Thr Gly Glu Met Ala Leu His Leu Ala Ala Arg Tyr Ser Arg Ala Asp
    290                 295                 300
Ala Ala Lys Arg Leu Leu Asp Ala Gly Ala Asp Ala Asn Ala Gln Asp
305                 310                 315                 320
Asn Met Gly Arg Cys Pro Leu His Ala Ala Val Ala Ala Asp Ala Gln
                325                 330                 335
Gly Val Phe Gln Ile Leu Ile Arg Asn Arg Val Thr Asp Leu Asp Ala
            340                 345                 350
Arg Met Asn Asp Gly Thr Thr Pro Leu Ile Leu Ala Ala Arg Leu Ala
        355                 360                 365
Val Glu Gly Met Val Ala Glu Leu Ile Asn Cys Gln Ala Asp Val Asn
    370                 375                 380
Ala Val Asp Asp His Gly Lys Ser Ala Leu His Trp Ala Ala Ala Val
385                 390                 395                 400
Asn Asn Val Glu Ala Thr Leu Leu Leu Leu Lys Asn Gly Ala Asn Arg
                405                 410                 415
Asp Met Gln Asp Asn Lys Glu Glu Thr Pro Leu Phe Leu Ala Ala Arg
            420                 425                 430
Glu Gly Ser Tyr Glu Ala Ala Lys Ile Leu Leu Asp His Phe Ala Asn
        435                 440                 445
Arg Asp Ile Thr Asp His Met Asp Arg Leu Pro Arg Asp Val Ala Arg
    450                 455                 460
Asp Arg Met His His Asp Ile Val Arg Leu Leu Asp Glu Tyr Asn Val
465                 470                 475                 480
Thr Pro Ser Pro Pro Gly Thr Val Leu Thr Ser Ala Leu Ser Pro Val
                485                 490                 495
Ile Cys Gly Pro Asn Arg Ser Phe Leu Ser Leu Lys His Thr Pro Met
            500                 505                 510
Gly Lys Lys Ser Arg Arg Pro Ser Ala Lys Ser Thr Met Pro Thr Ser
        515                 520                 525
Leu Pro Asn Leu Ala Lys Glu Ala Lys Asp Ala Lys Gly Ser Arg Arg
    530                 535                 540
Lys Lys Ser Leu Ser Glu Lys Val Gln Leu Ser Glu Ser Ser Val Thr
545                 550                 555                 560
Leu Ser Pro Val Asp Ser Leu Glu Ser Pro His Thr Tyr Val Ser Asp
                565                 570                 575
Thr Thr Ser Ser Pro Met Ile Thr Ser Pro Gly Ile Leu Gln Ala Ser
            580                 585                 590
Pro Asn Pro Met Leu Ala Thr Ala Ala Pro Pro Ala Pro Val His Ala
        595                 600                 605
Gln His Ala Leu Ser Phe Ser Asn Leu His Glu Met Gln Pro Leu Ala
    610                 615                 620
His Gly Ala Ser Thr Val Leu Pro Ser Val Ser Gln Leu Leu Ser His
625                 630                 635                 640
His His Ile Val Ser Pro Gly Ser Gly Ser Ala Gly Ser Leu Ser Arg
                645                 650                 655
Leu His Pro Val Pro Val Pro Ala Asp Trp Met Asn Arg Met Glu Val
            660                 665                 670
Asn Glu Thr Gln Tyr Asn Glu Met Phe Gly Met Val Leu Ala Pro Ala
        675                 680                 685
Glu Gly Thr His Pro Gly Ile Ala Pro Gln Ser Arg Pro Pro Glu Gly
    690                 695                 700
Lys His Ile Thr Thr Pro Arg Glu Pro Leu Pro Pro Ile Val Thr Phe
705                 710                 715                 720
Gln Leu Ile Pro Lys Gly Ser Ile Ala Gln Pro Ala Gly Ala Pro Gln
                725                 730                 735
Pro Gln Ser Thr Cys Pro Pro Ala Val Ala Gly Pro Leu Pro Thr Met
            740                 745                 750
Tyr Gln Ile Pro Glu Met Ala Arg Leu Pro Ser Val Ala Phe Pro Thr
        755                 760                 765
Ala Met Met Pro Gln Gln Asp Gly Gln Val Ala Gln Thr Ile Leu Pro

770                 775                 780
Ala Tyr His Pro Phe Pro Ala Ser Val Gly Lys Tyr Pro Thr Pro Pro
785                 790                 795                 800
Ser Gln His Ser Tyr Ala Ser Ser Asn Ala Ala Glu Arg Thr Pro Ser
                805                 810                 815
His Ser Gly His Leu Gln Gly Glu His Pro Tyr Leu Thr Pro Ser Pro
            820                 825                 830
Glu Ser Pro Asp Gln Trp Ser Ser Ser Ser Pro His Ser Ala Ser Asp
        835                 840                 845
Trp Ser Asp Val Thr Thr Ser Pro Thr Pro Gly Gly Ala Gly Gly Gly
    850                 855                 860
Gln Arg Gly Pro Gly Thr His Met Ser Glu Pro Pro His Asn Asn Met
865                 870                 875                 880
Gln Val Tyr Ala

<210> 88
<211> 773
<212> PRT
<213> Homo sapiens

<220>
<223> N2IC truncation

<400> 88

## Description

The present invention relates to the field of genotyping and treating cancers such as leukemia and lymphoma, especially chronic lymphocytic leukemia (CLL).

CLL is the most common form of adult hematologic malignancies occurring in the Western hemisphere. For instance, in the USA, prevalence of CLL was 126,299 as of January 2011 - approximately 0.6 percent of men and women will be diagnosed with CLL at some point during their lifetime. The median age of diagnosis lies between 70 and 75 years. Men are affected almost twice as often as women and there is a substantial geographic variation in CLL incidence, with high incidences in North America and Europe. Both hereditary and acquired factors, such as being exposed to organic solvents, as e.g. benzene, are considered as risk factors. Common symptoms include lymph node enlargement, constitutional symptoms and bone marrow failure, though most patients are asymptomatic at diagnosis.

Prognostication and treatment is based on different features, and different subgroups, of the disease and the patient. An antineoplastic treatment is usually indicated only when symptoms are present. Typical therapies are a combination treatment with fludarabin, cyclophosphamide and rituximab or with bendamustin and rituximab; the latter is usually administered to patients with impaired kidney function and uncontrolled autoimmune hemolysis.

CLL shows a clinically and biologically heterogeneous disease pattern. However, it has a characteristic immunophenotype, enabling a differentiation from other B cell lymphomas (Zenz et al., 2010). CLL risk loci at chromosome bands 2q13, 2q37.1, 6p25.3, 11q24.1, 15q23 and 19q13.32 have been identified in a genome-wide association study (Di Bernardo et al., 2008). Importantly, different genotypes in different CLL forms have an influence on the success of a specific therapy. 17p13 and 11q23 deletions, TP53 mutation and immunoglobulin (Ig) heavy chain (IGHV) mutation status are known prognostic factors (Zenz et al., 2010; Eichhorst et al., 2015). For example, patients with 17p13 deletion or TP53 deletion have a lower response rate and a shorter survival rate after chemo-immunotherapy. Moreover, it has been shown that patients that have CLL with unmutated IGHVs show poorer survival than CLL patients with mutated IGHVs. Furthermore, gene mutations in SF3B1, MYD88, NOTCH1 or BIRC3 have also been suggested to predict an unfavourable prognosis in the absence of TP53 mutation (Eichhorst et al., 2015; Stilgenbauer et al., 2014; Villamor et al., 2013).

Despite the availability of various treatment options, there is still a lack of effective medicaments and treatment regimens for CLL (as well as other leukemias and lymphoma, and cancer in general). In addition, reliable prognostic factors based on CLL genotype are directly needed, also as a basis for treatment decisions. Therefore, it is an object of the present invention to provide novel tools and methods for genotyping CLL (and other cancers), in particular for predicting disease progression and informing treatment decisions. A further object of the invention is to provide tools and methods for targeted treatment of CLL (and other cancers) based on cancer genotype.

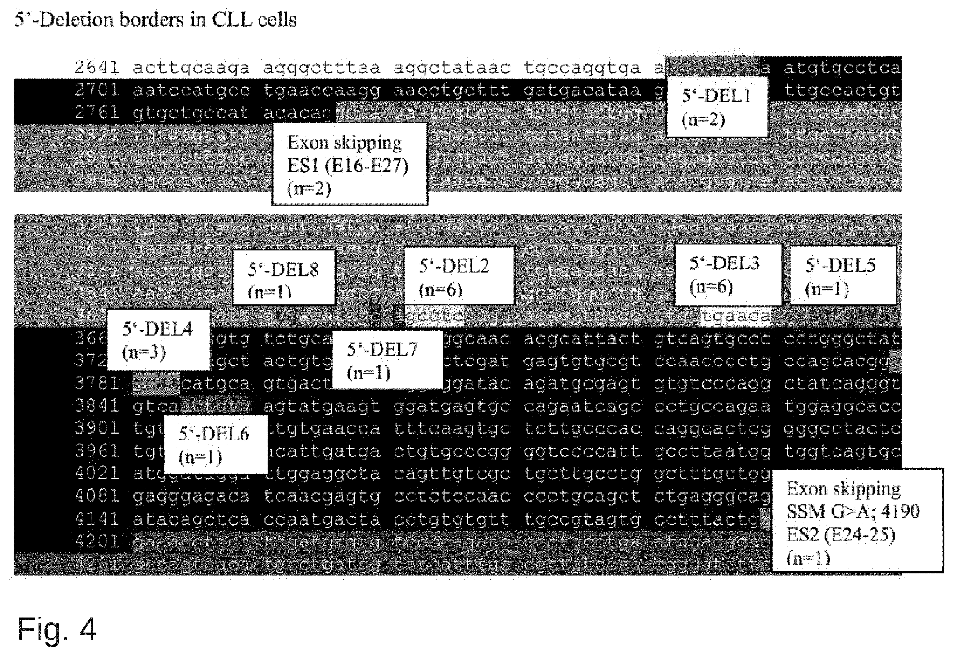

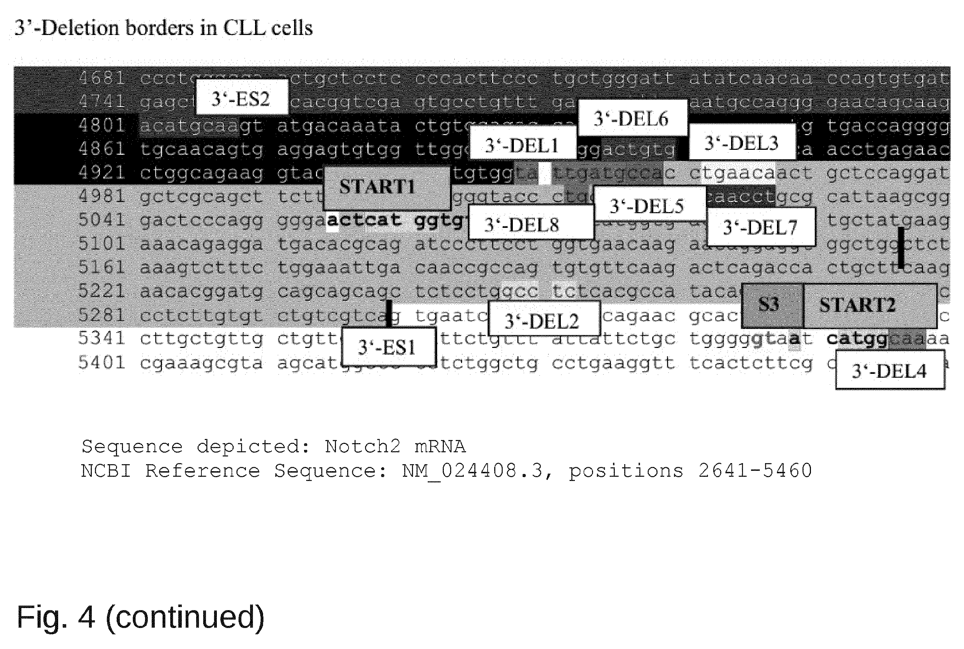

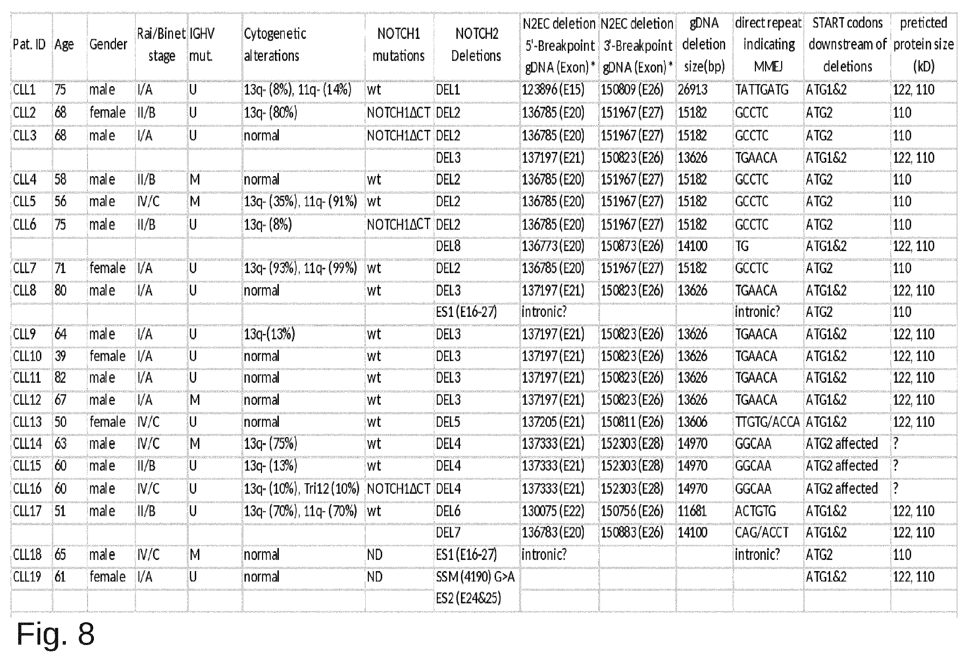

Surprisingly, in the course of the present invention, deletions in the NOTCH2 gene region coding for the NOTCH2 extracellular domain were found in cells obtained from blood samples of CLL patients (Figs. 4 and 8).

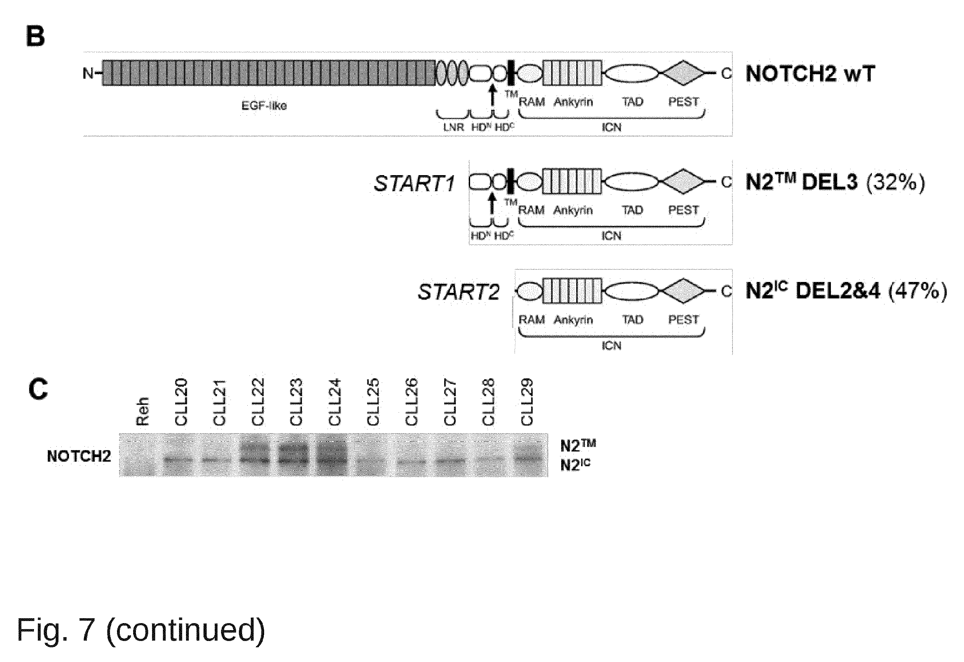

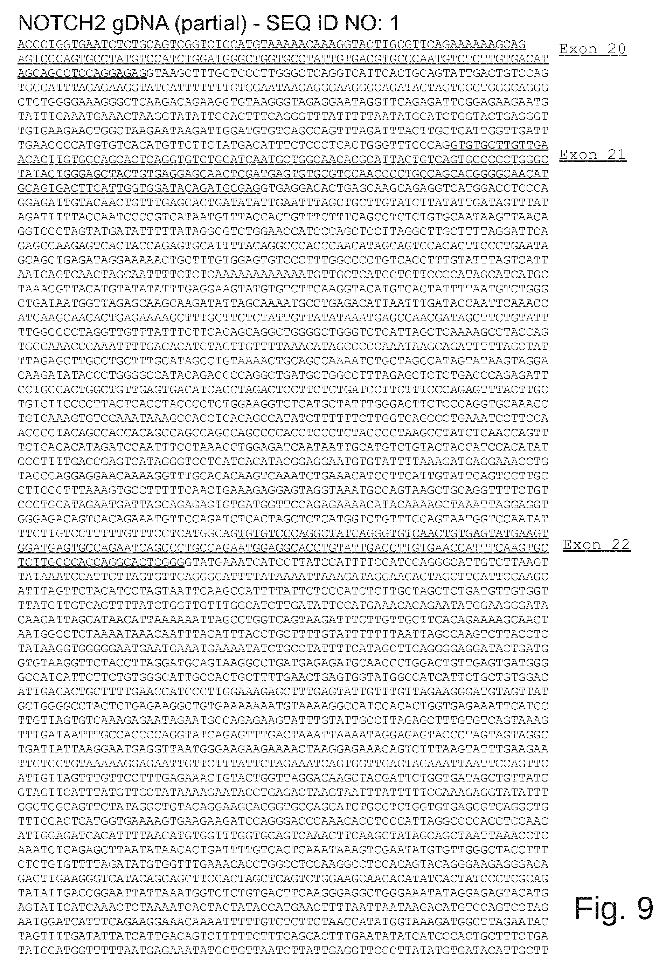

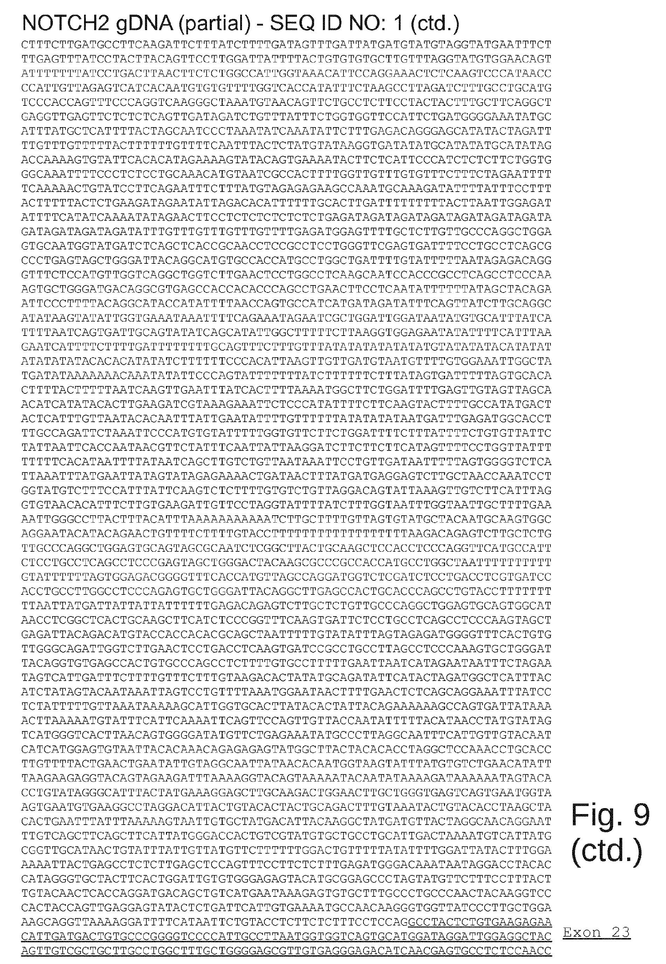

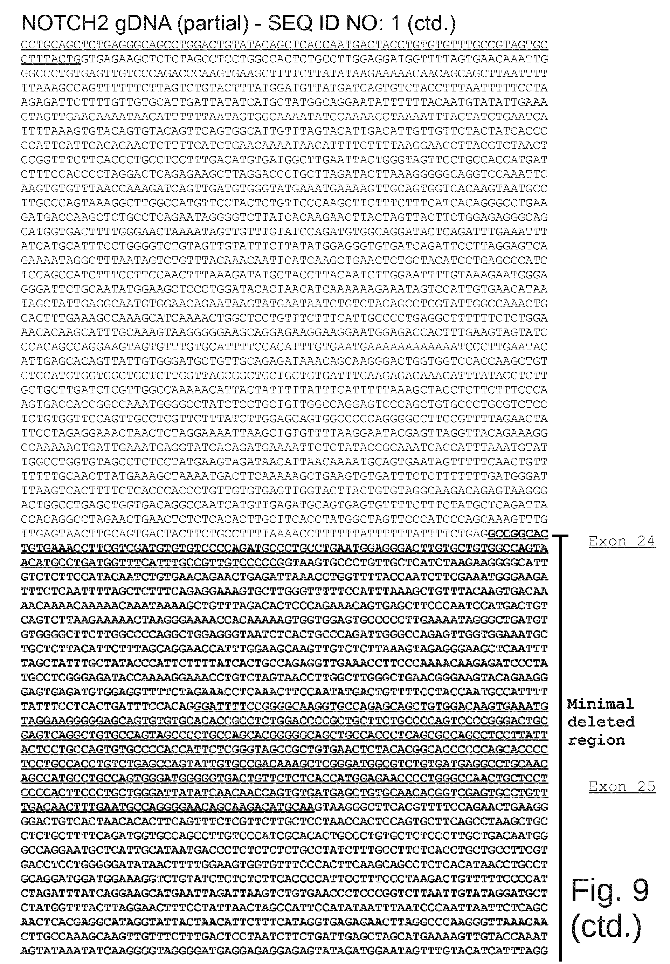

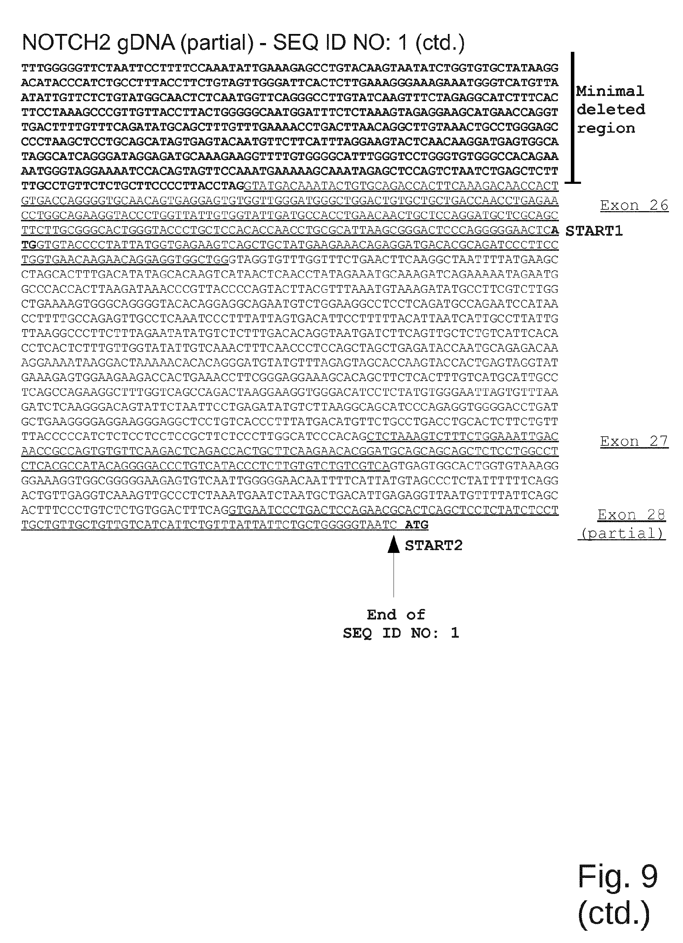

More specifically, it was found in the course of the present invention that peripheral blood mononuclear cells (PBMCs) obtained from CLL patients have deletions at specific hotspots in Notch2 (Figs. 4, 6 and 8), leading to truncated forms of Notch2 having alternative translational start codons ("START1" and "START2"; Fig. 7B or Fig. 9). START1 is in the extracellular domain of Notch2 and START2 in the intracellular domain, directly adjacent to the transmembrane domain. Remarkably, both Notch2 truncations can be expected to lead to ligand-independent activation of Notch2: most likely, the truncated Notch2 protein translated from START1 (also called "N2^{IC} truncation" herein, with "N2" being the abbreviation for Notch 2 and "IC" being the abbreviation for intracellular) is still anchored to the transmembrane domain and, due to the lack of the auto inhibitory LNR repeats, eventually processed by ADAM10 metalloprotease at S2, and γ-secretase at S3, to create an active Notch2 protein without requiring ligand binding. The truncated Notch2 protein translated from START2 (also called "N2^{™} truncation" herein, with "N2" being the abbreviation for Notch 2 and "TM" being the abbreviation for transmembrane) can be expected not to be anchored to the cell membrane and thus to be γ-secretase independent (and, due to the lack of extracellular domains, also to be ligand independent). Both N-terminal Notch2 truncations (i.e. the N2^{™} truncation and the N2^{IC} truncation) result in gain-of-function phenotypes due to their independence of a ligand. These NOTCH2 deletions leading to the Notch2 truncations and discovered in the course of the present invention have the potential to be the CLL-initiating event and, thus, to be the disease-defining malignant hit in a CLL precursor cell. The present invention provides a polynucleotide probe for detecting a region of the human NOTCH2 gene in a cell, the sequence of the region being at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 1. The probe is specific for said region or for its reverse complement. Furthermore, the probe is bound to a molecular tag such as a fluorescent tag. Alternatively, said sequence according to SEQ ID NO: 1 can be according to National Center for Biotechnology Information (NCBI) reference sequence NG_008163.1, position 136644-152299, or identified by "the sequence starting with the first nucleotide of exon 20 and ending with the nucleotide just upstream of alternative start codon START2 of the human NOTCH2 gene" or according to the sequence depicted in Fig. 9 (without the final ATG).

The polynucleotide probe can be used for detecting such Notch2 deletions leading to truncated versions of Notch2 and thus is useful in CLL diagnosis or genotyping, in particular for prediction of disease progression or informing treatment decisions.

In a further aspect, the present invention provides a polynucleotide probe, wherein at least 75%, preferably at least 80%, more preferably at least 85%, even more preferably at least 90%, especially at least 95% or even 100% of the sequence of the probe consist of at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 1 or its reverse complement. Furthermore, the probe is bound to a molecular tag such as a fluorescent tag. Alternatively, said sequence according to SEQ ID NO: 1 can be according to NCBI reference sequence NG_008163.1, position 136644-152299, or identified by "the sequence starting with the first nucleotide of exon 20 and ending with the nucleotide just upstream of alternative start codon START2 of the human NOTCH2 gene" or according to the sequence depicted in Fig. 9 (without the final ATG). In another aspect of the present invention, a container such as a vial, comprising a polynucleotide probe composition especially for fluorescence in-situ hybridization (FISH) is provided. The polynucleotide probe composition comprises at least one polynucleotide probe as defined herein, preferably at least two different polynucleotide probes as defined herein, and preferably a buffer in which the probes are dissolved. In particular, the composition is for determining whether a cell has a human NOTCH2 gene with a deletion starting at a locus in or downstream of exon 20 and ending at a locus upstream of the alternative start codon START2 (further identified e.g. by SEQ ID NO: 1 or SEQ ID NO: 2, respectively; or by NCBI reference sequence NG_008163.1, position 136644-152299 or position 136798-152299, respectively), preferably wherein the deletion encompasses at least a portion of the genomic region starting with the first nucleotide of exon 24 and ending with the last nucleotide upstream of exon 26 (the region alternatively according to SEQ ID NO: 3; or to NCBI reference sequence NG_008163.1, position 148043-149349).

Preferably, the container is sterile on the inside in order to increase storage stability. Beneficially, the container is lightproof (preferably in the UV-VIS range), also to increase stability of the probes.

In a further aspect, the present invention relates to the use of the polynucleotide probe or polynucleotide probe composition (of the container) as defined herein for determining whether a cell has a human NOTCH2 gene with a deletion starting at a locus in or downstream of exon 20 and ending at a locus upstream of the alternative start codon START2 (further identified e.g. by SEQ ID NO: 1 or SEQ ID NO: 2, respectively; or by NCBI reference sequence NG_008163.1, position 136644-152299 or position 136798-152299, respectively). In particular, the deletion encompasses at least a portion of the genomic region starting with the first nucleotide of exon 24 and ending with the last nucleotide upstream of exon 26 (the region alternatively according to SEQ ID NO:3; or to NCBI reference sequence NG_008163.1, position 148043-149349). The cell is preferably a cancer cell or a cell suspected of being a cancer cell. The probe or composition can especially be used for designating the cell as having a gain-of-function NOTCH2 mutation caused by the deletion, or for determining whether the patient from whom the cell was obtained is eligible for a therapy that targets NOTCH2, in particular a gliotoxin-based therapy.

In a still further aspect, the invention relates to a method for detecting the presence or absence of a region of a human NOTCH2 gene on a chromosome of a cell, the sequence of the region being at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 1, the method comprising
- providing the cell,
- contacting a probe specific for the region or for its reverse complement with the chromosome or a fragment thereof
- detecting whether the probe is bound to the chromosome or fragment thereof;
preferably for determining whether the NOTCH2 gene has a deletion starting at a locus in or downstream of Exon20 and ending at a locus upstream of the alternative start codon START2"] (further identified e.g. by SEQ ID NO: 1 or SEQ ID NO: 2, respectively; or by NCBI reference sequence NG_008163.1, position 136644-152299 or position 136798-152299, respectively), wherein the deletion comprises said region. Alternatively, said sequence according to SEQ ID NO: 1 can be according to NCBI reference sequence NG_008163.1, position 136644-152299, or identified by "the sequence starting with the first nucleotide of exon 20 and ending with the nucleotide just upstream of alternative start codon START2 of the human NOTCH2 gene" or according to the sequence depicted in Fig. 9 (without the final ATG).

The present invention further relates to a NOTCH2 signalling pathway inhibitor, in particular a gliotoxin, for use in treatment of cancer or prevention of cancer relapse in a patient, wherein at least a portion of the cells of the cancer have a human NOTCH2 gene with a deletion starting at a locus in or downstream of exon 20 and ending at a locus upstream of the alternative start codon START2 (further identified e.g. by SEQ ID NO: 1 or SEQ ID NO: 2, respectively; or by NCBI reference sequence NG_008163.1, position 136644-152299 or position 136798-152299, respectively).

In a further aspect, the present invention provides a gamma-secretase inhibitor for use in treatment of cancer or prevention of cancer relapse in a patient, wherein at least a portion of the cells of the cancer have a human NOTCH2 gene with a deletion starting at a locus in or downstream of exon 20 and ending at a locus upstream of the alternative start codon START1 (further identified e.g. by SEQ ID NO: 7 or SEQ ID NO: 85, respectively; or by NCBI reference sequence NG_008163.1, position 136644-150919 or position 136798-150919, respectively).

In another aspect, the present invention relates to an antibody specific for an N-terminally truncated human Notch2 protein starting with the sequence MVYP (SEQ ID NO: 4) or the sequence MAKR (SEQ ID NO: 5). This antibody can be used in diagnosis, prognosis and targeted treatment of cancers such as CLL.

NOTCH2 belongs to a family of highly conserved transmembrane receptors that modulate a wide variety of differentiation processes including binary lineage specifications and adult tissue homeostasis. NOTCH receptors are short range signalling molecules which are stimulated by ligands, e.g. Delta or Jagged, expressed on adjacent cells (Bray, 2006). After ligand binding, the intracellular domain of NOTCH (N^{IC}) is cleaved by a series of proteolytic events involving γ-secretase followed by translocation to the nucleus where it acts as context dependent transcription factor on genes involved in differentiation, apoptosis regulation, and metabolism (Louvi and Artavanis-Tsakonas, 2012; Ntziachristos et al., 2013). Deregulation of NOTCH signalling is observed in an increasing number of human neoplasias (Hubmann et al., 2002; Rosati et al., 2009). Therefore, targeting oncogenic NOTCH activity, for example with γ-secretase inhibitors (GSIs), represents a suitable therapeutic strategy in the treatment of NOTCH expressing tumours/leukaemias.

CLL cells typically express constitutively activated NOTCH2 which is not only associated with the expression of the B-cell activation/differentiation marker CD23 but is also functionally linked with CLL cell viability (Hubmann et al., 2012; Willander et al., 2013).

Several methods are known that target DNA sequences, partly of Notch components, to diagnose, predict or treat cancer:
WO 2003/039443 A2 relates to methods for detecting leukemia cells by determining the expression profile of a group of markers, including NOTCH2.
WO 2015/092614 A1 provides methods of identifying and treating cancer patients having activating alterations in the PEST domain of Notch1, Notch2 and Notch3.
US 2013/0210014 A1 relates to a method for determining the prognosis for or predicting responsiveness to a therapeutic regimen in a subject diagnosed with CLL.
WO 2005/031001 A2 discloses sequences for use in detection, diagnosis and treatment of cancers, especially lymphomas.

However, neither of them disclose or suggest the present invention.

Notch is a type I transmembrane receptor that has the four homologs Notch 1-4 in mammals. The receptor has an extracellular domain of up to 36 epidermal growth factor repeats, followed by Lin repeats (LNR) and a heterodimerization domain (HD), which together build the negative regulatory region (NRR). The transmembrane domain (TMD) is followed by an intracellular domain, consisting of nuclear localization signals (NLS), a RAM domain, ankyrin (Ank) repeats, a transactivation domain (TAD) and a C-terminal proline glutamic acid serine and threonine (PEST) degradation domain (Chillakuri et al., 2012).

Components of the Notch signalling pathway have been shown to be mutated and/or deregulated in several common cancers. (Lee et al., 2007; Kiel, 2012; Li et al., 2013; Willander et al., 2013; Ashworth et al., 2015). In T cell acute lymphoblastic leukemia (T-ALL), translocations that remove a large portion of the ECD, as well as HD domain mutations involving Notch1, have been reported to disrupt the normal function of the ECD/NRR and effectively lead to ligand-independent Notch activation. (Ellisen et al., 1991; Weng et al., 2004. Inactivating PEST domain mutations in Notch have been reported in T-ALL, chronic lymphocytic leukemia (CLL), splenic marginal zone lymphoma (SMZL) and mantle cell lymphoma (see e.g. Weng et al. (2004); Kridel et al., 2012; Puente et al., 2011;; Rossi et al. (2012); Lee et al., 2009; Lohr et al., 2012; Rossi et al. (2012); Kiel et al., 2012; Isidor et al., 2011; Simpson et al., 2011; Westhoff et al., 2009)). A PEST domain mutation in one breast cancer sample has been reported, but was not characterized and the functional relevance of this mutation is unknown. (Jiao et al., 2012). Thus, Notch signalling has a major role in tumorigenesis.

In CLL, the most common form of leukemias in the Western world, Notch1 and Notch2 are typically constitutively active (Hubmann et al., 2012; Li et al., 2013; Willander et al., 2013). However, the connection between CLL pathogenesis and constitutive activity of Notch2 had not been elucidated before the present invention was made. The human NOTCH2 gene is located in the centromeric region on the short arm of chromosome 1 (1p13-p11). The mRNA sequence of human NOTCH2 can be accessed under the NCBI accession number NM_024408.3 and the genomic DNA (gDNA) sequence under NCBI accession number NG_008163.1. Previous studies have investigated the chromosomal region 1p12 as a prognostic marker for other cancers. Panani et al. (2004) identified 1p12 as an adverse prognostic factor in multiple myeloma patients. Arriba et al. (2015) showed that losses of 1p12 DNA copy number was significantly more frequent in younger patients diagnosed with colorectal cancer.

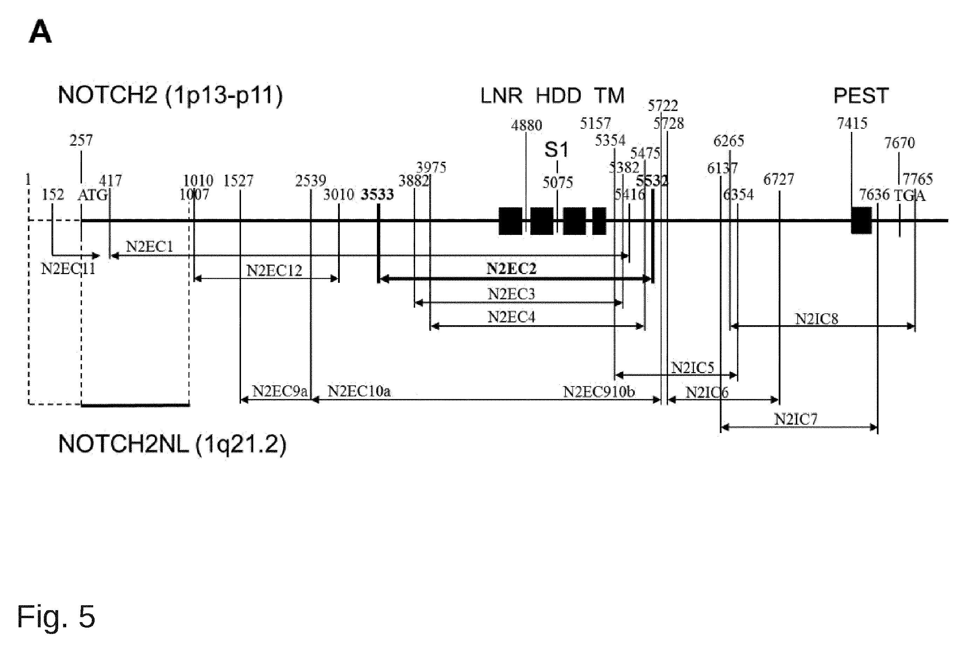

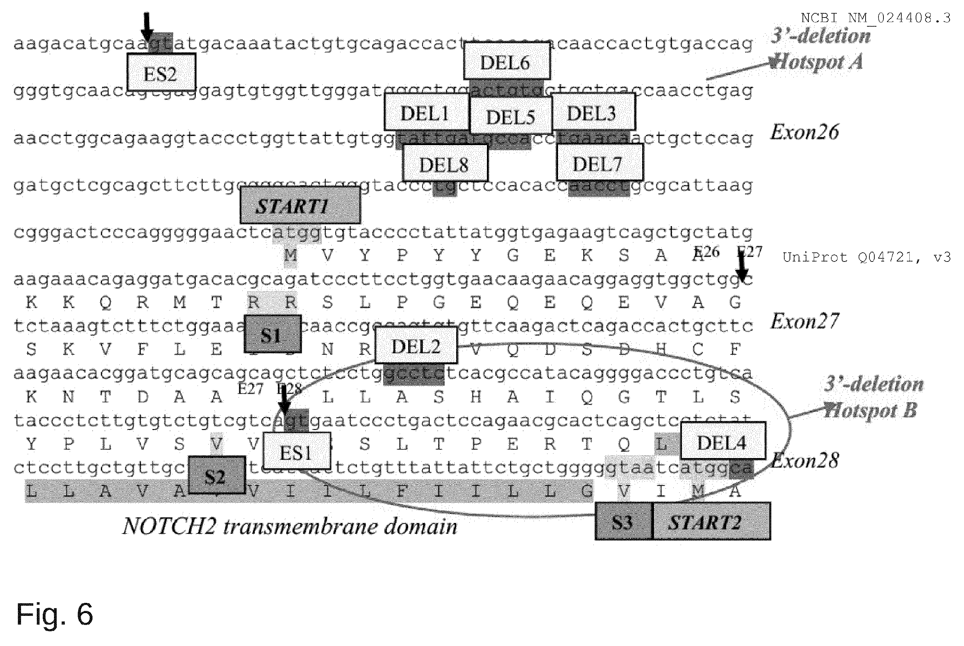

In the course of the present invention, the NOTCH2 gene was screened by a long range PCR approach on the cDNA level (Fig. 5A) and recurring Notch 2 extracellular (N2^{EC}) deletions in all CLL cases were found (Fig. 8). Interestingly, the breakpoints from the majority of detected deletions were identified in confined exonic sequences (5'-hot spot: exon 20/21; 3'-hot spots: exon 26-28) (see Fig. 6 and 8). Exon 24 to Exon 25 (together with the intron between exon 25 and exon 26) was identified as a minimal deleted region according to these experiments. Exon 26 marked the 3'-deletion hotspot A, exon28 marked the 3'-deletion hotspot B (see Fig. 6). These deletions are not known in the prior art - due to their size, this kind of deletions would not be detected by the currently available next generation gDNA sequencing (NGS) approaches and were also therefore overlooked by the field.

The polynucleotide probe of the present invention can be used to detect these mutations. The molecular tag bound to the polynucleotide probe of the present invention typically facilitates or enables this detection. Preferred molecular tags bound to the polynucleotide probe of the present invention include radioactive phosphates, biotin, enzymes and fluorescent tags (fluorophores). Preferably, the molecular tag is covalently attached to the probe, in particular via 5'-end labelling or via 3'-end labelling (see e.g. US 2015/252412 A1).

In a preferred embodiment, the sequence of the region of the human NOTCH2 gene to be detected is at least 11, at least 12, at least 13, at least 14, at least 15, at least 16, at least 17, at least 18, at least 19, at least 20, at least 21, at least 22, at least 23, at least 24, at least 25, at least 30, at least 40 or at least 50 consecutive nucleotides selected from the sequence according to SEQ ID NOs: 1, 2, 3, 6, 7 or 85. Preferably, the sequence of the region encompasses at least a portion (i.e. at least 1 nucleotide, preferably at least 2 nucleotides, more preferably at least 3 nucleotides, yet more preferably at least 5 nucleotides, even more preferably at least 7 nucleotides, yet even more preferably at least 10 nucleotides or even at least 15 nucleotides) of a human NOTCH2 exon sequence (especially as defined in Fig. 9), selected from any one of exons 20, 21, 22, 23, 24, 25, 26, 27 and 28, in particular from any one of exons 24 and 25. In particular, the sequence of the region is from a human NOTCH2 exon sequence (especially as defined in Fig. 9), selected from any one of exons 20, 21, 22, 23, 24, 25, 26, 27 and 28, in particular from any one of exons 24 and 25.

In another preferred embodiment, the sequence of the region of the human NOTCH2 gene to be detected is at least 11, preferably at least 15, more preferably at least 16, yet more preferably at least 17, even more preferably at least 18, yet even more preferably at least 19 or even at least 20 consecutive nucleotides selected from the sequence according to SEQ ID NOs: 1, 2, 3, 6, 7 or 85, preferably according to SEQ ID NOs: 3 or 6. Preferably, the sequence of the region encompasses at least a portion (i.e. at least 1 nucleotide, preferably at least 2 nucleotides, more preferably at least 3 nucleotides, yet more preferably at least 5 nucleotides, even more preferably at least 7 nucleotides, yet even more preferably at least 10 nucleotides or even at least 15 nucleotides) of a human NOTCH2 exon sequence (especially as defined in Fig. 9), selected from any one of exons 20, 21, 22, 23, 24, 25, 26, 27 and 28, in particular from any one of exons 24 and 25. In particular, the sequence of the region is from a human NOTCH2 exon sequence (especially as defined in Fig. 9), selected from any one of exons 20, 21, 22, 23, 24, 25, 26, 27 and 28, in particular from any one of exons 24 and 25.

In a particularly preferred embodiment, the sequence of the region of the human NOTCH2 gene to be detected is at least at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 3 (found to be a minimal deleted region in the course of the present invention). Preferably, the sequence of the region encompasses at least a portion (i.e. at least 1 nucleotide, preferably at least 2 nucleotides, more preferably at least 3 nucleotides, yet more preferably at least 5 nucleotides, even more preferably at least 7 nucleotides, yet even more preferably at least 10 nucleotides or even at least 15 nucleotides) of a human NOTCH2 exon sequence (especially as defined in Fig. 9), selected from any one of exons 24 and 25. In particular, the sequence of the region is from an exon sequence (especially as defined in Fig. 9), selected from any one of exons 24 and 25.

In another particularly preferred embodiment, the sequence of the region of the human NOTCH2 gene to be detected is at least at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 6. In the course of the present invention, it was found that deletions in this region can determine sensitivity to GSIs. Preferably, the sequence of the region encompasses at least a portion (i.e. at least 1 nucleotide, preferably at least 2 nucleotides, more preferably at least 3 nucleotides, yet more preferably at least 5 nucleotides, even more preferably at least 7 nucleotides, yet even more preferably at least 10 nucleotides or even at least 15 nucleotides) of a human NOTCH2 exon sequence (especially as defined in Fig. 9), selected from any one of exons 26, 27 and 28. In particular, the sequence of the region is from an exon sequence (especially as defined in Fig. 9), selected from any one of exons 26, 27 and 28.

In a further, especially preferred embodiment, the sequence of the region of the human NOTCH2 gene to be detected is at least 18 consecutive nucleotides selected from the sequence according to SEQ ID NOs: 1, 2, 3, 6, 7 or 85, preferably according to SEQ ID NOs: 3 or 6. Preferably, the sequence of the region encompasses at least a portion (i.e. at least 1 nucleotide, preferably at least 2 nucleotides, more preferably at least 3 nucleotides, yet more preferably at least 5 nucleotides, even more preferably at least 7 nucleotides, yet even more preferably at least 10 nucleotides or even at least 15 nucleotides) of a human NOTCH2 exon sequence (especially as defined in Fig. 9), selected from any one of exons 20, 21, 22, 23, 24, 25, 26, 27 and 28, in particular from any one of exons 24 and 25 or from any one of exons 26, 27 and 28. In particular, the sequence of the region is from a human NOTCH2 exon sequence (especially as defined in Fig. 9), selected from any one of exons 20, 21, 22, 23, 24, 25, 26, 27 and 28, in particular from any one of exons 24 and 25 or from any one of exons 26, 27 and 28.

The polynucleotide of the polynucleotide probe of the present invention can be DNA or RNA. One or more nucleotides of said polynucleotide can also carry chemical mutations, such as 5-methylcytosine, 5-hydroxymethylcytosine, 5-formylcytosine, 5-carboxycytosine, N6-methyladenine, pseudouridin or 5-methylcytidin. It is possible to convert DNA sequences into RNA sequences (exchange of nucleotides: T->U) and vice versa (exchange of nucleotides: U->T). Therefore, whenever a sequence is given as a DNA sequence herein (including the sequence listing), it shall also be read as the respective RNA sequence thereof and vice versa. The maximum length of the polynucleotide probe of the present invention may for instance be 30, 50, 100, 1000, 5000, or 10000 nucleotides. Preferably, the polynucleotide probe of the present invention is isolated.

In an especially preferred embodiment, the molecular tag bound to the polynucleotide of the present invention (preferably covalently attached to the probe, in particular via 5'-end labelling or via 3'-end labelling) is a fluorescent tag.

FISH is a common technique for the detection of nucleic acids in tissues, single cells or metaphase chromosomes. Common fluorescent dyes used for FISH are AMCA, Cy2, Cy3, Cy5, Texas Red or TRITC. FISH is a suitable technique for detecting cancer (especially CLL) in a patient, since only few cells obtained from the patient may need to be investigated so that CLL can be detected at an early stage. Cytogenetic methods in CLL (including FISH) are for instance described in Haferlach & Bacher, 2011. Interphase FISH has become a standard technique for cytogenetic analysis in CLL and was also used in a comprehensive characterization of CLL cases (see Haferlach et al., 2007). Interphase FISH is also described e.g. in Tibiletti, 2007. An interphase FISH protocol is disclosed e.g. in Buhmann et al., 2002 or in van Stedum & King, 2003.

The inventive FISH probe (i.e. a polynucleotide of the embodiment described directly above, suitable for FISH) preferably binds to a portion of the human NOTCH2 gene region according to SEQ ID NO: 3 (i.e. the region found to be a minimal deleted region in the course of the present invention), especially a portion of exon 24 or 25 (in particular as defined in Fig. 9) or a portion of the intron between exon 24 and exon 25 (in particular as defined in Fig. 9).

Moreover, two inventive FISH probes having fluorescent tags in different colors can be used together, in particular to distinguish between the two different truncated forms of Notch2 in cancer (in particular CLL), wherein one form (expressed from the START1 codon, i.e. the NC^{™} truncation) is still tethered to the cell membrane and thus highly likely to be GSI-sensitive and the other form (expressed from the START2 codon i.e. the NC^{IC} truncation) is not tethered to the cell membrane and thus highly likely to be GSI-insensitive. This way, it can be decided whether a treatment of the patient with GSIs is likely to be beneficial or not. To this end, the first inventive FISH probe may bind to a portion of the human NOTCH2 gene region according to SEQ ID NO: 3 (i.e. the region found to be a minimal deleted region in the course of the present invention), especially a portion of exon 24 or 25 (in particular as defined in Fig. 9), and the second FISH probe may bind to a portion of the human NOTCH2 gene region according to SEQ ID NO: 6, especially a portion of exon 26, 27 or 28. On a human chromosome with a wild-type NOTCH2, typically the first and the second inventive FISH probe co-locate (as the region designated as minimal deleted region where the first probe binds, and the region between START1 and START2 where the second probe binds are quite close together (in relation to the entire chromosome), see Fig. 9), thereby yielding a mixed color spot on the chromosome (e.g. visible in the fluorescence microscope). On a human chromosome with a deletion leading to the NC^{™} truncation, typically only the second inventive FISH probe binds (between START1 and START2, see Fig. 9), thereby yielding a color spot with the color of the second FISH probe. On a human chromosome with a deletion leading to the NC^{IC} truncation, typically neither the first nor the second inventive FISH probe binds, thereby not yielding a color spot. As a diploid human cancer cell with a NOTCH2 deletion described herein is typically heterozygous for the deletion, a mixed color spot (i.e. indicating a chromosome with a wild-type NOTCH2; serving as an internal control) and either another spot with the color of the second probe (this typically indicates sensitivity of the cancer to GSI treatment) or no further spot (this typically indicates resistance of the cancer to GSI treatment) can be observed in the cancer cell upon labelling with the first and the second FISH probe. By contrast, two mixed color spots (one for each NOTCH2 locus) can be expected to appear in a diploid human cell not having a NOTCH2 deletion described herein (e.g. non-cancer cells) upon labelling with the first and the second FISH probe. As a further control, a FISH probe such as known in the art (beneficially with a third color) binding e.g. to the entire NOTCH2 gene (or e.g. at least 20%, at least 30% or at least 50% thereof) or to a segment not encompassing SEQ ID NO: 1 (e.g. of the region coding for the intracellular domain) may be added together with (or after) the first and the second inventive FISH probes, to further corroborate the negative signal described above in the NC^{IC} truncation case. See also Example 9.

As described above, it has been interestingly found in the course of the present invention that CLL patients have deletions at specific hotspots in Notch2 (see Fig. 4 and 8). Accordingly, truncated forms of Notch2 receptors were found to be expressed, with the translation starting from alternative START codons (i.e. START1 and START2) that follow the KOZAK rule (AUGG) (Fig. 4). Since START2 is downstream of the γ-secretase cleavage site S3, the N2^{IC} truncation, present in CLL, is resistant to treatment with gamma-secretase inhibitors such as DAPT or RO4929097 (Lauring et al., 2000). Screening of 10 randomly chosen CLL cases for the expression of both truncated Notch2 proteins confirmed the presence of either the N2^{IC} truncation alone, or both truncated forms in CLL cells (Fig. 7C). Accordingly, a probe as described above can be used for determining whether a cell has the above explained mutated regions and thus whether the patient has CLL and which treatment should be chosen. Gliotoxin, a secondary fungus metabolite which is formed by *Aspergillus fumigatus, A. flavus, A. niger and A. terreus,* has been shown to be a Notch2 transactivation inhibitor that is also suitable for truncated, ligand-independent variants (WO 2006/135949 A2).

In the context of the present invention, the cell (e.g. obtained from a human patient or individual, in particular through tumor biopsy or by obtaining a blood sample, in particular PBMCs) is preferably a cancer cell or a cell suspected of being a cancer cell. The cell is preferably mammalian, especially human.

In the context of the present invention, the cancer (to be treated, diagnosed or genotyped)may e.g. be selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL). In a more preferred embodiment, the cancer is CLL.

In the context of the present invention, any compound disclosed in WO 2006/135949 A2 as a NOTCH2 inhibitor can be used as a NOTCH2 signalling pathway inhibitor for the present invention, especially a compound disclosed in the claims of WO 2006/135949 A2, in particular a NOTCH2 signalling pathway inhibitor selected from the group of gliotoxin, 6-C₁₋₃-alkoxygliotoxin, 6-_{C2-3}-acyloxy-gliotoxin, 6-dihydro-gliotoxin, 6-dihydroxy-gliotoxin, 6-[(methoxycarbonyl)methoxy]-gliotoxyin, 6-cyanomethoxy-gliotoxin and pharmaceutically acceptable salts thereof.

In a preference, the inventive use of the polynucleotide probe or polynucleotide probe composition (of the container) as defined herein for determining whether a cell has a human NOTCH2 gene with a deletion as defined herein is for designating the cell as having a gain-of-function NOTCH2 mutation caused by the deletion, or for determining whether the patient from whom the cell was obtained is eligible for a therapy that targets NOTCH2, in particular a gliotoxin-based therapy. Preferably, the patient is eligible when the cell has a human NOTCH2 gene with a deletion starting at a locus in or downstream of exon 20 and ending at a locus upstream of the alternative start codon START2 (further identified e.g. by SEQ ID NO: 1 or SEQ ID NO: 2, respectively; or by NCBI reference sequence NG_008163.1, position 136644-152299 or position 136798-152299, respectively). In particular, said deletion encompasses at least a portion of the genomic region starting with the first nucleotide of exon 24 and ending with the last nucleotide upstream of exon 26 (the region alternatively according to SEQ ID NO: 3; or according to NCBI reference sequence NG_008163.1, position 148043-149349). It is especially preferred that the probe is a FISH probe and/or the inventive use comprises FISH.

In a further, especially preferred embodiment, the inventive use of the polynucleotide probe or polynucleotide probe composition (of the container) as defined herein for determining whether a cell has a human NOTCH2 gene with a deletion as defined herein is for determining whether the cancer cell is gamma-secretase-inhibitor sensitive. Preferably, cell is designated as GSI-sensitive if it has a human NOTCH2 gene with a deletion starting at a locus in or downstream of exon 20 and ending at a locus upstream of the alternative start codon START1 (further identified e.g. by SEQ ID NO: 7 or SEQ ID NO: 85, respectively; or by NCBI reference sequence NG_008163.1, position 136644-150919 or position 136798-150919, respectively). In particular, said deletion encompasses at least a portion of the genomic region starting with the first nucleotide of exon 24 and ending with the last nucleotide upstream of exon 26 (the region alternatively according to SEQ ID NO: 3; or according to NCBI reference sequence NG_008163.1, position 148043-149349). It is especially preferred that the probe is a FISH probe and/or the inventive use comprises FISH.

In a further preferred embodiment of the present invention, the inventive method for detecting the presence or absence of a region of a human NOTCH2 gene on a chromosome of a cell is for determining whether the patient the cell was obtained from is eligible for a therapy that targets NOTCH2, in particular a gliotoxin-based therapy. The cell is preferably mammalian, especially human.

In another preferred embodiment of the present invention, the probe used in the inventive method for detecting the presence or absence of a region of a human NOTCH2 gene on a chromosome of a cell is the polynucleotide probe of the present invention, especially wherein the method is for determining whether the cancer cell is gamma-secretase inhibitor sensitive. The probe used in the inventive method for detecting the presence or absence of a region of a human NOTCH2 gene on a chromosome of a cell may be bound to a molecular tag, especially to a fluorescent tag, and the detecting step may comprise the detection of the molecular tag.

In a further preferred embodiment of this method or the inventive use described above, the probe, being bound to the region of the chromosome, is detected *in situ,* preferably by a microscope, especially a fluorescence microscope. Alternative or additional detection options include a photometer, a fluorometer or a scintillation counter. In another preferred embodiment, the method is for detection of minimal residual disease (tumor stem cells).

In a further preferred embodiment, this method (or the inventive use described above) is for quantification of the region of a human NOTCH2 gene present in a population of cells. The method (or the inventive use described above) may also be for evaluation of response to cancer therapy, especially CLL therapy. The method (or the inventive use described above) may also be used for distinguishing between premalignant cells and transformed cells (e.g. in a cancer patient, especially a CLL patient). The method (or the inventive use described above) may also be for predicting the response to GSI therapy.

Typically, the patient (who preferably has a cancer as defined herein and especially has been diagnosed with a cancer as defined herein) is in need of the inventive treatment. In the context of the inventive treatment for prevention of a cancer, the patient may be a patient that does not have the cancer but is at risk of developing the cancer. Typically, an effective amount of the Notch2 signalling pathway inhibitor, the GSI or the antibody of the present invention is administered to the patient. The preferred route of administration of said Notch2 signalling pathway inhibitor or said GSI is oral administration The preferred route of administration of said antibody is parenteral administration, especially through intravenous, intraarterial, intramuscular, intrathecal, subcutaneous or intraperitoneal administration.

GSIs can also be used in the inventive treatment of cancer (or prevention of cancer relapse), e.g. with the cancer being CLL, if the truncated version of Notch2 has an extracellular and transmembrane domain including the S3 cleavage site, i.e. is expressed from the codon START1. Common GSIs are DAPT or RO4929097. In the context of the present invention, any GSI selected from Table I of Olsauskas-Kuprys et al. (2013) or a pharmaceutically acceptable salt thereof is a preferred GSI, in particular DAPT or RO4929097.

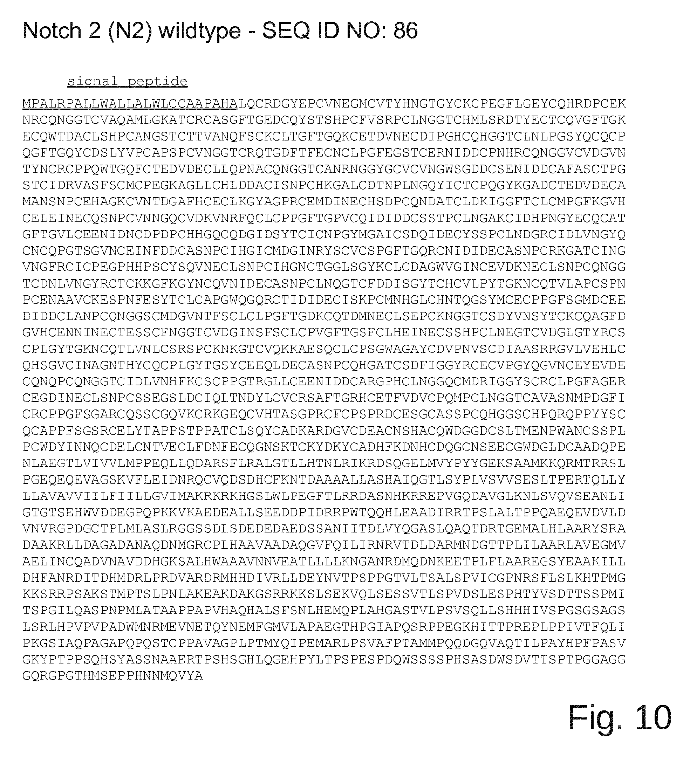

In a further aspect, the present invention relates to an antibody specific for an N-terminally truncated human Notch2 protein starting with the sequence MVYP (SEQ ID NO: 4) (i.e. translated at the START1 codon described herein) or the sequence MAKR (SEQ ID NO: 5) (i.e. translated at the START2 codon described herein). The inventive antibody can be used in diagnosis, prognosis and targeted treatment of cancers such as CLL. Preferably, this antibody does essentially not bind or only weakly binds to the wildtype (i.e. non-truncated) human Notch2. In particular the binding to the wild-type human Notch2 (preferably as defined in SEQ ID NO: 86, optionally without the signal peptide as defined in Fig. 10) is weaker than to said N-terminally truncated human Notch2 protein, preferably at least 10x weaker, more preferably at least 100x weaker, yet more preferably at least 1000x weaker, even more preferably at least 10000x weaker, yet even more preferably at least 100000x weaker or even at least 1000000x weaker. Especially the antibody specific for an N-terminally truncated human Notch2 protein starting with the sequence MVYP (SEQ ID NO: 4) is for use in therapy (in particular cancer therapy, e.g. of CLL). Preferably, the antibody of the present invention is isolated.

Preferably, the antibody of the present invention is monoclonal. Both expression and purification of monoclonal antibodies is well-known in the art. See for instance Birch & Racher, 2006, for an overview of large-scale antibody production (expression and purification). Techniques also comprise conventional monoclonal antibody methodology, for example the standard somatic cell hybridization technique of Köhler and Milstein. Other techniques for producing monoclonal antibodies can also be employed such as viral or oncogenic transformation of B lymphocytes.

Furthermore, the antibody of the present invention is preferably humanised. Methods to obtain humanised antibodies are well known in the art. One method is to insert the variable regions of the antibody into a human antibody scaffold. See e.g. Almagro & Fransson, 2008, for an overview of humanisation techniques. For facilitated humanisation, monoclonal antibodies can also be raised in genetically modified mice, such as the VELOCIMMUNE® mouse; see e.g. the US patents no. 6,596,541 and US 7,105,348.

In a preferred embodiment, the antibody is specific for an N-terminal epitope of a peptide, the peptide consisting of the sequence MVYPYYGEKSAAMKKQRMTR (SEQ ID NO: 8).

In a further preferred embodiment of the present invention, the antibody is specific for an epitope selected from MVYP (SEQ ID NO: 4), MVYPY (SEQ ID NO: 9), MVYPYY (SEQ ID NO: 10), MVYPYYG (SEQ ID NO: 11), MVYPYYGE (SEQ ID NO: 12), MVYPYYGEK (SEQ ID NO: 13), MVYPYYGEKS (SEQ ID NO: 14), MVYPYYGEKSA (SEQ ID NO: 15), MVYPYYGEKSAA (SEQ ID NO: 16), MVYPYYGEKSAAM (SEQ ID NO: 17), MVYPYYGEKSAAMK (SEQ ID NO: 18), MVYPYYGEKSAAMKK (SEQ ID NO: 19), MVYPYYGEKSAAMKKQ (SEQ ID NO: 20), MVYPYYGEKSAAMKKQR (SEQ ID NO: 21), MVYPYYGEKSAAMKKQRM (SEQ ID NO: 22), MVYPYYGEKSAAMKKQRMT (SEQ ID NO: 23) and MVYPYYGEKSAAMKKQRMTR (SEQ ID NO: 8). It is essential that the epitope comprises a free N-terminus, such that the antibody does essentially not recognize the corresponding internal epitope in wild-type Notch2 or at least has a weaker affinity to wild-type Notch2 than to the truncation.

In a further preferred embodiment, the antibody is specific for an N-terminal epitope of a peptide consisting of the sequence MAKRKRKHGSLWLPEGFTLR (SEQ ID NO: 24).

In a further preferred embodiment of the present invention, the antibody is specific for an epitope selected from MAKR (SEQ ID NO: 5), MAKRK (SEQ ID NO: 25), MAKRKR (SEQ ID NO: 26), MAKRKRK (SEQ ID NO: 27), MAKRKRKH (SEQ ID NO: 28), MAKRKRKHG (SEQ ID NO: 29), MAKRKRKHGS (SEQ ID NO: 30), MAKRKRKHGSL (SEQ ID NO: 31), MAKRKRKHGSLW (SEQ ID NO: 32), MAKRKRKHGSLWL (SEQ ID NO: 33), MAKRKRKHGSLWLP (SEQ ID NO: 34), MAKRKRKHGSLWLPE (SEQ ID NO: 35), MAKRKRKHGSLWLPEG (SEQ ID NO: 36), MAKRKRKHGSLWLPEGF (SEQ ID NO: 37), MAKRKRKHGSLWLPEGFT (SEQ ID NO: 38), MAKRKRKHGSLWLPEGFTL (SEQ ID NO: 39), and MAKRKRKHGSLWLPEGFTLR (SEQ ID NO: 24). It is essential that the epitope comprises a free N-terminus, such that the antibody does not recognize the corresponding internal epitope in wild-type Notch2 or at least has a weaker affinity to wild-type Notch2 than to the truncation.

The antibody of the present invention may be analysed for specificity in an ELISA-based primary screening. The antibody can further be classified by their dissociation constant K_{d} in regard to the respective epitope (which may also be called "affinity") and by their off-rate value in regard to the respective epitope. Both terms (and how they are measured) are well-known in the art. If not taking other parameters into account, the higher the affinity (i.e. the lower the K_{d} is) and/or the lower the off-rate value of the antibody is, the more suitable it is.

Hence, in another preferable embodiment of the invention, the dissociation constant K_{d} of the antibody in regard to the respective epitope is lower than 50 nM, preferably lower than 20 nM, more preferably lower than 10 nM, even more preferably lower than 5 nM, most preferably lower than 2 nM. Moreover, in another preferable embodiment of the invention, the off-rate value of the antibody in regard to the respective epitope is lower than 5x10⁻³s⁻¹, preferably lower than 3x10⁻³s⁻¹, more preferably lower than 1x10⁻³s⁻¹, even more preferably lower than 1x10⁻⁴s⁻¹.

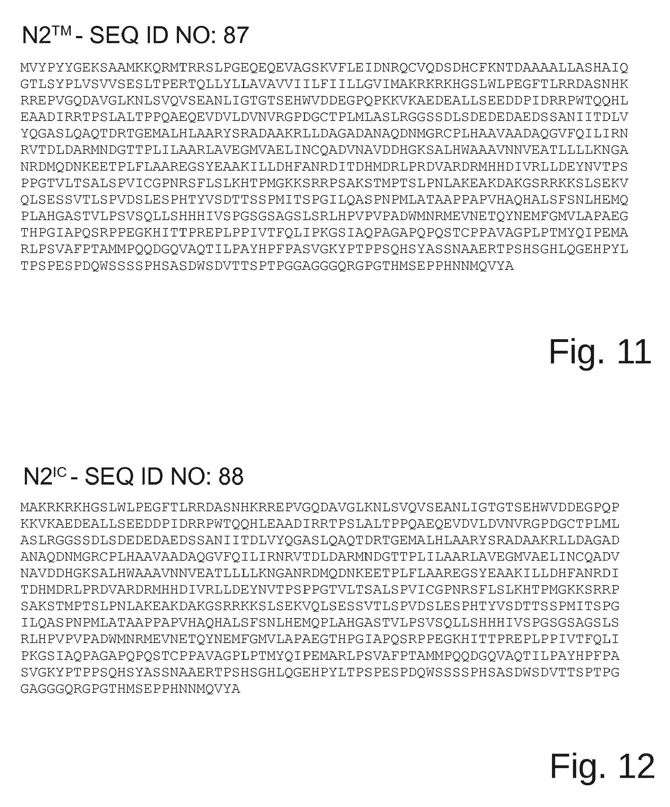

In another preferable embodiment of the invention, the antibody has an affinity (e.g. expressed as K_{d}) to wild-type human Notch2 (preferably as defined in SEQ ID NO: 86, optionally without the signal peptide as defined in Fig. 10) that is weaker (i.e. lower) than the antibody's affinity (e.g. expressed as K_{d}) to an N-terminally truncated human Notch2 protein as defined in SEQ ID NO: 87 (or Fig. 11) or SEQ ID NO: 88 (or Fig. 12), preferably at least 10x weaker, more preferably at least 100x weaker, yet more preferably at least 1000x weaker, even more preferably at least 10000x weaker, yet even more preferably at least 100000x weaker or even at least 1000000x weaker. Preferably, the affinity to the respective Notch2 variant is measured by ELISA on a plate coated by wild-type human Notch2 (SEQ ID NO: 86), N-terminally truncated human Notch2 (SEQ ID NO: 87 or Fig. 11) or N-terminally truncated human Notch2 (SEQ ID NO: 88 or Fig. 12), respectively; especially after blocking with 1% BSA comprising the following conditions: concentration of the antibody: 0.25µg/ml, concentration of the secondary, HRP-coupled antibody: 0.1µg/ml, HRP substrate: ABTS and 0.1% hydrogen peroxide, read-out: absorbance at 405nm.

In a further preferred embodiment, the antibody is labelled by a molecular tag such as a fluorescent tag and can be used e.g. in fluorescence microscopy or FACS, in particular to find or purify cells with the truncated Notch2 variants described herein.

In a further aspect, the antibody of the present invention is for use in the treatment of cancer or prevention of cancer relapse. Preferably, the cancer is selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL).

A still further aspect relates to the use of the inventive antibody in diagnostics, especially in cancer diagnosis or prognosis (in particular CLL). Preferably, this use is in-vitro. The prognosis involves predicting disease-free survival, event-free survival, puncture-free survival and/or overall survival of the patient. Disease-free survival is defined as the length of time after primary treatment for a cancer ends that the patient survives without any signs or symptoms of that cancer, or time to death, which ever occurred first. Event-free survival is defined as length of time after primary treatment for a cancer ends that the patient remains free of certain complications or events that the treatment was intended to prevent or delay, or time to death, which ever occurred first. These events may include the return of the cancer or the onset of certain symptoms, such as bone pain from cancer that has spread to the bone. Puncture-free survival is defined as time to first need of paracentesis after primary treatment, or time to death, which ever occurred first. Overall survival is defined as time to death after primary treatment has ended.

In a preferred embodiment, the inventive antibody is contacted with PBMCs obtained from a patient having cancer, especially CLL, or suspected of having cancer, especially CLL.

"NOTCH2 signalling pathway inhibitors" as used herein refer to compounds or agents that inhibit or reduce the biological activity of the molecule to which they bind, wherein the molecule has an activating activity in the NOTCH2 signalling pathway. Notch2 signalling pathway inhibitors include, e.g., GSIs, Notch regulator peptides and proteins, and anti-Notch antibodies or fragments thereof that bind to Notch2, which may be optionally conjugated with another molecule to form an antibody-drug conjugate(ADC).

The terms "γ-secretase inhibitor," "gamma secretase inhibitor" and "GSI" are used interchangeably herein to refer to refer to compounds (including pharmaceutically acceptable salts, solvates, and prodrugs thereof) or other agents that inhibit or reduce the biological activity of γ-secretase. Exemplary small molecule GSIs include, e.g., the dipeptide class of GSIs, the sulfonamide class of GSIs, the transition state mimic class of GSIs, the benzocaprolactam class of GSIs, and other GSIs known in the art.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-, yeast- or mammalian cell-display methods, and methods utilizing transgenic animals containing all or part of the human immunoglobulin loci.

The term "preventing" or "prevention" as used herein means to stop a disease state or condition from occurring in a patient or subject completely or almost completely or at least to a (preferably significant) extent, especially when the patient or subject is predisposed to such a risk of contracting a disease state or condition.

Herein, the term "specific for" - as in "molecule A specific for molecule B" - means that molecule A has a binding preference for molecule B compared to other molecules in a patient's body (or in a sample thereof). Typically, this entails that molecule A (such as an antibody) has a dissociation constant (may also be called "affinity") in regard to molecule B (such as the antigen, specifically the binding epitope thereof) that is lower than (i.e. "stronger than") 1000 nM, preferably lower than 100 nM, more preferably lower than 50 nM, even more preferably lower than 10 nM, especially lower than 5 nM.

As used herein, "START1" or "the alternative start codon START1" refer to an alternative start codon in the human NOTCH2 gene according to NCBI reference sequence NG_008163.1, positions 150920-150922, or to the start codon labelled START1 as depicted in Fig. 9. As used herein, "START2" or "the alternative start codon START2" refer to an alternative start codon in the human NOTCH2 gene according to NCBI reference sequence NG_008163.1, positions 152300-152302, or to the start codon labelled START2 as depicted in Fig. 9.

The following embodiments further define the present invention:
Embodiment 1. A polynucleotide probe for detecting a region of the human NOTCH2 gene in a cell, the sequence of the region being at least 10 consecutive nucleotides selected from the sequence according to SEQ ID NO: 1, wherein the probe is specific for the region or for its reverse complement, and wherein the probe is bound to a molecular tag; preferably, the sequence of the region is at least 11, 12, 13, 14, 15, 16, 17, 18, 19, 20, 21, 22, 23, 24, 25, 30, 40 or 50 consecutive nucleotides selected from the sequence according to any one of SEQ ID NOs: 1, 2, 3, 6, 7 and 85.
Embodiment 2. The probe of embodiment 1, wherein the sequence of the region is at least 11, preferably at least 15, more preferably at least 16, yet more preferably at least 17, even more preferably at least 18, yet even more preferably at least 19 or even at least 20 consecutive nucleotides selected from the sequence according to SEQ ID NO: 1.
Embodiment 3. The probe of embodiment 1 or 2, wherein the sequence of the region is at least 10, preferably at least 15 consecutive nucleotides selected from the sequence according to SEQ ID NO: 3.
Embodiment 4. The probe of embodiment 1-3, wherein the sequence of the region is at least 10, preferably at least 15 consecutive nucleotides selected from the sequence according to SEQ ID NO: 6.
Embodiment 5. The probe of any one of embodiments 1-4, wherein the sequence of the region is at least 20 consecutive nucleotides selected from the sequence according to SEQ ID NO: 1, preferably from the sequence according to SEQ ID NO: 3 or from the sequence according to SEQ ID NO: 6.
Embodiment 6. A polynucleotide probe, wherein at least 75%, preferably at least 80%, more preferably at least 85%, even more preferably at least 90%, especially at least 95% or even 100% of the sequence of the probe consist of at at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 1 or its reverse complement, wherein the probe is bound to a molecular tag.
Embodiment 7. A polynucleotide probe, wherein the sequence of the probe consists of at least 10, preferably at least 12, more preferably at least 14, yet more preferably at least 15, even more preferably at least 16, yet even more preferably at least 17 or even at least 18, in particular at least 19 or even at least 20 consecutive nucleotides selected from the sequence according to SEQ ID NO: 1 or its reverse complement, wherein the probe is bound to a molecular tag.
Embodiment 8. The probe of embodiments 6 or 7, wherein said consecutive nucleotides are selected from the sequence according to SEQ ID NO: 3 or its reverse complement.
Embodiment 9. The probe of embodiments 6 or 7, wherein said consecutive nucleotides are selected from the sequence according to SEQ ID NO: 6 or its reverse complement.
Embodiment 10. The probe of any one of embodiments 1-9, wherein the molecular tag is a fluorescent tag.
Embodiment 11. A container such as a vial, comprising a polynucleotide probe composition especially for fluorescence in-situ hybridisation (FISH), wherein the composition comprises at least one probe, preferably at least two different probes, as described in any one of embodiments 1-10 and preferably a buffer in which the probes are dissolved; in particular wherein the composition is for determining whether a cell has a human NOTCH2 gene with a deletion starting at a locus in or downstream of exon 20 and ending at a locus upstream of the alternative start codon START2, preferably wherein the deletion encompasses at least a portion of SEQ ID NO: 3.
Embodiment 12. Use of the probe or composition of any one of embodiments 1 to 11 for determining whether a cell has a human NOTCH2 gene with a deletion starting at a locus in or downstream of exon 20 and ending at a locus upstream of the alternative start codon START2, in particular wherein the deletion encompasses at least a portion of SEQ ID NO: 3, preferably wherein the cell is a cancer cell or a cell suspected of being a cancer cell, especially for designating the cell as having a gain-of-function NOTCH2 mutation caused by the deletion, or for determining whether the patient from whom the cell was obtained is eligible for a therapy that targets NOTCH2, in particular a gliotoxin-based therapy. Embodiment 13. The use of embodiment 12, wherein said cancer is selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL).
Embodiment 14. The use of embodiment 13, wherein said cancer is CLL.
Embodiment 15. The use of any one of embodiments 12 to 14, for determining whether the cancer cell is gamma-secretase-inhibitor sensitive, wherein said probe is, or said composition comprises, the probe as set forth in embodiments 4 or 9.
Embodiment 16. The use of any one of embodiments 12 to 15, comprising the use of FISH for the detecting, wherein the probe is a FISH probe.
Embodiment 17. A method for detecting the presence or absence of a region of a human NOTCH2 gene on a chromosome of a cell, the sequence of the region being at least 10, preferably at least 11, more preferably at least 12, even more preferably at least 13, yet even more preferably at least 14, or even at least 15 consecutive nucleotides selected from the sequence according to SEQ ID NO: 1, the method comprising
   - providing the cell,
   - contacting a probe specific for the region or for its reverse complement with the chromosome or a fragment thereof
   - detecting whether the probe is bound to the chromosome or fragment thereof; preferably for determining whether the NOTCH2 gene has a deletion starting at a locus in or downstream of exon 20 and ending at a locus upstream of the alternative start codon START2, wherein the deletion comprises said region;
   preferably wherein the probe is bound to a molecular tag such as a fluorescent tag.
Embodiment 18. The method of embodiment 17 wherein the cell is a cancer cell or a cell suspected of being a cancer cell, in particular wherein said cancer is selected from leukemia, preferably CLL, melanoma, pancreas carcinoma, hepatocellular carcinoma, medulloblastoma, glioblastoma and lymphoma, preferably marginal zone lymphoma or mantle cell lymphoma (MCL); preferably wherein the method is for determining whether the patient the cell was obtained from is eligible for a therapy that targets NOTCH2, in particular a gliotoxin-based therapy.
Embodiment 19. The method of embodiment 17 or 18, wherein said probe is the probe of any one of embodiments 1 to 10.
Embodiment 20. The method of embodiment 18, wherein said probe is the probe as set forth in embodiment 4 or 9, especially wherein the method is for determining whether the cancer cell is gamma-secretase-inhibitor sensitive.
Embodiment 21. The method of any one of embodiments 17 to 20, wherein the probe is bound to a molecular tag, especially to a fluorescent tag, and the detecting step comprises the detection of the molecular tag.
Embodiment 22. The method of any one of embodiments 17 to 21, wherein the probe, being bound to the region of the chromosome, is detected in situ, preferably by a microscope, especially a fluorescence microscope.
Embodiment 23. A NOTCH2 signalling pathway inhibitor, in particular a gliotoxin, for use in treatment of cancer or prevention of cancer relapse in a patient, wherein at least a portion of the cells of the cancer have a human NOTCH2 gene with a deletion starting at a locus in or downstream of exon 20 and ending at a locus upstream of the alternative start codon START2 .
Embodiment 24. A gamma-secretase inhibitor for use in treatment of cancer or prevention of cancer relapse in a patient, wherein at least a portion of the cells of the cancer have a human NOTCH2 gene with a deletion starting at a locus in or downstream of exon 20 and ending at a locus upstream of the alternative start codon START1.
Embodiment 25. The inhibitor for use of embodiment 23 or 24, wherein the deletion encompasses at least a portion ofSEQ ID NO: 3.
Embodiment 26. The inhibitor for use of any one of embodiments 23 to 25, wherein the cancer is selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL).
Embodiment 27. An antibody specific for an N-terminally truncated human Notch2 protein, the N-terminally truncated human Notch2 protein starting with the sequence MVYP (SEQ ID NO: 4).
Embodiment 28. The antibody of embodiment 27, wherein the antibody is specific for an N-terminal epitope of a peptide consisting of the sequence MVYPYYGEKSAAMKKQRMTR (SEQ ID NO: 8).
Embodiment 29. The antibody of embodiment 27, wherein the antibody is specific for an epitope selected from MVYP (SEQ ID NO: 4), MVYPY (SEQ ID NO: 9), MVYPYY (SEQ ID NO: 10), MVYPYYG (SEQ ID NO: 11), MVYPYYGE (SEQ ID NO: 12), MVYPYYGEK (SEQ ID NO: 13), MVYPYYGEKS (SEQ ID NO: 14), MVYPYYGEKSA (SEQ ID NO: 15), MVYPYYGEKSAA (SEQ ID NO: 16), MVYPYYGEKSAAM (SEQ ID NO: 17), MVYPYYGEKSAAMK (SEQ ID NO: 18), MVYPYYGEKSAAMKK (SEQ ID NO: 19), MVYPYYGEKSAAMKKQ (SEQ ID NO: 20), MVYPYYGEKSAAMKKQR (SEQ ID NO: 21), MVYPYYGEKSAAMKKQRM (SEQ ID NO: 22), MVYPYYGEKSAAMKKQRMT (SEQ ID NO: 23) and MVYPYYGEKSAAMKKQRMTR (SEQ ID NO: 8), wherein the epitope has a free N-terminus.
Embodiment 30. An antibody specific for an N-terminally truncated human Notch2 protein, the N-terminally truncated human Notch2 protein starting with the sequence MAKR (SEQ ID NO: 5).
Embodiment 31. The antibody of embodiment 30, wherein the antibody is specific for an N-terminal epitope of a peptide consisting of the sequence MAKRKRKHGSLWLPEGFTLR (SEQ ID NO: 24).
Embodiment 32. The antibody of embodiment 30, wherein the antibody is specific for an epitope selected from MAKR (SEQ ID NO: 5), MAKRK (SEQ ID NO: 25), MAKRKR (SEQ ID NO: 26), MAKRKRK (SEQ ID NO: 27), MAKRKRKH (SEQ ID NO: 28), MAKRKRKHG (SEQ ID NO: 29), MAKRKRKHGS (SEQ ID NO: 30), MAKRKRKHGSL (SEQ ID NO: 31), MAKRKRKHGSLW (SEQ ID NO: 32), MAKRKRKHGSLWL (SEQ ID NO: 33), MAKRKRKHGSLWLP (SEQ ID NO: 34), MAKRKRKHGSLWLPE (SEQ ID NO: 35), MAKRKRKHGSLWLPEG (SEQ ID NO: 36), MAKRKRKHGSLWLPEGF (SEQ ID NO: 37), MAKRKRKHGSLWLPEGFT (SEQ ID NO: 38), MAKRKRKHGSLWLPEGFTL (SEQ ID NO: 39), and MAKRKRKHGSLWLPEGFTLR (SEQ ID NO: 24), wherein the epitope has a free N-terminus.
Embodiment 33. The antibody of any one of embodiments 27-32, wherein the antibody is monoclonal and preferably humanized or human.
Embodiment 34. The antibody of any one of embodiments 27-33, for use in therapy or diagnostics.
Embodiment 35. The antibody of any one of embodiments 27-31, for use in the treatment of cancer or prevention of cancer relapse.
Embodiment 36. The antibody for use of embodiment 35, wherein the cancer is selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL).
Embodiment 37. Use of the antibody of any one of embodiments 27-33, in cancer diagnosis or prognosis.
Embodiment 38. The use of embodiment 37, wherein the cancer is selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL).

The present invention is further illustrated by the following figures and examples, without being restricted thereto.

### Figures

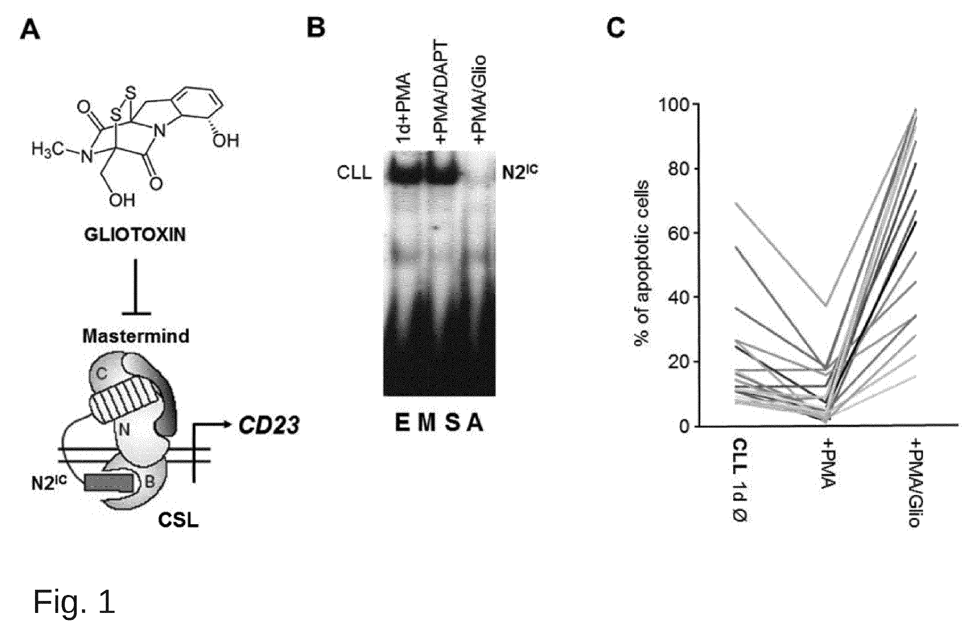

**Fig. 1****:** Effectiveness of Notch2 inhibitor Gliotoxin.
   (A and B) 0.2µM Gliotoxin interferes with the assembly of the ternary NOTCH2 (N2^{IC} / Mastermind / CSL) transcription factor complex on DNA. In contrast, 5µM DAPT had no effect on the nuclear NOTCH2 activity in the majority of CLL cases. (C) Gliotoxin reversed the protective effect of PMA on CLL cells and could also overcome the anti-apoptotic effect of bone marrow stromal cells (Hubmann et al., 2013) as evaluated in a microenvironment model (Shehata et al., 2004; Shehata et al., 2010; Shehata et al. , 2010b).

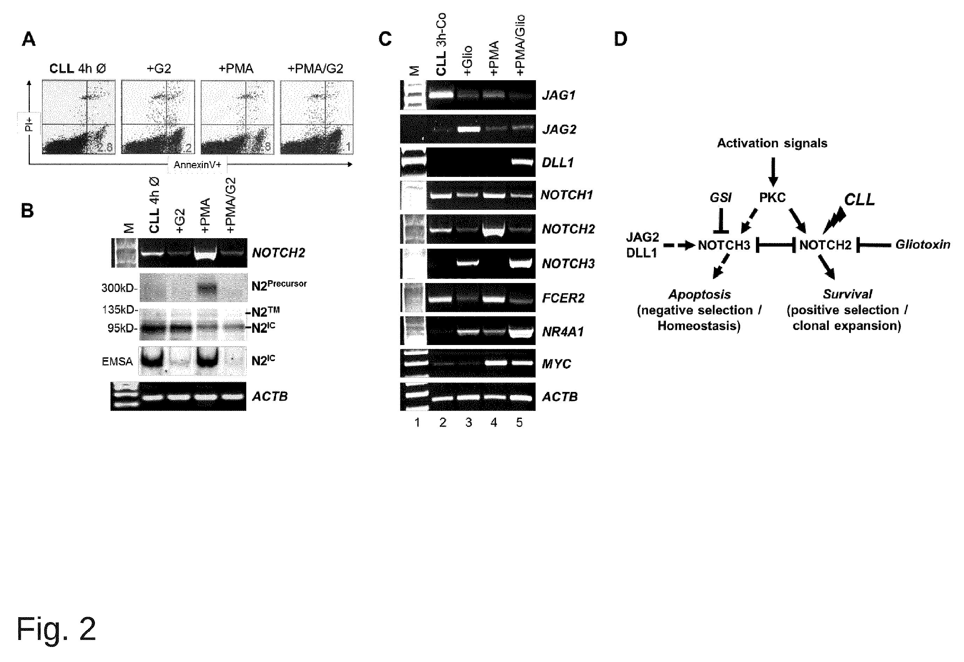

**Fig. 2****:** Gliotoxin inhibits NOTCH2 at the transcription factor level in unstimulated and in PMA-activated CLL cells and can be expected to trigger NR4A1 induced apoptosis by a mechanism involving the induction of NOTCH3 signalling.
   (A) FACS analysis showing the percentage of early apoptotic CLL cells (AnnexinV+) after 3h treatment with 0.2µM gliotoxin ±PMA. (B) Corresponding western blot and EMSA analysis indicating the effect of gliotoxin on DNA-bound N2^{IC} complexes in CLL cells. (C) Corresponding RT-PCR analysis showing the effect of gliotoxin on the transcription of NOTCH and apoptosis related genes. PMA induces homotypic cell aggregation and upregulates *MYC* (Carlsson et al., 19), resembling the situation found in CLL proliferation centres (Herishanu et al., 2011). (D) NOTCH2 can be expected to regulate the binary cell fate decision between positive and negative selection of activated CD5+ (B-1a) B-cells (Hubmann et al., 2010; Limnander et al., 2014) by inhibiting a postulated pro-apoptotic NOTCH3-NR4A1 axis. In CLL cells, constitutively activated NOTCH2 can be expected to facilitate the antigen driven aberrant positive selection/clonal expansion of the malignant clone and, thus, fulfils the criteria of a CLL initiating oncogene. GSIs can be expected to selectively target tumour suppressive NOTCH3 signalling in CLL cases expressing a GSI resistant form of NOTCH2 limiting their therapeutic value for CLL patients.

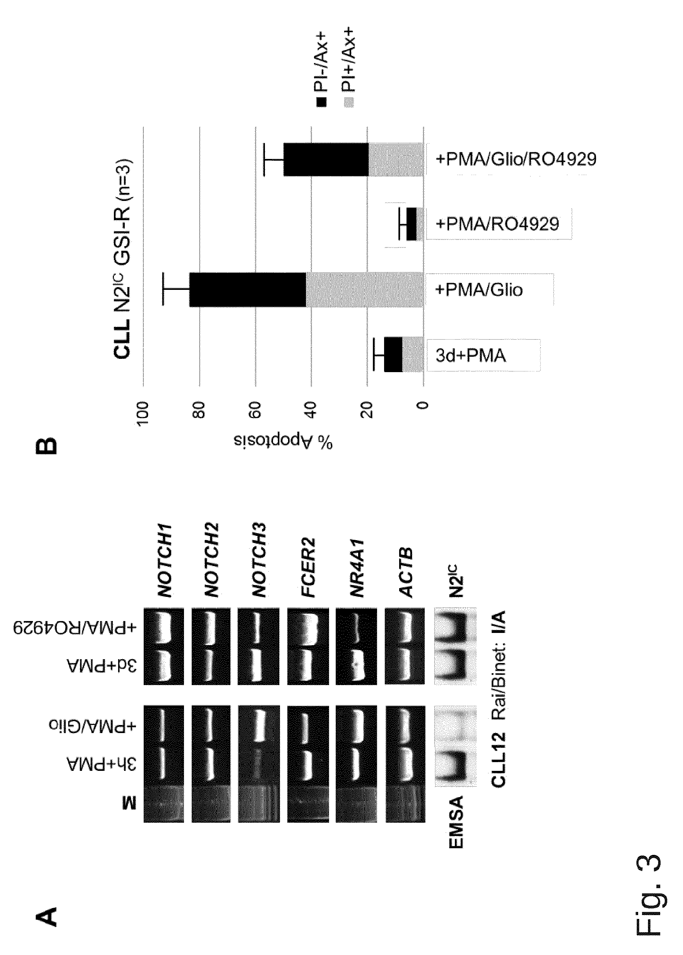

**Fig. 3****:** RO4929097 counteracts spontaneous and gliotoxin mediated apoptosis in PMA-activated CLL cells expressing a GSI resistant form of nuclear NOTCH2.
   (A) RT-PCR showing the opposite effects of gliotoxin (0.2µM) and RO4929097 (0.5µM) on the transcription of *NOTCH2*/*FCER2* (CD23) and *NOTCH3*/*NR4A1* in PMA-activated CLL cells. The different incubation time points for gliotoxin and RO4929097 were selected due to their different mode of action. (B) Corresponding FACS analysis showing the inhibition of spontaneous and gliotoxin induced apoptosis by RO4929097 in this patient sample.
**Fig. 4****:** Schematic presentation of the N2EC 5'- and 3'-deletion breakpoint (DEL1-8) hotspots in CLL cells
   The downstream *START* codons (i.e. START1 and START2) for the expression of truncated, ligand independent NOTCH2 forms are indicated as well as the S3 (Presenillin/γ-secretase complex) cleavage site. The sequence depicted is the NCBI Reference Sequence NM_024408.3 (positions 2641-5460) of the human NOTCH2 mRNA.

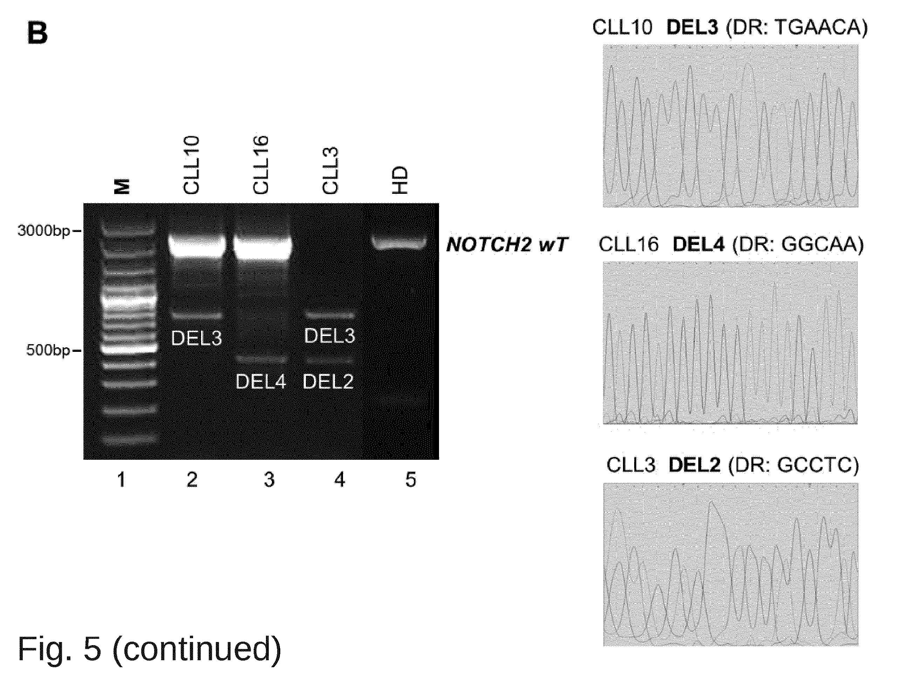

**Fig. 5****:** Identification of N2EC deletions in CLL cells by long range RT-PCR.
   This strategy is based on the assumption that PCR primers flank putative deletions and thus yield a detectable PCR product. (A) PCR primer map covering the whole *NOTCH2* mRNA coding strand (CDS). The relative position of the primers is indicated according to the NOTCH2 reference mRNA sequence with the NCBI accession number NM_024408.3. LNR, Lyn/Notch repeats; HDD, heterodimerization domain; PEST, pest domain. The overlapping sequence of NOTCH2 with NOTCH2NL is indicated. (B) Representative RT-PCR using primer pair N2EC2 showing the monoallelic (CLL10, C1116) or biallelic (CLL3) nature of the dominant N2EC deletions (DEL2, DEL3, and DEL4) in CLL cells. The wild type (WT) NOTCH2 allele is indicated. A representative healthy donor (HD) sample is included as control. The PCR products were gel purified and Sanger sequenced (LGC genomics) as indicated in the respective chromatograms on the right side. The remaining short direct repeat (DR) characterizing the deletion border is indicated.
**Fig. 6****:** Schematic presentation of the N2EC 3'-deletion breakpoint (DEL1-8) hotspots including the 3'-exon skipping (ES1&2) borders in CLL cells and the downstream *START* codons for the expression of truncated, ligand independent NOTCH2 forms.
   The S1 (furin like convertase), S2 (ADAM10/Kuzbanian metalloprotease), and S3 (Presenillin/γ-secretase complex) cleavage sites and the NOTCH2 transmembrane domain on the aligned amino acid sequence are indicated.
   The nucleotide sequence depicted is the NCBI Reference Sequence NM_024408.3 (positions 4798-5397) of the human NOTCH2 mRNA. The protein sequence depicted is the UniProt entry Q04721 (sequence version 3, positions 1588-1700) of the human NOTCH2 protein.

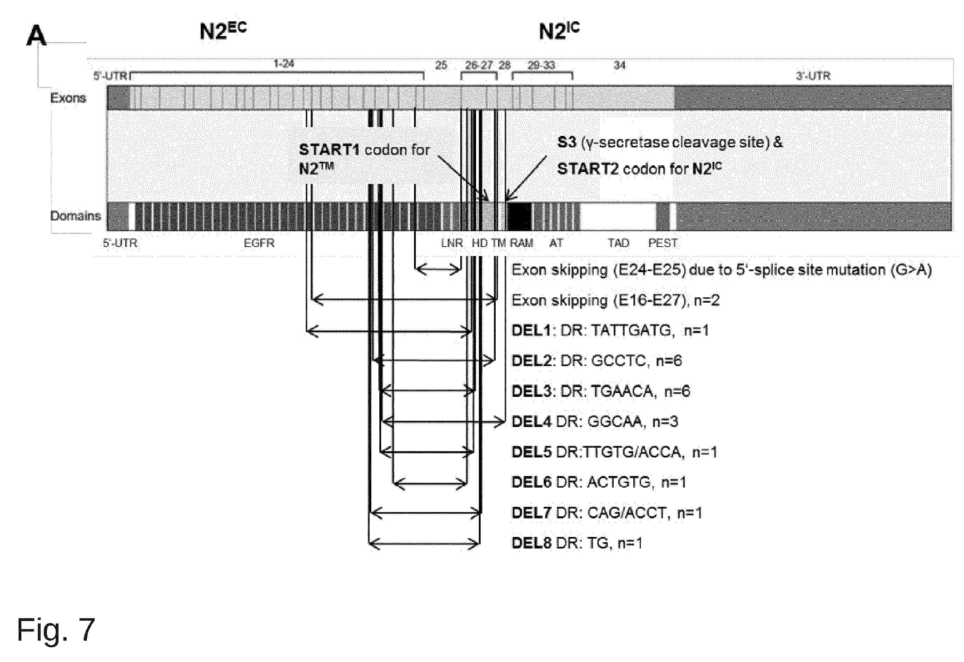

**Fig. 7****:** Notch2 domains.
   (A) *NOTCH2* deletion map for CLL cells (for details see table 1). Schematic presentation of the *NOTCH2* mRNA (34 exons) and the corresponding protein domains (see Fig. 5). The relative position of the deletion breakpoints in the gene region coding for the NOTCH2 extracellular domain (N2^{EC}) and the flanking direct repeats (DR) in the wild type sequence are indicated.
   (B) Schematic presentation of the N2^{™} truncation (with translation starting at *START1*) and the N2^{IC} truncation (with translation starting at *START2)* NOTCH2 forms caused by N2^{EC} deletions in CLL cells. DEL2, 3 and 4 are the dominant deletions found in CLL samples and account for 79% of all NOTCH2 mutations identified so far. DEL3 is found in 32% of CLL cases and can be expected to lead to the expression of a GSI sensitive N2^{™} truncation. DEL2&4 are found in 47% of CLL cases and can be expected to lead to the expression of a GSI sensitive N2^{™} truncation. DEL2&4 are found in 47% of CLL cases and can be expected to lead to the expression of a GSI resistant N2^{IC} truncation. (C) Western blotting showing the N2^{™} and N2^{IC} truncation bands in 10 representative CLL cases. The co-expression of both NOTCH2 protein forms may be explained by the usage of both START codons in parallel, the co-expression of a deleted and a wild type NOTCH2 allele, or the immediate processing of the N2^{™} truncation to the N2^{IC} truncation.
**Fig. 8****:** Clinical and prognostic parameters of CLL patients in relation to N2^{EC} deletions/mutations.
   Abbreviations: U, *IGHV* unmutated; M, *IGHV* mutated; NA, not amplifiable; Del, deleted; ES, exon skipping; SSM, splice site mutation. The relative nucleotide positions of the deletion borders according to the NOTCH2 gDNA (genomic DNA) reference sequences NG_008163.1 and the affected exons are indicated. Two *START* codons following the KOZAK rule could be found (see Fig 6) and the calculated protein sizes of the resulting expected NOTCH2 protein truncations (N2^{™} truncation: ~122 kD, N2^{IC} truncation: ~110 kD) are indicated (see also Fig. 2B).
**Fig. 9****:** Most relevant part of the human Notch2 genomic DNA (gDNA) sequence. Exons are indicated by underlining. Start codons START1 and START2, and the region designated as minimal deleted region are indicated by boldface. ctd.: continued.
**Fig. 10****:** Human Notch 2 wildtype protein sequence (with signal peptide indicated by underlining).
**Fig. 11****:** Sequence of a truncated Notch 2 protein (the N2^{™} truncation) discovered in the course of the present invention.
**Fig. 12****:** Sequence of a truncated Notch 2 protein (the N2^{IC} truncation) discovered in the course of the present invention.

### Examples

### Example 1 - Material and methods for subsequent examples

Sample collection and preparation: Heparinized peripheral blood was obtained from CLL patients after signed informed consent according to the approval of the local ethics committee. Peripheral blood mononuclear cells (PBMC) were isolated using Ficoll-Hypaque (Pharmacia, Uppsala, Sweden) centrifugation. Samples were routinely screened for the IGHV mutational status and for cytogenetic abnormalities.

Cell lines and culture: CLL cells were cultured in RPMI 1640 medium containing 10% fetal calf serum, 100U/ml penicillin and 100mg/ml streptomycin at 37 °C, 7.5% CO₂ in humidified atmosphere. The ex *vivo* CLL microenvironment model based on using primary human bone marrow stromal cells in co-culture with CLL cells was applied as previously described and was used for analysing the effect of gliotoxin, RO4929097, and blocking antibodies on CLL cells in a milieu resembling the situation in vivo (Shehata et al., 2004). The PMA activation model was used to analyse the function of PKC stimulation on the regulation of NOTCH2/NOTCH3 signalling in CLL cells.

Flow cytometry (FACS) and detection of cell viability: Antibodies (CD5, CD19, CD20, CD23, CD49d, CD37, CD52, and 7-Aminoactinomycin D (7-AAD) were purchased from Becton Dickinson (San Jose, CA). Species- and isotype-matched irrelevant antibodies are used as controls. Flow cytometry was performed on a FACScan^{®} using CellQuest Pro software version 5.2.1 (Becton Dickinson, San Jose, CA). Ultra high speed cell sorting was used to isolate CD5+/CD19+ CLL cells from normal B-cells (CD19+/CD5-) and T-cells (CD3+). AnnexinV, propidium iodide, and 7-AAD staining was performed to estimate the percentages of cells undergoing apoptosis. The overall effect on cell viability was calculated as the sum of early apoptotic (AnnexinV single positive; Ax+/PI-) and late apoptotic/necrotic (AnnexinV/propidium iodide double positive; Ax+/PI+) cells using a kit from Bender Med. Systems Inc. (Vienna, AT). Cell viability was evaluated by a nonisotopic MTT (3-(4,5-dimethylthiazolyl-2)-2,5-diphenyltetrazolium bromide) assay (Ez4U) in 96-well culture plates as described by the manufacturer (Biomedica, AT).

Chemical reagents/compounds/blocking antibodies: PMA, DAPT, and gliotoxin were obtained from Calbiochem (Merck Millipore). The γ-secretase inhibitor RO4929097 was obtained from Selleckchem. The NOTCH3 blocking antibody NRR3 was purchased from Genentech (Falk et al., 2012).

PCR, RT-PCR, and Western blot analysis: Total RNA was extracted using the Trizol isolation system (Invitrogen/Life Technologies, Paisley, UK). Genomic DNA (gDNA) was extracted using the Wizard Genomic DNA extraction kit from Promega. M-MLV reverse transcriptase and GoTaq PCR kits (Promega, WI, USA) were used for semiquantitative reverse transcriptase polymerase chain reactions (RT-PCR).

PCR and RT-PCR techniques (long range PCR using genomic DNA, semiquantitative RT-PCR), and western blotting (Modular Mini-Protean II system from Bio Rad) were used routinely according to standard protocols.

NOTCH2 gene characterization by sequencing: NOTCH2 deletions were analysed by sequencing gDNA PCR products spanning the deletions using a 3730 XL DNA analyzer from ABI Applied Biosystems (LGC Genomics, Berlin, Germany).

Preparation of nuclear extracts and EMSA: 3x10⁷ cells were lysed in 1ml hypotonic buffer (10mM HEPES, pH 7.9; 1.5mM MgCl₂; 10mM KCL) containing 0.15% NP-40 at 4°C for 10 min. The nuclear proteins were extracted from the nuclear fraction by resuspending the nuclei in 100µl extraction buffer (300mM KCl; 1.5mM MgCl₂; 20mM HEPES pH 7.9; 0.2mM EDTA; 25% Glycerin) at 4°C for 20 min with constant agitation. The CSL site spanning EMSA probe was derived from the *CD23a* promoter (5'-CAGCCCT*GTGGGAA*CTTGCTG-3' (SEQ ID NO: 40) annealed with the reverse complementary strand). The N1^{IC} (bTAN 20) and N2^{IC} (C651.6DbHN) antibodies was obtained from the Developmental Studies Hybridoma Bank (University of Iowa, Department of Biological Science, Iowa City, IA). EMSA was performed essentially as described (Hubmann et al., 2010)

siRNA mediated gene silencing: siRNA duplexes (siRNAs) for NOTCH1, NOTCH2, NOTCH3, and NR4A1 (ON-TARGETplusTM) and control (siGLO) were obtained from Dharmacon (Lafayette, CO, USA). Transfection of siRNAs into CLL cells was performed by using the lipid reagent siLentFectTM from Bio-Rad Laboratories. This method has been optimized to successfully transfect CLL cells (transfection efficiency 60-75%) (6).

Proliferation assay: Proliferation assays were performed using a colorimetric MTT assay (Biomedica, Vienna, Austria). This non-isotopic assay is based on the ability of functional mitochondria in the living cells to convert yellow coloured tetrazolium compound into red formazan.

### Example 2 - CLL cells express a constitutively activated form of NOTCH2

In the majority of CLL cases, DNA-bound NOTCH2 complexes are less sensitive/resistant to the selective GSI DAPT (Fig. 2B). This suggests that CLL cells express truncated, ligand independent N2^{IC} forms which are not tethered to the plasma membrane and, thus, do not require γ-secretase for processing and function (Hubmann et al., 2010). In the mouse models, such constitutive active N2^{IC} forms led to the selective development of Cd5+ (B-1a) B-cells (Witt et al., 2003), whose human equivalents are considered as non-transformed counterparts of CLL cells (Vardi et al., 2014; Chiorazzi et al., 2011). In CLL, the transcriptional activity of N2^{IC} is regulated by the protein kinase C (PKC) pathway linking NOTCH2 to the activated state of the leukemic cells *in vivo* and to the protective effect of PMA on CLL cells *in vitro* (Fig. 2B and C) (Hubmann et al., 2010).

### Example 3 - Gliotoxin is a potent NOTCH2 transactivation inhibitor and efficiently induces apoptosis in CLL cells by a mechanism involving the upregulation of NOTCH3 and NR4A1

It has been recently shown that the *Aspergillum* derived secondary metabolite gliotoxin is a potent NOTCH2 transactivation inhibitor (Fig 1A and 1B, see also WO/2006/135949 and US Patent No: 7,981,878) and efficiently induced apoptosis in CLL cells (Fig 2C).

The inhibition of oncogenic NOTCH2 signalling by gliotoxin may be associated with the concomitant induction of tumour suppressive NOTCH3 signalling as indicated by the induced co-expression of *NOTCH3* and its ligand *JAG2* (in unstimulated cells; Fig 2C, lane 3) or *DLL1* (in PMA activated cells; Fig 2C, lane 5) in the pre-apoptotic phase after gliotoxin treatment. NOTCH2 has been shown to antagonize NOTCH3 signalling (Shimizu et al., 2002) suggesting that the upregulation of NOTCH3 might be a consequence of gliotoxin mediated NOTCH2 inhibition in CLL cells. A putative CLL relevant NOTCH3 effector might be the activation induced cell death (AICD) mediator *NR4A1* (Fig 2C), a gene implicated in homeostasis regulation of splenic CD5+ (B-1a) B-cells (Hubmann et al., 2013; Tumang et al., 2004).

The downregulation of all NOTCH ligands in PMA-activated CLL cells (Fig 2C, lane 4) indicates that homotypic cell-cell contact is not able to trigger physiologic NOTCH2 signalling in normal, PKC stimulated CD5+ (B-1a) B-cells. In terms of CLL biology, it was hypothesized that constitutive active, ligand independent NOTCH2 signalling might dominantly suppress apoptotic NOTCH3 signalling, thereby enabling the (self-) antigen driven progredient expansion of the malignant clone outside of the protective MZ niche (Fig 2D).

### Example 4 - RO4929097 inhibits NOTCH3 and NR4A1 mRNA expression and counteracts spontaneous and gliotoxin mediated apoptosis in CLL cells

In a first attempt to investigate whether the postulated NOTCH3/NR4A1 axis is involved in the regulation of apoptosis in CLL cells, CLL cells were treated in the presence or absence of gliotoxin with the clinical relevant GSI RO4929097 taking advantage of a PMA activation model (Hubmann et al., 2010). In this model, NOTCH2 signalling, CD23 expression, and cell viability was preserved for at least one day (Fig 1B) and the cells formed tight clusters and expressed high amounts of the CLL proliferation centre marker *MYC* (Fig 2C). To exclude GSI mediated effects on NOTCH2 signalling, a CLL case was selected expressing a GSI resistant form of nuclear NOTCH2 as indicated by EMSA (CLL12 in Fig 3A).

As expected, gliotoxin rapidly inhibited NOTCH2 signalling and upregulated *NOTCH3* and *NR4A1* mRNA expression (Fig 3A, left panel). Interestingly, chronic stimulation with PMA led also to an upregulation of *NOTCH3* and *NR4A1* after 3 days (Fig 3A, right panel) suggesting that spontaneous apoptosis observed in CLL long-term cultures might be related to increased NOTCH3 activity.

In line with the hypothesis, RO4929097 inhibits *NOTCH3* and *NR4A1* expression (Fig 3A, right panel), and counteracts spontaneous and gliotoxin mediated apoptosis (Fig 3B) which stands in sharp contrast to the effect of gliotoxin. This suggests that *NR4A1* is a target gene for NOTCH3 signalling and supports the hypothesis that NOTCH3 has a tumour suppressive function in the leukemic cells.

### Example 5 - The NOTCH2 gene is consistently affected by gain of function mutations in CLL cells

One type of mutation which would explain the CLL specific NOTCH2 *gain of function* phenotype are deletions affecting the NOTCH extracellular domain (N^{EC}) (Ashworth et al., 2010). In a first attempt to address this issue, the NOTCH2 gene was screened by a long range PCR approach on the cDNA level (Fig. 5A) and recurring N2^{EC} deletions in all CLL cases independent of their clinical stage and their prognostic marker profile were found (Fig. 8):
DEL1: n=1 5'-123896 (E15) 3'-150809 (E26) Size: 26913 bp
DEL2: n=6 5'-136785 (E20) 3'-151967 (E27) Size: 15182 bp
DEL3: n=6 5'-137197 (E21) 3'-150823 (E26) Size: 13626 bp
DEL4: n=3 5'-137333 (E21) 3'-152303 (E28) Size: 14970 bp
DEL5: n=1 5'-137205 (E21) 3'-150811 (E26) Size: 13606 bp
DEL6: n=1 5'-130075 (E22) 3'-150756 (E26) Size: 11681 bp
DEL7: n=1 5'-136783 (E20) 3'-150883 (E26) Size: 14100 bp
DEL8: n=1 5'-136773 (E20) 3'-150873 (E26) Size: 14100 bp.

Whereas in some patients, the deletions are caused by splice site mutations (SSM; n=1), or intronic breakpoints resulting in exon skipping (ES1&2; n=3), the breakpoints from the majority of detected deletions (DEL1-8; n=16) were identified in confined exonic sequences (5'-hot spot: E20/21; 3'-hot spots: E26-28) as indicated in Fig. 8. All breakpoints are characterized by the loss of one short direct repeat (DR, Fig. 8) reminiscent of microhomology mediated end-joining (MMEJ), a non-replicative repair mechanism after a DNA double strand break. Due to their large size and their monoallelic nature (Fig. 5B), this kind of deletions are difficult to detect by standard next generation gDNA sequencing (NGS) approaches without confining the *NOTCH2* gene region by targeted sequencing. Given the short read length of NGS data (35-250 bp), their bioinformatics analysis remains challenging due to the short read alignment and the resulting bias against wild type sequences. Moreover, PCR based Sanger sequencing of selected *NOTCH2* gene regions, where one of the primers overlap with the deleted region, will also selectively amplify the wild type *NOTCH2* allele. This might explain why these deletions which were detected in the present invention were not found in former whole genome or transcriptome CLL sequencing studies.

### Example 6 - The localization of the N2^{EC} 3'-deletion break point can be expected to define the GSI sensitivity of the resulting truncated NOTCH2 proteins in CLL cells

Since N^{EC} deletions lead to the expression of truncated NOTCH proteins, the *NOTCH2* mRNA sequence downstream of the 3'-deletion border was screened for putative START codons. Two confined 3'-deletion breakpoint hotspots in exon 26 (*Hotspot* A) and exon 27&28 (*Hotspot* B) could be identified indicative for a stringent selection pressure to create a stable NOTCH2 *gain of function* phenotype in CLL cells (Fig. 6). Interestingly, two translational *START* codons following the KOZAK rule (AUGG) could be found downstream of the hotspots (Fig. 6).

The usage of START1 would yield in the N2^{™} truncation (~122 kD) which is expected to be cleaved at S1 by a furin like convertase to produce a mature N2^{™} form (Fig. 7B). N2^{™} is anchored to the transmembrane domain and, due to the lack of the auto inhibitory LNR (Lyn12/Notch) repeats, immediately processed by ADAM10 metalloprotease at S2, and γ-secretase at S3, to create the active N2^{IC} protein (Fig. 7B). The N2^{™} truncation is expected to be sensitive to GSI treatment and may account for the GSI sensitive CLL cases identified in previous work of the present inventors. Alternatively, the usage of the functionally confirmed *START2* codon downstream of the S3 site (Hubmann et al., 2010; Lauring et al., 2000) would yield a γ-secretase independent and, thus, a GSI resistant N2^{IC} truncation (~110kD) (Fig. 7B) which can be expected to account for GSI resistant CLL cases. In fact, screening of 10 randomly chosen CLL cases for N2^{™} and N2^{IC} expression by western blotting confirmed the presence of either N2^{IC} truncation alone, or both NOTCH2 protein truncations in CLL cells (Fig. 7C).

### Example 7 - Correlating the different N2^{EC} deletions with the GSI sensitivity of NOTCH2 in CLL cells

In the majority of patient samples the 3'-ends of the identified deletions clustered in confined hot spots within Exon 26 (n=11) or Exon 27 (n=6) indicative for a stringent selection pressure for such deletions to create a CLL initiating NOTCH2 *gain of function* phenotype. This hot spots are located upstream of a conserved internal ribosomal entry site (IRES) which has been shown to regulate the expression of a nuclear NOTCH2 form via a functional start codon immediately downstream of the γ-secretase cleavage site (S3, indicated in Fig 5) (Lauring et al., 2000). To correlate the different N2^{EC} deletions with the GSI sensitivity of NOTCH2, the expression of the NOTCH2 protein subspecies (NOTCH transmembrane form: N^{™} ~120 kD = GSI sensitive, and the NOTCH intracellular form: N^{IC} ~100 kD = GSI resistant) were analysed by western blotting. The GSI sensitivity of the samples was determined by incubating the CLL cells with RO4929097 for one day and by quantifying DNA-bound NOTCH2 complexes by EMSA (Fig 2).

### Example 8 - Analysing the distribution of the N2^{EC} deletions within the CLL clone by FISH analysis

All of the identified breakpoints so far were characterised by the loss of a short direct repeat (DR) at the deletion borders. This is a characteristic of microhomology mediated end-joining (MMEJ), a non-replicative repair mechanism after a DNA double strand break (see Fig 5) (McVey et al., 2008). Due to the high prevalence of these N2^{EC} deletions, these mutations may well be the most frequent disease defining genetic alteration in CLL. This initial malignant hit may take place in a self-renewing CD5+ (B-1a) B-cell (IgM memory B-cell; IGHV mutated CLL cases) in the marginal zone (MZ) of the spleen and, thus, may be the primary event in CLL leukemogenesis. Alternatively, the hit may also take place in a self-reactive B-cell precursor (IGHV unmutated CLL cases) which would be normally eliminated via tolerance mechanisms (anergy/negative selection). The distribution of the N2^{EC} deletion within the CLL clone should reflect the origin of the deletion event. If it is a primary event, one would expect that all CLL cells within the clone are positive for the deletion irrespective of the clinical stage of the disease. Moreover, the N2^{EC} deletion may be already detectable in the pre-phase of CLL (monoclonal B-cell lymphocytosis, MBL).

### Example 9 - Production and use of FISH probes

Two fish probe sets, A and B, were created. Probe set A

Probes of this set bind to the minimal deleted *NOTCH2* gene region according to SEQ ID NO: 3 (if said region is not deleted). This region is common to all deletions in CLL samples identified so far and, thus, serves as template for FISH probe set A. Specific oligonucleotides were calculated using the Primer-BLAST program from the NCBI-website.

Probe set A (color red, labelled with Texas Red) comprising 20 nt-oligonucleotides (5' to 3') specific for the NOTCH2 minimal deleted region in CLL cells (SEQ ID NO: 3); see also Figs. 6 and 9):
GCCCCCTTGAAAATAGGGCT (SEQ ID NO: 41)
AGATTGGGCCAGAGTTGGTG (SEQ ID NO: 42)
TTCCACAGGGATTTTCCGGG (SEQ ID NO: 43)
ATTACTCCTGCCAGTGTGCC (SEQ ID NO: 44)
TGTGAACTCTACACGGCACC (SEQ ID NO: 45)
CCAGGGGAACAGCAAGACAT (SEQ ID NO: 46)
CCACTTCAAGCAGCCTCTCA (SEQ ID NO: 47)
ACCTTCGTCGATGTGTGTCC (SEQ ID NO: 48)
AGCTTAGGCTGAAGCACTGG (SEQ ID NO: 49)
ATGTAGGAAGGGGGAGCAGT (SEQ ID NO: 50)
ATGTCTTGCTGTTCCCCTGG (SEQ ID NO: 51)
GAGTCAGGCTGTGCCAGTAG (SEQ ID NO: 52)
TAATAAGGAGGCTGGCGCTG (SEQ ID NO: 53)
GCACTGTGAAACCTTCGTCG (SEQ ID NO: 54)
CACAGCACAAGTCCCTCCAT (SEQ ID NO: 55)
TTTCAGATGGTGCCAGCCTT (SEQ ID NO: 56)
TAAGACCGGGAGGGTTCACA (SEQ ID NO: 57)
TGTGAACCCTCCCGGTCTTA (SEQ ID NO: 58)
CTCCTCATCCCCTACCCCTT (SEQ ID NO: 59)
GTGCTCTCCCTTGCTGACAA (SEQ ID NO: 60)
GGCTGCTTGAAGTGGGAAAC (SEQ ID NO: 61)
TGAGCAACAGGGCACTTACC (SEQ ID NO: 62)
TGCCTGAATGGAGGGACTTG (SEQ ID NO: 63)
CAGCCCAAGCCAAGGTTACT (SEQ ID NO: 64)

### Probe set B

Probes of this set bind to the NOTCH2 deleted gene region between START1 and START2 (if said regions is not deleted). This region serves as template for FISH probe set B, that identifies NOTCH2 gene deletions which yield truncated NOTCH2 forms that are not tethered to the plasma membrane and, thus, are independent for γ-secretase for processing and function. These CLL patients will not benefit from GSI treatment.

Probe set B (color green, labelled with Cy2) comprising 20 bp-oligonucleotides (5' to 3') specific for the region between START1 and START2 (SEQ ID NO: 6):
ACCTTTTGCCAGAGTTGCCT (SEQ ID NO: 65)
CCGCCAGTGTGTTCAAGACT (SEQ ID NO: 66)
GTCTGTCGTCAGTGAGTGGC (SEQ ID NO: 67)
TCTTGAACACACTGGCGGTT (SEQ ID NO: 68)
GGATGACACGCAGATCCCTT (SEQ ID NO: 69)
GTACCCCTGCCCACTTTTCA (SEQ ID NO: 70)
GAAAAGTGGGCAGGGGTACA (SEQ ID NO: 71)
CTCCCCTTCAGCATCAGGTC (SEQ ID NO: 72)
GTCTTGGCTGAAAAGTGGGC (SEQ ID NO: 73)
GTGCTTTCCTCCCGAAGGTT (SEQ ID NO: 74)
AATCCCTGACTCCAGAACGC (SEQ ID NO: 75)
TTGCCATGATTACCCCCAGC (SEQ ID NO: 76)
AGACCACTGAAACCTTCGGG (SEQ ID NO: 77)
CCCACCTTCCTTAGTCTGGC (SEQ ID NO: 78)
CTGAAAAGTGGGCAGGGGTA (SEQ ID NO: 79)
CCTCCCGAAGGTTTCAGTGG (SEQ ID NO: 80)
GCCAGACTAAGGAAGGTGGG (SEQ ID NO: 81)
AGTCTTGAACACACTGGCGG (SEQ ID NO: 82)
GTGGGGACCTGATGCTGAAG (SEQ ID NO: 83)
CCGTGTTCTTGAAGCAGTGG (SEQ ID NO: 84)

PBMCs are obtained from a patient having CLL, labelled with the FISH probe sets A and B described above and inspected in a fluorescence microscope. Specifically, the protocol for interphase analysis for fresh specimens (fresh cells) by FISH as disclosed in van Stedum & King, 2003 is used.

The Notch2 deletions described herein are typically heterozygous, i.e. present on a single chromosome only. Probe set A and B yield a mixed color signal (i.e. yellow) on the NOTCH2 WT allele of obtained PBMCs. This also serves as an internal positive control that the FISH labelling worked.

The NOTCH2 allele harbouring the deletions described herein yields either no signal (sets A and B not binding, i.e. expression of N2^{IC} truncation can be expected) or just a green signal B (set A not binding, i.e. expression of the N2^{™} truncation can be expected).

In the former case, the patient is designated as eligible for GSI treatment, in the latter case, the patient is designated as not eligible for GSI treatment.

### References

Almagro & Fransson. Front Biosci 13.1 (2008): 1619-1633.
Andersson et al. Nat Rev Drug Discov. 13(5), 357-78 (2014).
Arriba et al. Molecular Carcinogenesis 1-12 (2015).
Ashworth et al. Blood 116(25), 5455-64 (2010).
Birch & Racher, Adv Drug Deliv Rev 2006, PMID: 16822577
Buhmann, et al. British journal of haematology 118.4 (2002): 968-975.
Bray Nat Rev Mol Cell Biol. 7(9), 678-89 (2006).
Carlsson et al. Blood 71(2), 415-21 (1988).
Chillakuri et al. Seminars in cell & developmental biology 23(4), 421-428 (2012).
Chiorazzi et al. Blood 117(6), 1781-91 (2011).
Di Bernardo et al. Nature genetics 40(10), 1204-1210 (2008).
Duechler et al. Leukemia 19(2), 260-7 (2005).
Eichhorst et al. Annals of Oncology 26, v78-v84 (2015).
Ellisen et al. Cell 66, 649-661 (1991).
Falk et al. Methods 58(1), 69-78 (2012).
Haferlach, et al. Leukemia 21.12 (2007): 2442-2451.
Haferlach & Bacher. Cancer Cytogenetics: Methods and Protocols (2011): 119-130.
Herishanu et al. Blood 117(2), 563-74 (2011).
Hubmann et al. Blood 99(10), 3742-7 (2002).
Hubmann et al. Br J Haematol. 148(6), 868-78 (2010).
Hubmann et al. Br J Haematol. 160(5), 618-29 (2013).
Isidor et al. Nat Genet 43(4), 306- 308 (2011).
Jiao et al. Genes, chromosomes & cancer 51(5), 480-489 (2012).
Kiel et al. J Exp Med 209(9), 1553-1565 (2012).
Kridel et al. Blood 19(9), 1963-1971 (2012).
Lauring et al. Mol Cell. 6(4), 939-45 (2000).
Lee et al. Cancer ResAPMIS 11568, 5273-52811357-63 (20078).
Li et al. Chin Med J 126(21), 4175-4182 (2013).
Limnander et al. Mol Cell Biol. 34(8), 1474-85 (2014).
Lohr et al. Proc Natl Acad Sci U S A 109(10), 3879-3884 (2012).
Louvi et al. Seminars in cell & developmental biology 23(3), 473-480 (2012).
McVey et al. Trends Genet. 24(11), 529-38 (2008).
Ntziachristos et al. Cancer cell 25(3), 318-334 (2014).
Olsauskas-Kuprys et al. Onco Targets Ther 6 (2013): 943-955.
Panani et al. Anticancer Research 24, 4141-4146 (2004).
Puente et al. Nature 475(7354), 101 -105 (2011).
Rosati et al. Blood 113(4), 856-65 (2009).
Rossi et al. J Exp Med 209(9), 1537-1551 (2012).XLee et al.
Cancer science 100(5), 920-926 (2009).
Shehata et al. Blood 116(14), 2513-21 (2010).
Shehata et al. J Clin Invest. 113(5), 676-85 (2004).
Shehata et al. Leukemia 24(12), 2122-7 (2010).
Shimizu et al. Biochem Biophys Res Commun. 291(4), 775-9 (2002).
Simpson et al. Nat Genet 43(4), 303-305 (2011).
Stilgenbauer et al. Blood 123(21), 3247-3254 (2014).
Tibiletti, Cytogenetic and genome research 118.2-4 (2007): 229-236.
Tumang et al. Eur J Immunol. 34(8), 2158-67 (2004).
Van Stedum & King. Molecular cytogenetics: protocols and applications (2003): 51-63.
Vardi et al. Cancer Res. 74(16), 4211-6 (2014).
Villamor et al. Leukemia 27(5), 1100-1106 (2013).
Weng et al. Science 306(5694), 269-271 (2004).
Westhoff et al. Proc. Natl. Acad. Sci. USA 106, 22293-22298 (2009) .
Willander et al. BMC cancer 13(1), 1-6 (2013).
Witt et al. Mol Cell Biol. 23(23), 8637-50 (2003).
Zenz et al. Nature Reviews Cancer 10(1), 37-50 (2010).

## Claims

1. A polynucleotide probe for detecting a region of the human NOTCH2 gene in a cell, the sequence of the region being at least 10, preferably at least 15 consecutive nucleotides selected from the sequence according to SEQ ID NO: 1,
wherein the probe is specific for the region or for its reverse complement, and wherein the probe is bound to a molecular tag.

2. The probe of claim 1, wherein the sequence of the region is at least 10, preferably at least 15 consecutive nucleotides selected from the sequence according to SEQ ID NO: 3.

3. The probe of claim 1, wherein the sequence of the region is at least 10, preferably at least 15 consecutive nucleotides selected from the sequence according to SEQ ID NO: 6.

4. A polynucleotide probe, wherein at least 75%, preferably at least 80%, more preferably at least 85%, even more preferably at least 90%, especially at least 95% of the sequence of the probe consist of at least 10, preferably at least 15 consecutive nucleotides selected from the sequence according to SEQ ID NO: 1 or its reverse complement,
wherein the probe is bound to a molecular tag.

5. A container, comprising a polynucleotide probe composition especially for fluorescence in-situ hybridisation (FISH),
wherein the composition comprises at least one probe, preferably at least two different probes, the probe as claimed in any one of claims 1 to 4, and preferably a buffer in which the probes are dissolved;
in particular wherein the composition is for determining whether a cell has a human NOTCH2 gene with a deletion starting at a locus in or downstream of exon 20 and ending at a locus upstream of the alternative start codon START2, preferably wherein the deletion encompasses at least a portion of SEQ ID NO: 3.

6. Use of the probe or container of any one of claims 1 to 5 for determining whether a cell has a human NOTCH2 gene with a deletion starting at a locus in or downstream of exon 20 and ending at a locus upstream of the alternative start codon START2, in particular wherein the deletion encompasses at least a portion of SEQ ID NO: 3;
preferably wherein the cell is a cancer cell or a cell suspected of being a cancer cell, especially for designating the cell as having a gain-of-function NOTCH2 mutation caused by the deletion, or for determining whether the patient from whom the cell was obtained is eligible for a therapy that targets NOTCH2, in particular a gliotoxin-based therapy.

7. The use of claim 6, wherein said cancer is selected from leukemia, preferably chronic lymphocytic leukemia (CLL), melanoma, pancreas carcinoma, hepatocellular carcinoma, medulloblastoma, glioblastoma and lymphoma, preferably marginal zone lymphoma or mantle cell lymphoma (MCL).

8. The use of any one of claims 6 or 7, for determining whether the cancer cell is gamma-secretase-inhibitor sensitive, wherein said probe is, or the composition of said container comprises, the probe as set forth in claim 3.

9. The use of any one of claims 6 to 8, comprising the use of FISH for the detecting, wherein the probe is a FISH probe.

10. A method for detecting the presence or absence of a region of a human NOTCH2 gene on a chromosome of a cell, the sequence of the region being at least 10, preferably at least 15 consecutive nucleotides selected from the sequence according to SEQ ID NO: 1, the method comprising
- providing the cell, and
- contacting a probe specific for the region or for its reverse complement with the chromosome or a fragment thereof, and
- detecting whether the probe is bound to the chromosome or fragment thereof;
preferably for determining whether the NOTCH2 gene has a deletion starting at a locus in or downstream of exon 20 and ending at a locus upstream of the alternative start codon START2, wherein the deletion comprises said region.

11. The method of claim 10, wherein the cell is a cancer cell or a cell suspected of being a cancer cell, in particular wherein said cancer is selected from leukemia, preferably CLL, melanoma, pancreas carcinoma, hepatocellular carcinoma, medulloblastoma, glioblastoma and lymphoma, preferably marginal zone lymphoma or mantle cell lymphoma (MCL);
preferably wherein the method is for determining whether the patient the cell was obtained from is eligible for a therapy that targets NOTCH2, in particular a gliotoxin-based therapy.

12. The method of claim 10 or 11, wherein said probe is the probe of any one of claims 1 to 5.

13. An antibody specific for an N-terminally truncated human Notch2 protein, the N-terminally truncated human Notch2 protein starting with the sequence MVYP (SEQ ID NO: 4) or the sequence MAKR (SEQ ID NO: 5).

14. A NOTCH2 signalling pathway inhibitor, in particular a gliotoxin, for use in treatment of cancer or prevention of cancer relapse in a patient, wherein at least a portion of the cells of the cancer have a human NOTCH2 gene with a deletion starting at a locus in or downstream of exon 20 and ending at a locus upstream of the alternative start codon START2.

15. A gamma-secretase inhibitor for use in treatment of cancer or prevention of cancer relapse in a patient, wherein at least a portion of the cells of the cancer have a human NOTCH2 gene with a deletion starting at a locus in or downstream of exon 20 and ending at a locus upstream of the alternative start codon START1.
